# EUROPEAN PATENT SPECIFICATION

(11) **EP 1 841 452 B1**
(45) Date of publication and mention of the grant of the patent: **02.04.2014**
(21) Application number: 05789778.7
(22) Date of filing: 07.10.2005
(51) Int. Cl.: A61K 39/395, C07K 16/28

(54) **SINGLE DOMAIN ANTIBODIES AGAINST TNFR1 AND METHODS OF USE THEREFOR**
EINZEL-DOMÄN ANTIKÖRPER GEGEN TNFR1 UND VERFAHREN UND DEREN VERWENDUNGEN
ANTICORPS A DOMAINE UNIQUE CONTRE LE TNFR1 ET PROCEDES ET LEUR UTILISATIONS

(30) Priority: 08.10.2004 WO PCT/GB2004/004253; 10.11.2004 US 985847
(43) Date of publication of application: 10.10.2007
(60) Divisional application: 10182954.7
(73) Proprietor: Domantis Limited, Cambridge Cambridgeshire, CB4 OWG (GB)
(72) Inventor: BREWIS, Neil, D., Cambridge CB4 0WG (GB); WOOLVEN, Benjamin, P., Cambridge CB4 0WG (GB); HOLMES, Steve, Cambridge CB4 0WG (GB); TOMLINSON, Ian, M., Cambridge CB4 0WG (GB); LEE, Jennifer, Cambridge CB4 0WG (GB); ENEVER, Carolyn, Cambridge CB4 0WG (GB); BASRAN, Amrik, Cambridge CB4 0WG (GB); JONES, Kate, Cambridge CB4 0WG (GB); WILDT, Ruud de, Cambridge CB4 0WG (GB); BLEIN, Stanislas, Charles, Cambridge CB4 0WG (GB)
(74) Representative: Clube, Jasper Rupert
(86) International application number: PCT/GB2005/003873
(87) International publication number: WO 2006/038027

(56) References cited:
- WO-A-2004/101790
- WO-A-2005/035572
- WO-A2-2004/003019
- WO-A2-2004/058821
- WO-A2-2004/081026

## Description

### RELATED APPLICATIONS

This application is a continuation-in-part of U.S. Patent Application No. 10/985,847, filed on November 10, 2004, which is:
1) a continuation-in-part of International Application No. PCT/GB2004/004253, which designated the United States and was filed on October 8, 2004; and
2) a continuation-in-part of International Application No. PCT/GB2003/005646, which designated the United States, was filed on December 24, 2003, and claims priority to United Kingdom Application No. GB 0230202.4, filed December 27, 2002 and United Kingdom Application No. GB 0327706.8 filed November 28, 2003, which is
   a continuation-in-part of International Application No. PCT/GB2003/002804, which designated the United States, was filed on June 30, 2003, and claims priority to United Kingdom Application No. GB 0230202.4, filed December 27, 2002, which is
   a continuation-in-part of International Application No. PCT/GB02/03014, which designated the United States and was filed on June 28, 2002.

### BACKGROUND OF THE INVENTION

The antigen binding domain of an antibody comprises two separate regions: a heavy chain variable domain (V_{H}) and a light chain variable domain (V_{L}: which can be either V_{κ} or V_{λ}). The antigen binding site itself is formed by six polypeptide loops: three from V_{H} domain (H1, H2 and H3) and three from V_{L} domain (L1, L2 and L3). A diverse primary repertoire of V genes that encode the V_{H} and V_{L} domains is produced by the combinatorial rearrangement of gene segments. The V_{H} gene is produced by the recombination of three gene segments, V_{H}, D and J_{H}. In humans, there are approximately 51 functional V_{H} segments (Cook and Tomlinson (1995) Immunol Today, 16: 237), 25 functional D segments (Corbett et al. (1997) J. Mol. Biol., 268: 69) and 6 functional J_{H} segments (Ravetch et al. (1981) Cell, 27: 583), depending on the haplotype. The V_{H} segment encodes the region of the polypeptide chain which forms the first and second antigen binding loops of the V_{H} domain (H1 and H2), whilst the V_{H}, D and J_{H} segments combine to form the third antigen binding loop of the V_{H} domain (H3). The V_{L} gene is produced by the recombination of only two gene segments, V_{L} and J_{L}. In humans, there are approximately 40 functional V_{κ} segments (Schäble and Zachau (1993) Biol. Chem. Hoppe-Seyler, 374: 1001), 31 functional V_{λ} segments (Williams et al. (1996) J. Mol. Biol., 264: 220; Kawasaki et al. (1997) Genome Res., 7: 250), 5 functional J_{κ} segments (Hieter et al. (1982) J. Biol. Chem., 257: 1516) and 4 functional J_{λ} segments (Vasicek and Leder (1990) J. Exp. Med., 172: 609), depending on the haplotype. The V_{L} segment encodes the region of the polypeptide chain which forms the first and second antigen binding loops of the V_{L} domain (L1 and L2), whilst the V_{L} and J_{L} segments combine to form the third antigen binding loop of the V_{L} domain (L3). Antibodies selected from this primary repertoire are believed to be sufficiently diverse to bind almost all antigens with at least moderate affinity. High affinity antibodies are produced by "affinity maturation" of the rearranged genes, in which point mutations are generated and selected by the immune system on the basis of improved binding.

Analysis of the structures and sequences of antibodies has shown that five of the six antigen binding loops (H1, H2, L1, L2, L3) possess a limited number of main-chain conformations or canonical structures (Chothia and Lesk (1987) J. Mol. Biol., 196: 901; Chothia et al. (1989) Nature, 342: 877). The main-chain conformations are determined by (i) the length of the antigen binding loop, and (ii) particular residues, or types of residue, at certain key position in the antigen binding loop and the antibody framework. Analysis of the loop lengths and key residues has enabled us to the predict the main-chain conformations of H1, H2, L1, L2 and L3 encoded by the majority of human antibody sequences (Chothia et al. (1992) J. Mol. Biol., 227: 799; Tomlinson et al. (1995) EMBO J., 14: 4628; Williams et al. (1996) J. Mol. Biol., 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol., 263: 800; Shirai et al. (1996) FEBS Letters, 399: 1.

Bispecific antibodies comprising complementary pairs of V_{H} and V_{L} regions are known in the art. These bispecific antibodies must comprise two pairs of V_{H} and V_{L}s, each V_{H}/V_{L} pair binding to a single antigen or epitope. Methods described involve hybrid hybridomas (Milstein & Cuello AC, Nature 305:537-40), minibodies (Hu et al., (1996) Cancer Res 56:3055-3061;), diabodies (Holliger et al., (1993) Proc. Natl. Acad. Sci. USA 90, 6444-6448; WO 94/13804), chelating recombinant antibodies (CRAbs; (Neri et al., (1995) J. Mol. Biol. 246, 367-373), biscFv (e.g. Atwell et al., (1996) Mol. Immunol. 33, 1301-1312), "knobs in holes" stabilised antibodies (Carter et al., (1997) Protein Sci. 6, 781-788). In each case each antibody species comprises two antigen-binding sites, each fashioned by a complementary pair of V_{H} and V_{L} domains. Each antibody is thereby able to bind to two different antigens or epitopes at the same time, with the binding to EACH antigen or epitope mediated by a V_{H} and its complementary V_{L} domain. Each of these techniques presents its particular disadvantages; for instance in the case of hybrid hybridomas, inactive V_{H}/V_{L} pairs can greatly reduce the fraction of bispecific IgG. Furthermore, most bispecific approaches rely on the association of the different V_{H}/V_{L} pairs or the association of V_{H} and V_{L} chains to recreate the two different V_{H}/V_{L} binding sites. It is therefore impossible to control the ratio of binding sites to each antigen or epitope in the assembled molecule and thus many of the assembled molecules will bind to one antigen or epitope but not the other. In some cases it has been possible to engineer the heavy or light chains at the sub-unit interfaces (Carter *et al*., 1997) in order to improve the number of molecules which have binding sites to both antigens or epitopes but this never results in all molecules having binding to both antigens or epitopes.

There is some evidence that two different antibody binding specificities might be incorporated into the same binding site, but these generally represent two or more specificities that correspond to structurally related antigens or epitopes or to antibodies that are broadly cross-reactive. For example, cross-reactive antibodies have been described, usually where the two antigens are related in sequence and structure, such as hen egg white lysozyme and turkey lysozyme (McCafferty et al., WO 92/01047) or to free hapten and to hapten conjugated to carrier (Griffiths AD et al. EMBO J 1994 13:14 3245-60). In a further example, WO 02/02773 (Abbott Laboratories) describes antibody molecules with "dual specificity". The antibody molecules referred to are antibodies raised or selected against multiple antigens, such that their specificity spans more than a single antigen. Each complementary V_{H}/V_{L} pair in the antibodies of WO 02/02773 specifies a single binding specificity for two or more structurally related antigens; the V_{H} and V_{L} domains in such complementary pairs do not each possess a separate specificity. The antibodies thus have a broad single specificity which encompasses two antigens, which are structurally related. Furthermore natural autoantibodies have been described that are polyreactive (Casali & Notkins, Ann. Rev. Immunol. 7, 515-531), reacting with at least two (usually more) different antigens or epitopes that are not structurally related. It has also been shown that selections of random peptide repertoires using phage display technology on a monoclonal antibody will identify a range of peptide sequences that fit the antigen binding site. Some of the sequences are highly related, fitting a consensus sequence, whereas others are very different and have been termed mimotopes (Lane & Stephen, Current Opinion in Immunology, 1993, 5, 268-271). It is therefore clear that a natural four-chain antibody, comprising associated and complementary V_{H} and V_{L} domains, has the potential to bind to many different antigens from a large universe of known antigens. It is less clear how to create a binding site to two given antigens in the same antibody, particularly those which are not necessarily structurally related.

Protein engineering methods have been suggested that may have a bearing on this. For example it has also been proposed that a catalytic antibody could be created with a binding activity to a metal ion through one variable domain, and to a hapten (substrate) through contacts with the metal ion and a complementary variable domain (Barbas et al., 1993 Proc. Natl. Acad. Sci USA 90, 6385-6389). However in this case, the binding and catalysis of the substrate (first antigen) is proposed to require the binding of the metal ion (second antigen). Thus the binding to the V_{H}/V_{L} pairing relates to a single but multi-component antigen.

Methods have been described for the creation of bispecific antibodies from camel antibody heavy chain single domains in which binding contacts for one antigen are created in one variable domain, and for a second antigen in a second variable domain. However the variable domains were not complementary. Thus a first heavy chain variable domain is selected against a first antigen, and a second heavy chain variable domain against a second antigen, and then both domains are linked together on the same chain to give a bispecific antibody fragment (Conrath et al., J. Biol. Chem. 270, 27589-27594). However the camel heavy chain single domains are unusual in that they are derived from natural camel antibodies which have no light chains, and indeed the heavy chain single domains are unable to associate with camel light chains to form complementary V_{H} and V_{L} pairs.

Single heavy chain variable domains have also been described, derived from natural antibodies which are normally associated with light chains (from monoclonal antibodies or from repertoires of domains; see EP-A-0368684). These heavy chain variable domains have been shown to interact specifically with one or more related antigens but have not been combined with other heavy or light chain variable domains to create a ligand with a specificity for two or more different antigens. Furthermore, these single domains have been shown to have a very short *in vivo* half-life. Therefore such domains are of limited therapeutic value.

It has been suggested to make bispecific antibody fragments by linking heavy chain variable domains of different specificity together (as described above). The disadvantage with this approach is that isolated antibody variable domains may have a hydrophobic interface that normally makes interactions with the light chain and is exposed to solvent and may be "sticky" allowing the single domain to bind to hydrophobic surfaces. Furthermore, in the absence of a partner light chain the combination of two or more different heavy chain variable domains and their association, possibly via their hydrophobic interfaces, may prevent them from binding to one in not both of the ligands they are able to bind in isolation. Moreover, in this case the heavy chain variable domains would not be associated with complementary light chain variable domains and thus may be less stable and readily unfold (Worn & Pluckthun, 1998 Biochemistry 37, 13120-7).

### SUMMARY OF THE INVENTION

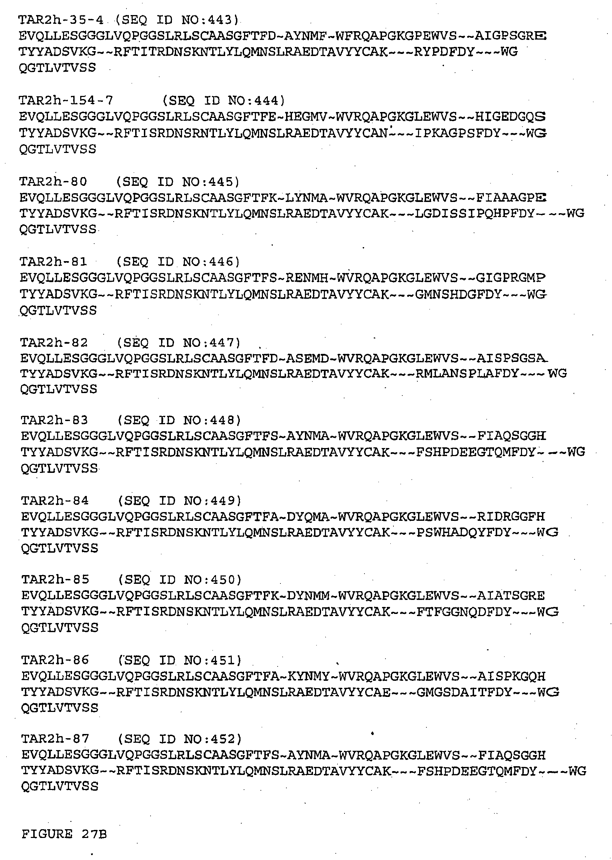

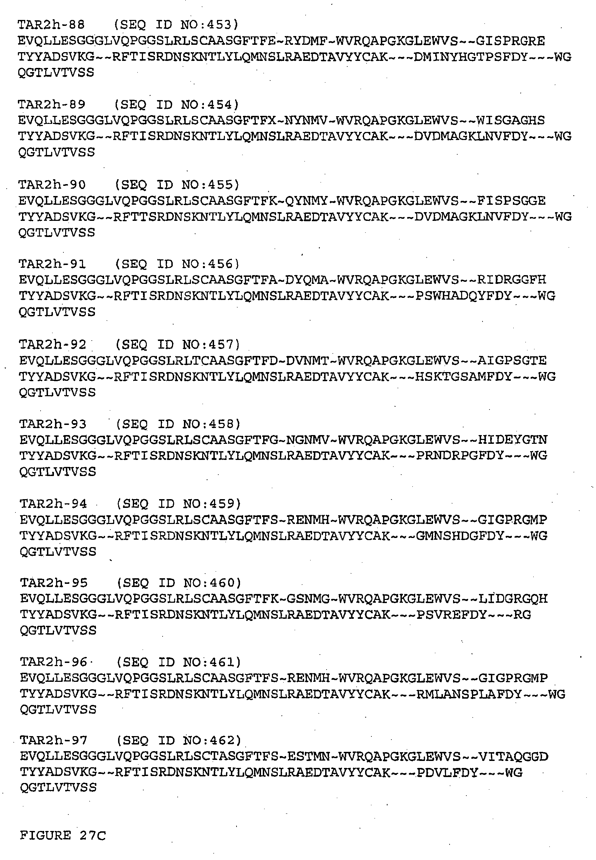

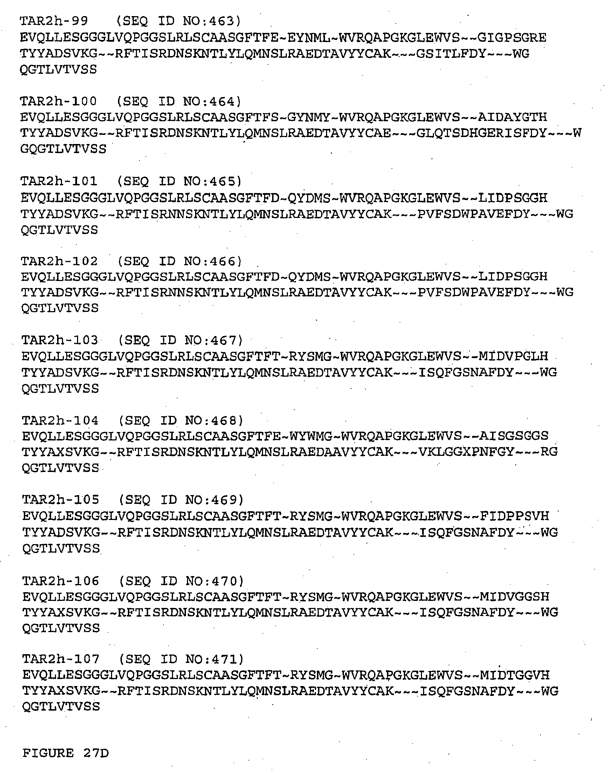

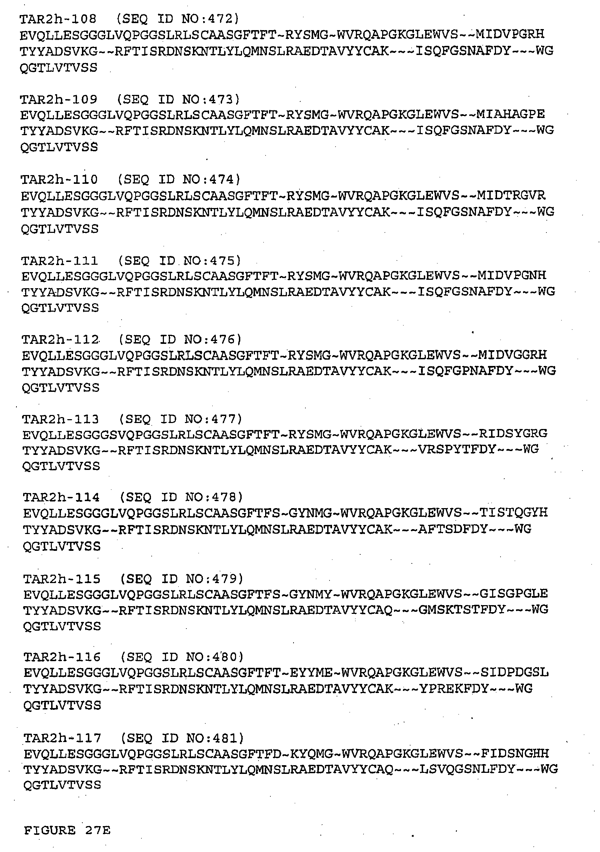

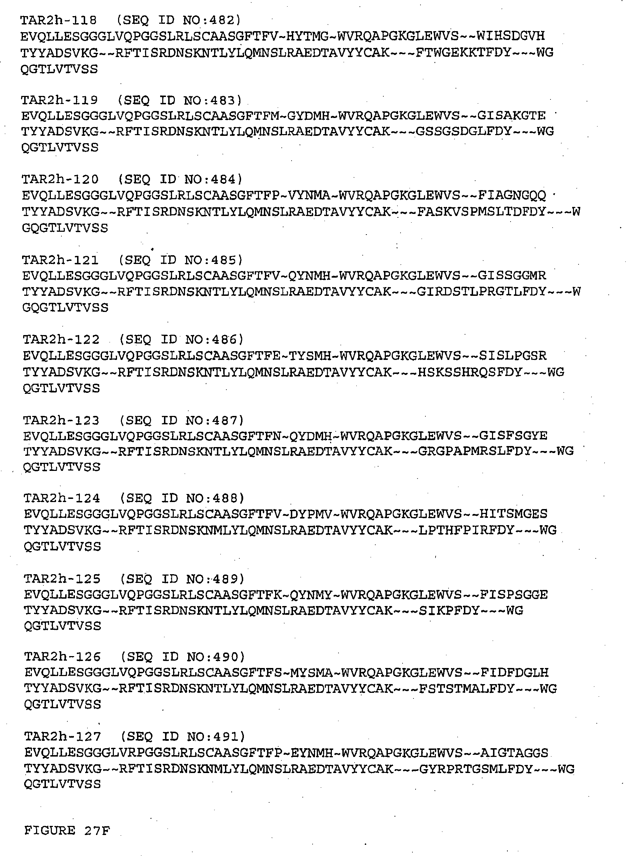

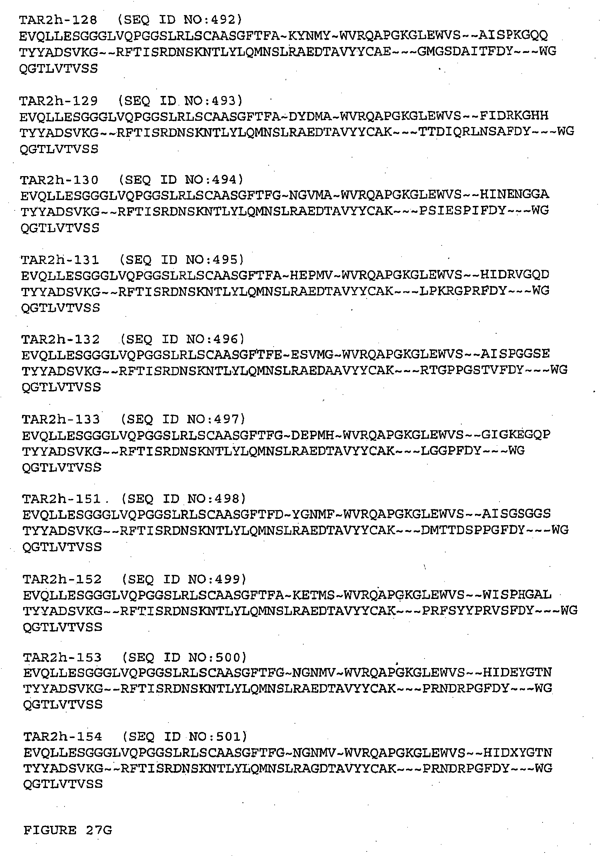

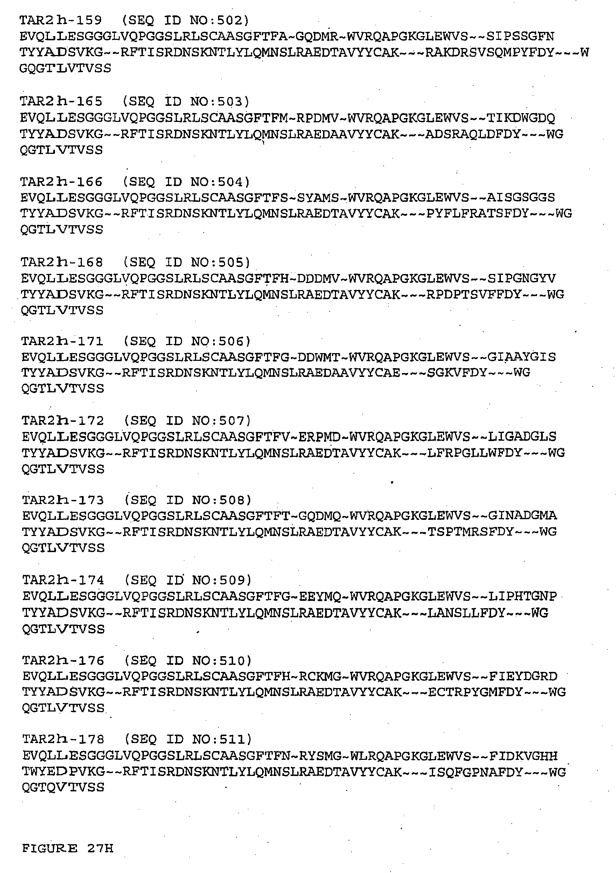

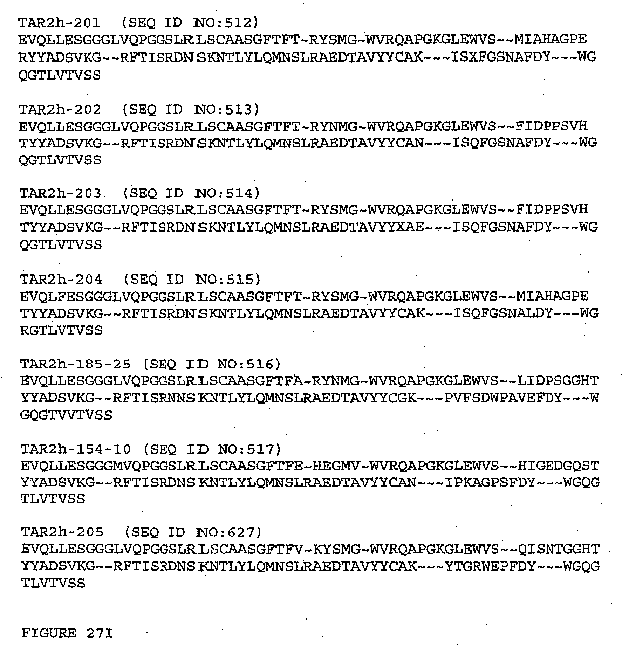

The invention is an antagonist of Tumor Necrosis Factor 1 (TNFR1) that binds TNFR1 and inhibits signal transduction through TNFR1; (i) wherein said antagonist does not inhibit binding of TNFα to TNFR1; (ii) wherein said antagonist is a domain antibody (dAb); and (iii) wherein said dAb comprises an amino acid sequence that is at least 90% homologous to the amino acid sequence of TAR2h-205 (SEQ ID NO: 627 in Figure 27I).

Other preferred antagonists are ligands described herein that bind TNFR1. The ligands comprise a domain antibody (dAb) of the invention that has binding specificity for TNFR1 in a suitable format. In some embodiments, the ligand is a dAb monomer that consists essentially of, or consists of, a dAb of the invention that has binding specificity for TNFR1. In other embodiments, the ligand is a polypeptide that comprises a dAb in a suitable format, such as an antibody format.

In certain embodiments, the ligand is a dual-specific ligand that comprises a first dAb of the invention that binds TNFR1 and a second dAb that has a different binding specificity from the first dAb. In one example, the dual-specific ligand comprises a first dAb of the invention that binds a first epitope on TNFR1 and a second dAb that binds an epitope on a different target. In another example, the second dAb binds an epitope on serum albumin.

The ligand or dAb monomer of the disclosure is characterized by one or more of the following: 1) dissociates from human TNFR1 with a dissociation constant (K_{d}) of 50 nM to 20 pM, and a K_{off} rate constant of 5x10⁻¹ to 1x10⁻⁷ s⁻¹; 2) inhibits binding of Tumor Necrosis Factor Alpha (TNFα) to TNFR1 with an IC50 of 500 nM to 50 pM; 3) neutralizes human TNFR1 in a standard L929 cell assay with an ND50 of 500 nM to 50 pM; 4) antagonizes the activity of the TNFR1 in a standard cell assay with an ND₅₀ of ≤ 100 nM, and at a concentration of ≤ 10µM the dAb agonizes the activity of the TNFR1 by ≤ 5% in the assay; 5) inhibits lethality in the mouse LPS/D-galactosamine-induced septic shock model; 6) resists aggregation; 7) is secreted in a quantity of at least about 0.5 mg/L when expressed in *E*. *coli* or *Pichia* species (e.g., *P. pastoris*); 8) unfolds reversibly; 9) has efficacy in a model of chronic inflammatory disease selected from the group consisting of mouse collagen-induced arthritis model, mouse ΔARE model of arthritis, mouse ΔARE model of inflammatory bowel disease, mouse dextran sulfate sodium-induced model of inflammatory bowel disease, mouse tobacco smoke model of chronic obstructive pulmonary disease, and suitable primate models (e.g., primate collagen-induced arthritis model); and/or 10) has efficacy in treating, suppressing or preventing a chronic inflammatory disease.

In particular embodiments, the ligand or dAb monomer of the disclosure dissociates from human TNFR1 with a dissociation constant (K_{d}) of 50 nM to 20 pM, and a K_{off} rate constant of 5x10⁻¹ to 1x10⁻¹ s⁻¹; inhibits binding of Tumor Necrosis Factor Alpha (TNFα) to TNFR1 with an IC50 of 500 nM to 50 pM; and neutralizes human TNFR1 in a standard L929 cell assay with an ND50 of 500 nM to 50 pM. In other particular embodiments, the ligand or dAb monomer dissociates from human TNFR1 with a dissociation constant (K_{d}) of 50 nM to 20 pM, and a K_{off} rate constant of 5x10⁻¹ to 1x10⁻⁷ s⁻¹; inhibits binding of Tumor Necrosis Factor Alpha (TNFα) to TNFR1 with an IC50 of 500 nM to 50 pM; and has efficacy in a model of chronic inflammatory disease selected from the group consisting of mouse collagen-induced arthritis model, mouse ΔARE model of arthritis, mouse ΔARE model of inflammatory bowel disease, mouse dextran sulfate sodium-induced model of inflammatory bowel disease, mouse tobacco smoke model of chronic obstructive pulmonary disease, and suitable primate models (e.g., primate collagen-induced arthritis model). In other particular embodiments, the ligand or dAb monomer dissociates from human TNFR1 with a dissociation constant (K_{d}) of 50 nM to 20 pM, and a K_{off} rate constant of 5x10⁻¹ to 1x10⁻⁷ s⁻¹; neutralizes human TNFR1 in a standard L929 cell assay with an ND50 of 500 nM to 50 pM; and antagonizes the activity of the TNFR1 in a standard cell assay with an ND₅₀ of ≤ 100 nM, and at a concentration of ≤ 10µM the dAb agonizes the activity of the TNFR1 by ≤ 5% in the assay.

The ligand or dAb monomer comprises an amino acid sequence that is at least about 90% homologous to an amino acid sequence of a dAb which is TAR2h-205 (SEQ ID NO:627).

The invention relates to an antagonist of Tumor Necrosis Factor I (TNFR1) that binds Tumor Necrosis Factor 1 (TNFR1) and inhibits signal transduction through TNFR1, wherein said antagonist does not inhibit binding of TNFα, to TNFR1. In some embodiments, the antagonist comprises a first domain antibody (dAb) monomer and a second dAb monomer, wherein said first dAb monomer of the invention binds a domain of TNFR1 selected from the group consisting of Domain 1, Domain 2, Domain 3 and Domain 4, and said second dAb monomer binds a domain of TNFR1 selected from the group consisting of Domain 1, Domain 2, Domain 3 and Domain 4, wherein said antagonist does not agonize TNFR1 when present at a concentration of about 1 µM in a standard L929 cytotoxicity assay or a standard HeLa IL-8 assay

In some embodiments the dAb of the invention monomer inhibits TNFα-induced crosslinking or clustering of TNFR1 on the surface of a cell.

The invention also provides isolated and/or recombinant nucleic acid molecules that encode the ligands of the invention, and vectors that comprise the recombinant nucleic acid molecules. Also provided are host cells comprising the recombinant nucleic acid molecules or vectors of the invention and methods for producing the ligands.

The invention also relates to pharmaceutical compositions comprising an antagonist or ligand of the invention and a pharmacologically, physiologically or pharmaceutically acceptable carrier.

The invention also provides for the use of a ligand of the invention for the manufacture of a medicament for treating, suppressing or preventing chronic inflammatory disease, an autoimmune disorder, inflammatory disease, arthritis, multiple sclerosis, inflammatory bowel disease, chronic obstructive pulmonary disease, pneumonia or septic shock.

The single variable domains or domain antibodies (dAb) that have binding specificity for TNFR1 and ligands comprising these single variable domains or dAbs have several advantages. For example, the dAbs that have binding specificity for TNFR1 described herein antagonize TNFR1. Accordingly therapeutic agents that comprise an anti-TNFR1 dAb of the invention can be administered (e.g., for therapeutic, diagnostic or prophylactic purposes) with substantially reduced risk of side effects caused by binding and/or antagonizing TNFR2 (e.g., immunosuppression). Therapeutic agents that target TNF alpha, such as ENBREL® (entarecept; Immunex Corporation) antagonize TNFR1 and TNFR2, and administering such agents can produce immunosuppression and related side effects (e.g., serious infections). These side effects can limit the use of such agents, particularly for chronic diseases where the agent is administered over a long period. (Kollias G. and Kontoyiannis D., Cytokine Growth Factor Rev., 13(4-5):315-321 (2002).) In contrast, because the ligands of the invention specifically antagonize TNFR1, they can be administered over long periods, on a chronic basis, with reduced risk of side effects and provide advantages for treating inflammatory conditions and chronic inflammatory conditions (including long duration diseases characterized by periods of quiescence and periods of active inflammation, such as inflammatory bowel disease and arthritis).

### BRIEF DESCRIPTION OF THE DRAWINGS

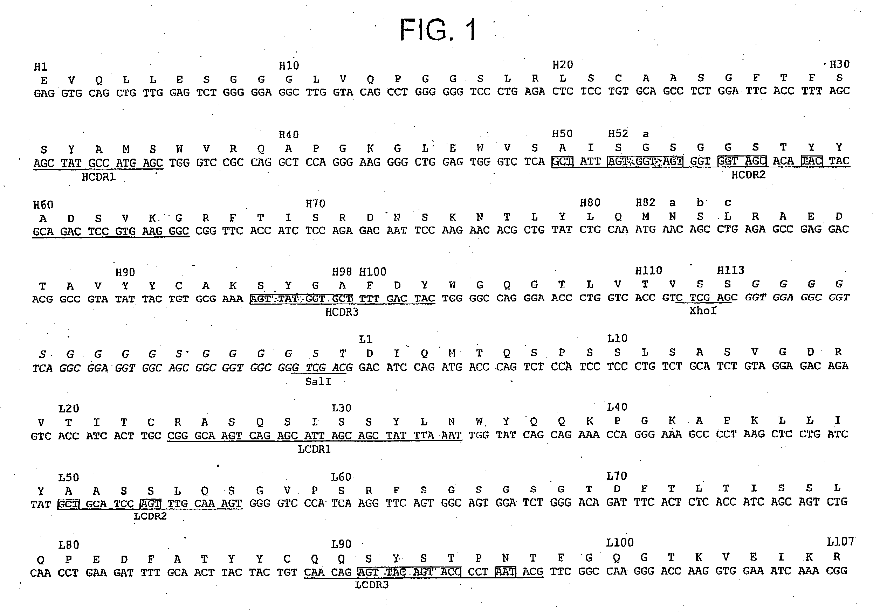

Figure 1 shows the diversification of V_{H}/HSA at positions H50, H52, H52a, H53, H55, H56, H58, H95, H96, H97, H98 (DVT or NNK encoded respectively) which are in the antigen binding site of V_{H} HSA. (SEQ ID NO:1, nucleotide sequence; SEQ ID NO:2, amino acid sequence.) The sequence of V_{K} is diversified at positions L50, L53.

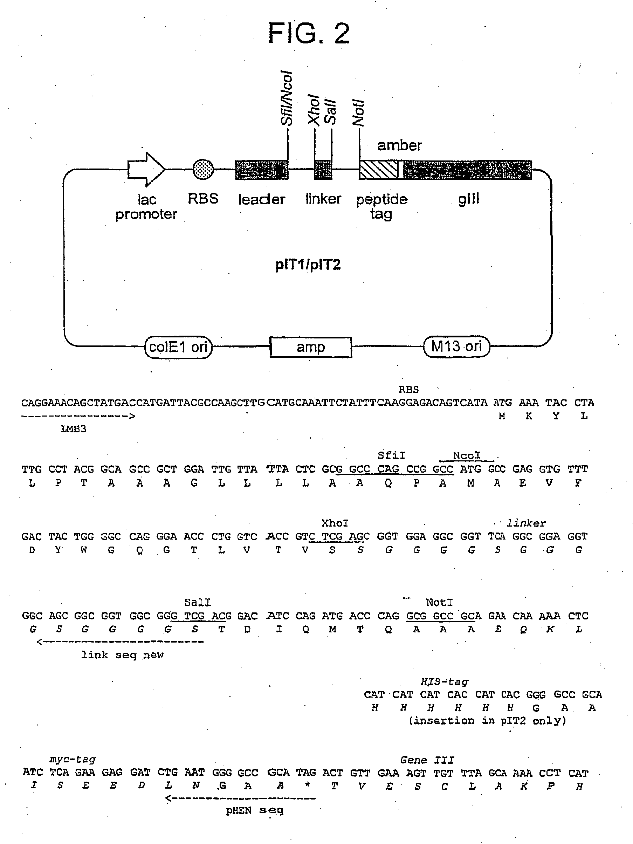

Figure 2 is a schematic showing the structure of the plasmid pIT1/pIT2 used to prepare single chain Fv (scFv) libraries, and shows the nucleotide sequence of the plasmid across the expression control and cloning regions (SEQ ID NO:3) and the encoded amino acid sequence (SEQ ID NO:4). The plasmid was used to prepare
   Library 1: Germline V_{K}/DVT V_{H},
   Library 2: Germline V_{K}/NNK V_{H},
   Library 3: Germline V_{H}/DVT V_{K}, and
   Library 4: Germline V_{H}/NNK V_{K} in phage display/ScFv format.
   These libraries were pre-selected for binding to generic ligands protein A and protein L so that the majority of the clones and selected libraries are functional. Libraries were selected on HSA (first round) and β-gal (second round) or HSA β-gal selection or on β-gal (first round) and HSA (second round) β-gal HSA selection. Soluble scFv from these clones of PCR are amplified in the sequence. One clone encoding a dual specific antibody K8 was chosen for further work.

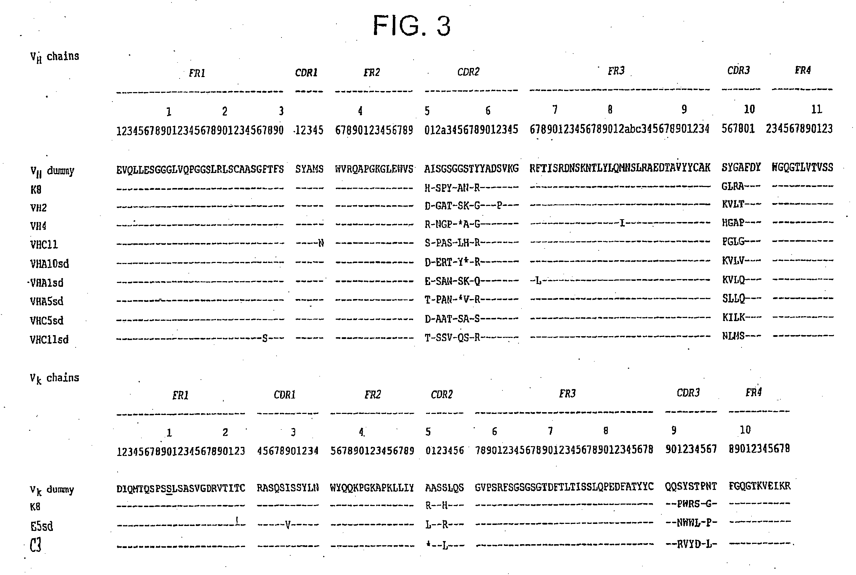

Figure 3 shows an alignment of V_{H} chains (V_{H} dummy (SEQ ID NO:5), K8 (SEQ ID NO:6), VH2 (SEQ ID NO:7), VH4 (SEQ ID NO:8), VHC11 (SEQ ID NO:9), VHA10sd (SEQ ID NO:10), VHA1sd (SEQ ID NO:11), VHA5sd (SEQ ID NO:12), VHC5sd (SEQ ID NO:13), VHC11sd (SEQ ID NO:14), VHC11 sd (SEQ ID NO:15)) and V_{κ} chains (Vk dummy (SEQ ID NO:16), K8 (SEQ ID NO:17), E5sc (SEQ ID NO:18), C3 (SEQ ID NO:19)).

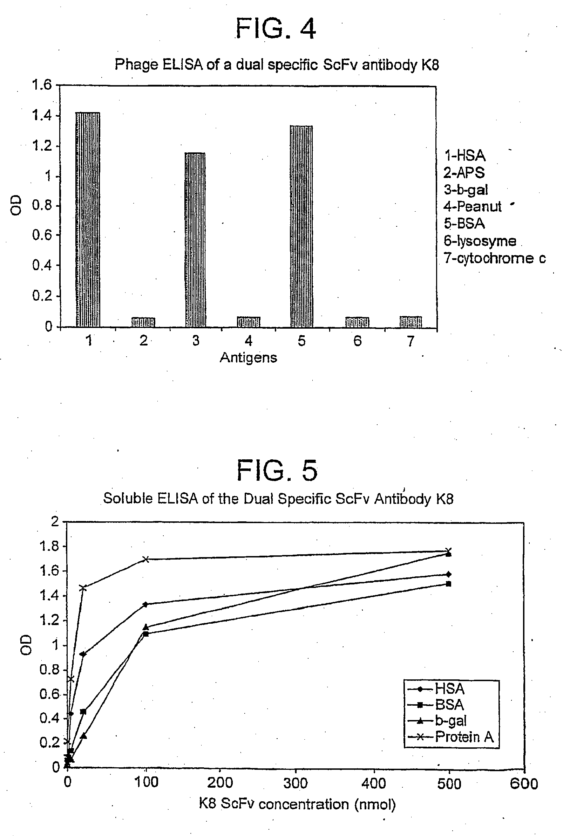

Figure 4 shows the characterisation of the binding properties of the K8 antibody, the binding properties of the K8 antibody characterised by monoclonal phage ELISA, the dual specific K8 antibody was found to bind HSA and β-gal and displayed on the surface of the phage with absorbant signals greater than 1.0. No cross reactivity with other proteins was detected.
Figure 5 shows soluble scFv ELISA performed using known concentrations of the K8 antibody fragment. A 96-well plate was coated with 100µg of HSA, BSA and β-gal at 10µg/ml and 100µg/ml of Protein A at 1µg/ml concentration. 50µg of the serial dilutions of the K8 scFv was applied and the bound antibody fragments were detected with Protein L-HRP. ELISA results confirm the dual specific nature of the K8 antibody.

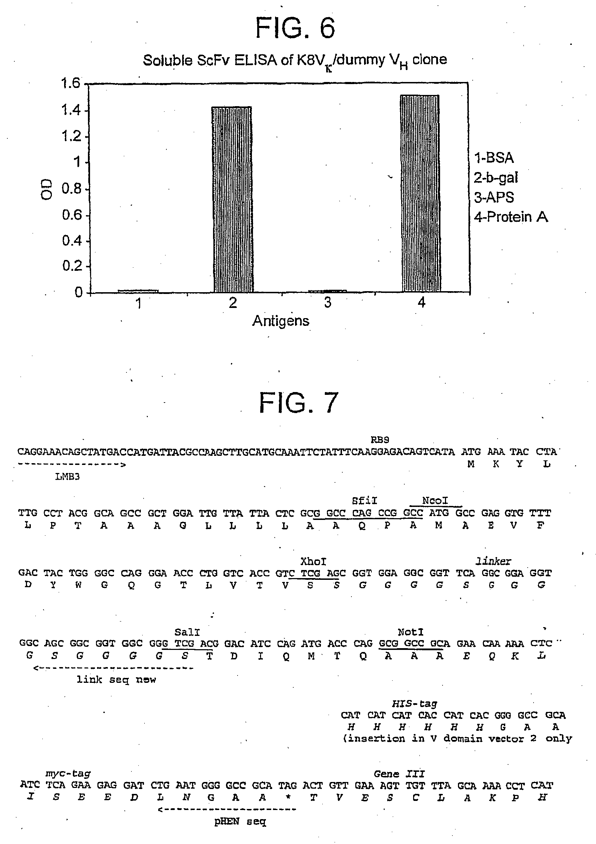

Figure 6 shows the binding characteristics of the clone K8V_{K}/dummy V_{H} analysed using soluble scFv ELISA. Production of the soluble scFv fragments was induced by IPTG as described by Harrison et al, Methods Enzymol. 1996;267:83-109 and the supernatant containing scFv assayed directly. Soluble scFv ELISA is performed as described in example 1 and the bound scFvs were detected with Protein L-HRP. The ELISA results revealed that this clone was still able to bind β-gal, whereas binding BSA was abolished.
Figure 7 shows the sequence (SEQ ID NO:2 and SEQ ID NO:3) of variable domain vectors 1 and 2.

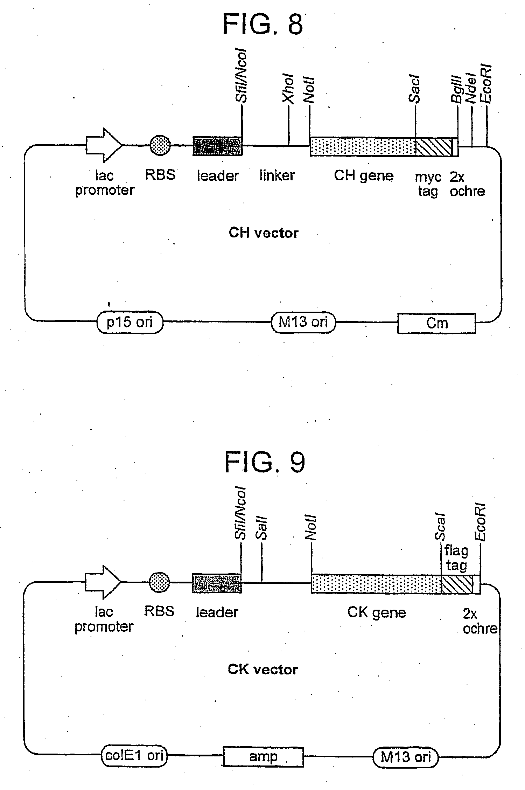

Figure 8 is a map of the C_{H} vector used to construct a V_{H}1/V_{H}2 multipsecific ligand.
Figure 9 is a map of the C_{κ} vector used to construct a V_{κ}1/N_{κ}2 multispecific ligand.

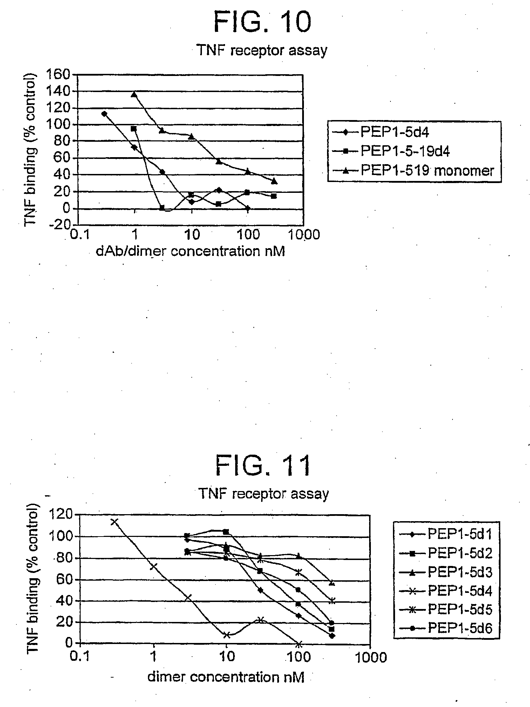

Figure 10 shows a TNF receptor assay comparing TAR1-5 dimer 4, TAR1-5-19 dimer 4 and TAR1-5-19 monomer.
Figure 11 shows a TNF receptor assay comparing TAR1-5 dimers 1-6. All dimers have been FPLC purified and the results for the optimal dimeric species are shown.

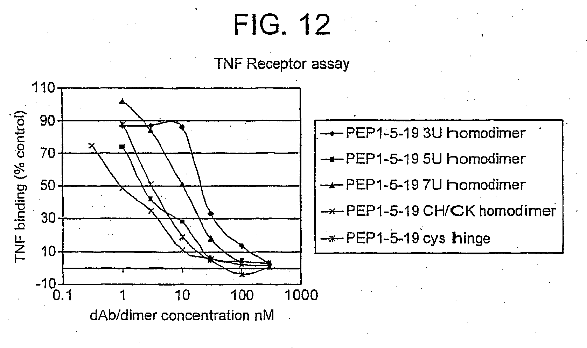

Figure 12 shows a TNF receptor assay of TAR1-5 19 homodimers in different formats: dAb-linker-dAb format with 3U, 5U or 7U linker, Fab format and cysteine hinge linker format.

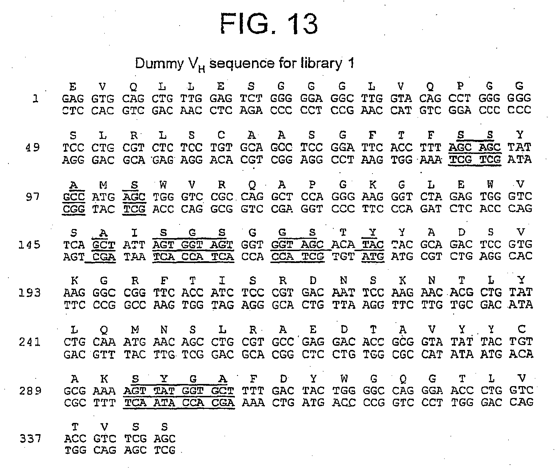

Figure 13 shows Dummy VH sequence for library 1. (amino acid sequence ((SEQ ID NO:5; nucleotide sequences: coding strand (SEQ ID NO:20), noncoding strand (SEQ ID NO:21) The sequence of the VH framework based on germline sequence DP47 - JH4b. Positions where NNK randomisation (N=A or T or C or G nucleotides; K = G or T nucleotides) has been incorporated into library 1 are indicated in bold underlined text.

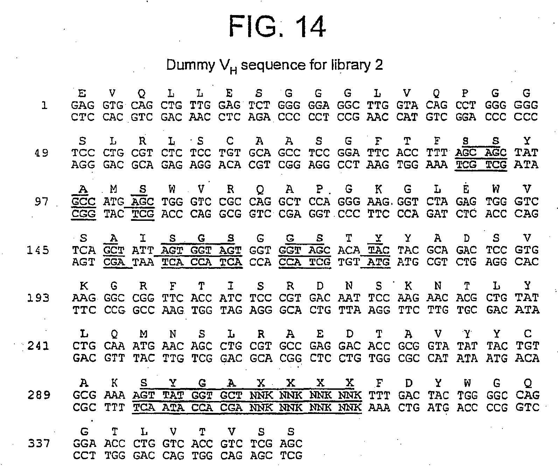

Figure 14 shows Dummy VH sequence for library 2. (amino acid sequence ((SEQ ID NO:22; nucleotide sequences: coding strand (SEQ ID NO:23), noncoding strand (SEQ ID NO:24) The sequence of the VH framework based on germline sequence DP47 - JH4b. Positions where NNK randomisation (N=A or T or C or G nucleotides; K = G or T nucleotides) has been incorporated into library 2 are indicated in bold underlined text.

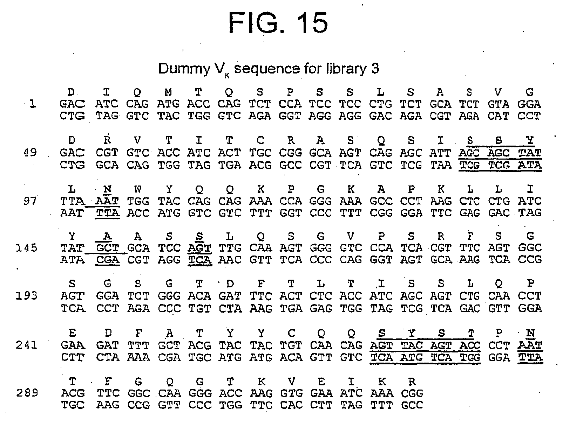

Figure 15 shows Dummy V_{κ} sequence for library 3. (amino acid sequence ((SEQ ID NO:16; nucleotide sequences: coding strand (SEQ ID NO:25), noncoding strand (SEQ ID NO:26) The sequence of the V_{κ} framework based on germline sequence DP_{K}9 - J_{K}1. Positions where NNK randomisation (N=A or T or C or G nucleotides; K = G or T nucleotides) has been incorporated into library 3 are indicated in bold underlined text.

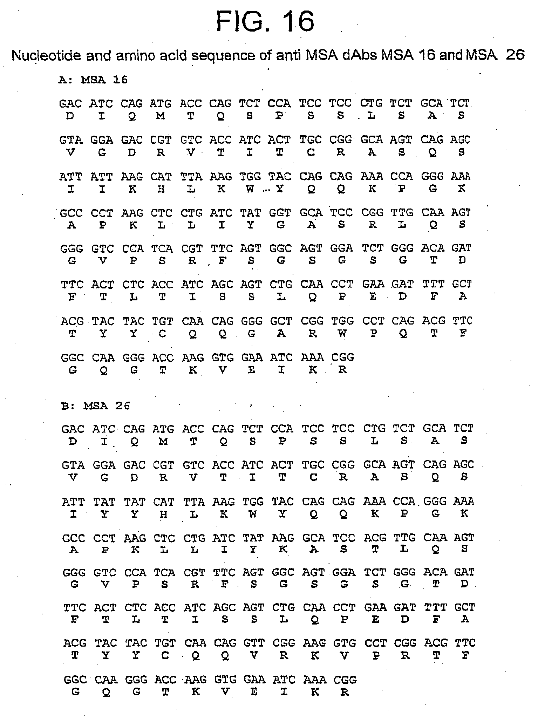

Figure 16 shows nucleotide and amino acid sequence of anti MSA dAbs MSA 16 (nucleotide sequence (SEQ ID NO:27), amino acid sequence (SEQ ID NO:28) and MSA 26 (nucleotide sequence (SEQ ID NO:29), amino acid sequence (SEQ ID NO:30).

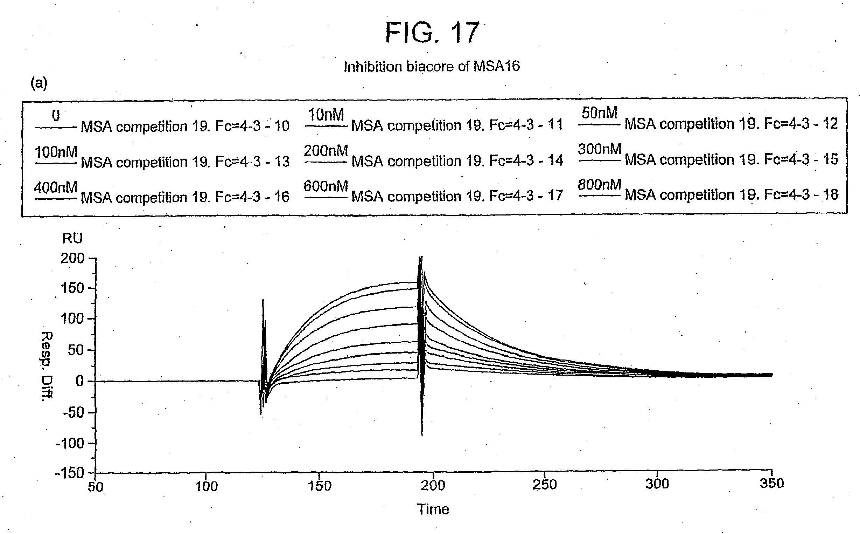

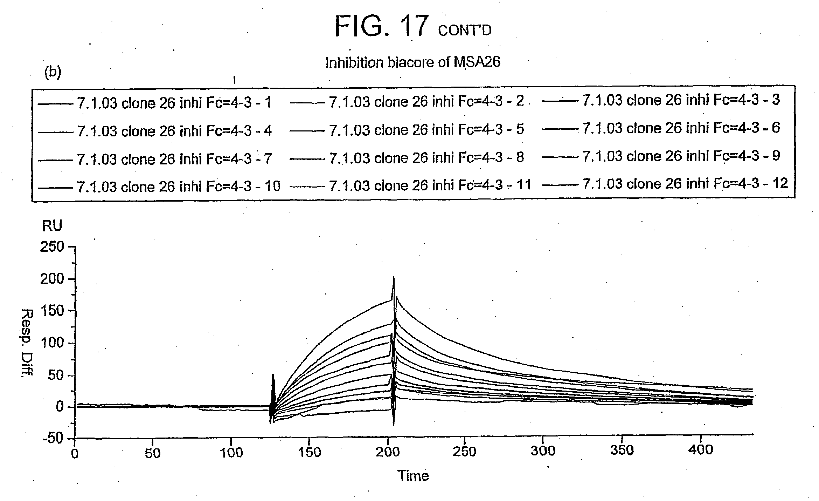

Figure 17 shows inhibition biacore of MSA 16 and 26. Purified dAbs MSA16 and MSA26 were analysed by inhibition biacore to determine K_{d}. Briefly, the dAbs were tested to determine the concentration of dAb required to achieve 200RUs of response on a biacore CM5 chip coated with a high density of MSA. Once the required concentrations of dAb had been determined, MSA antigen at a range of concentrations around the expected K_{d} was premixed with the dAb and incubated overnight. Binding to the MSA coated biacore chip of dAb in each of the premixes was then measured at a high flow-rate of 30 µl/minute.

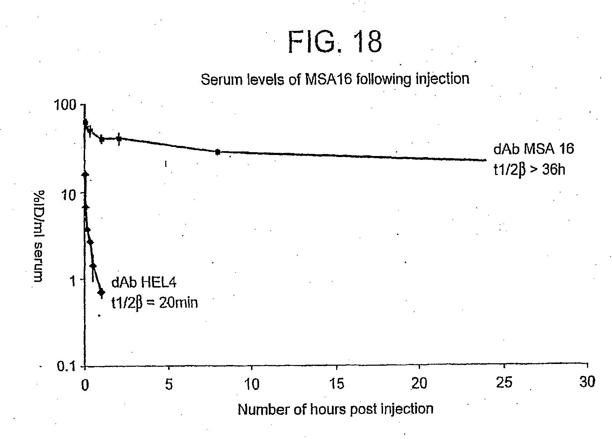

Figure 18 shows serum levels of MSA16 following injection. Serum half life of the dAb MSA16 was determined in mouse. MSA16 was dosed as single i.v. injections at approx 1.5mg/kg into CD1 mice. Modelling with a 2 compartment model showed MSA16 had a t1/2α of 0.98hr, a t1/2β of 36.5hr and an AUC of 913hr.mg/ml. MSA16 had a considerably lengthened half life compared with HEL4 (an anti-hen egg white lysozyme dAb) which had a t1/2α of 0.06hr and a t1/2β of 0.34hr.

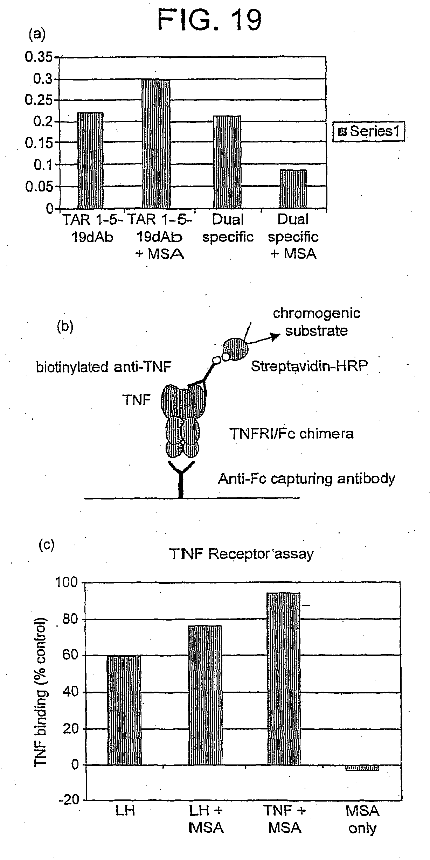

Figures 19a-19c shows an ELISA (Figure 19a) and TNF receptor assay (Figure 19b, 19c) showing inhibition of TNF binding with a Fab-like fragment comprising MSA26Ck and TAR1-5-19CH. Addition of MSA with the Fab-like fragment reduces the level of inhibition. An ELISA plate coated with 1µg/ml TNFα was probed with dual specific V_{κ} C_{H} and V_{κ} C_{κ} Fab like fragment and also with a control TNFα binding dAb at a concentration calculated to give a similar signal on the ELISA. Both the dual specific and control dAb were used to probe the ELISA plate in the presence and in the absence of 2mg/ml MSA. The signal in the dual specific well was reduced by more than 50% but the signal in the dAb well was not reduced at all (see Figure 19a). The same dual specific protein was also put into the receptor assay with and without MSA and competition by MSA was also shown (see Figure 19c). This demonstrates that binding of MSA to the dual specific is competitive with binding to TNFα.

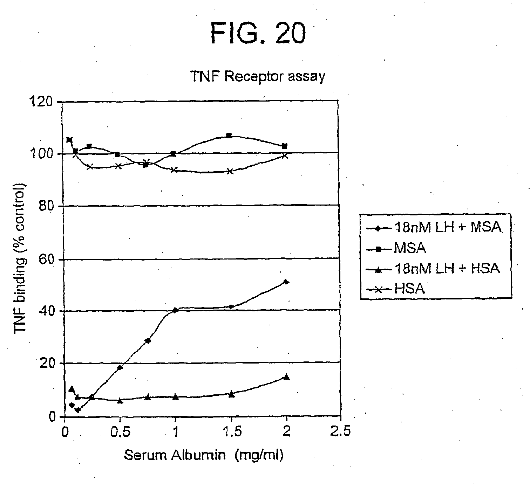

Figure 20 shows a TNF receptor assay showing inhibition of TNF binding with a disulphide bonded heterodimer of TAR1-5-19 dAb and MSA16 dAb. Addition of MSA with the dimer reduces the level of inhibition in a dose dependant manner. The TNF receptor assay (Figure 19 (b)) was conducted in the presence of a constant concentration of heterodimer (18nM) and a dilution series of MSA and HSA. The presence of HSA at a range of concentrations (up to 2 mg/ml) did not cause a reduction in the ability of the dimer to inhibit TNFα. However, the addition of MSA caused a dose dependant reduction in the ability of the dimer to inhibit TNFα (Figure 19a).This demonstrates that MSA and TNFα compete for binding to the cys bonded TAR1-5-19, MSA16 dimer. MSA and HSA alone did not have an effect on the TNF binding level in the assay.

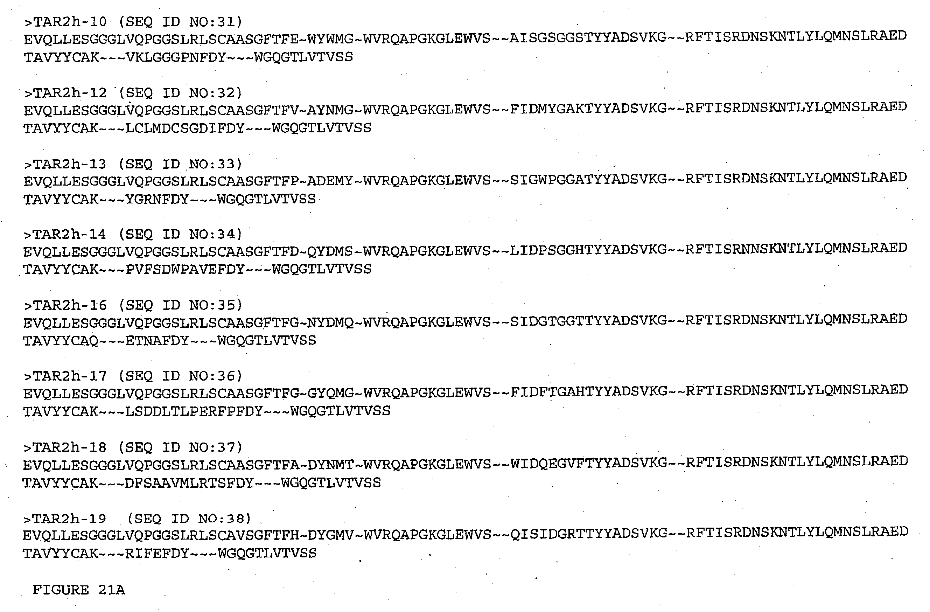

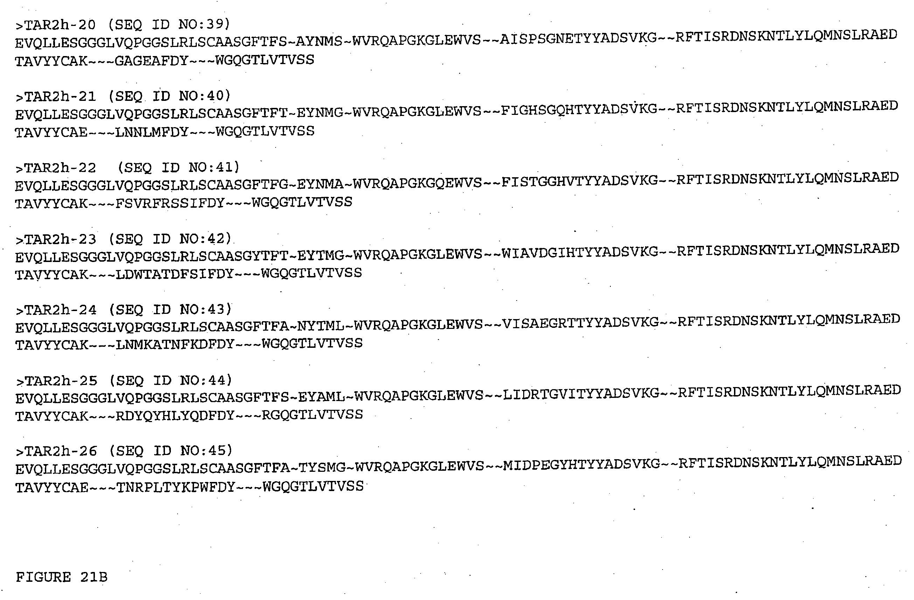

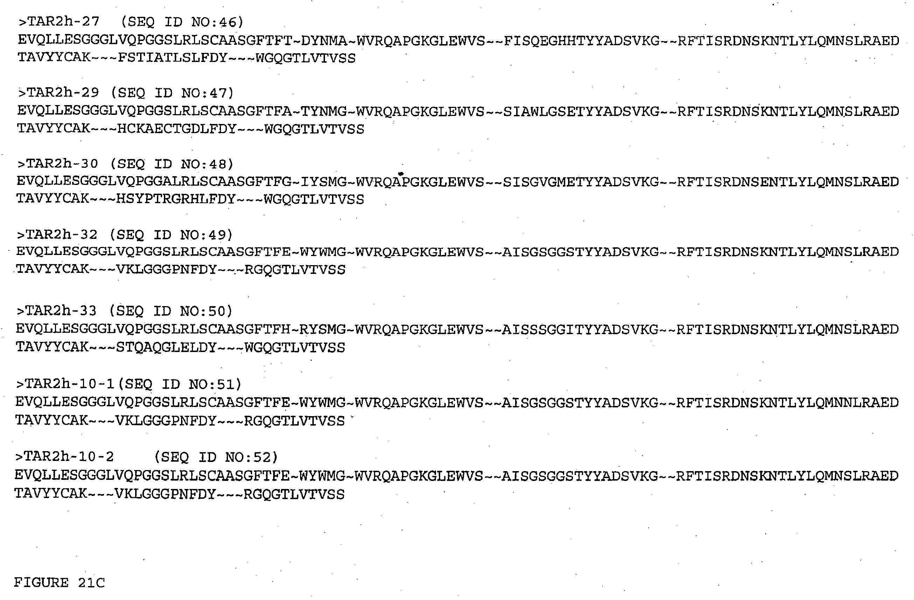

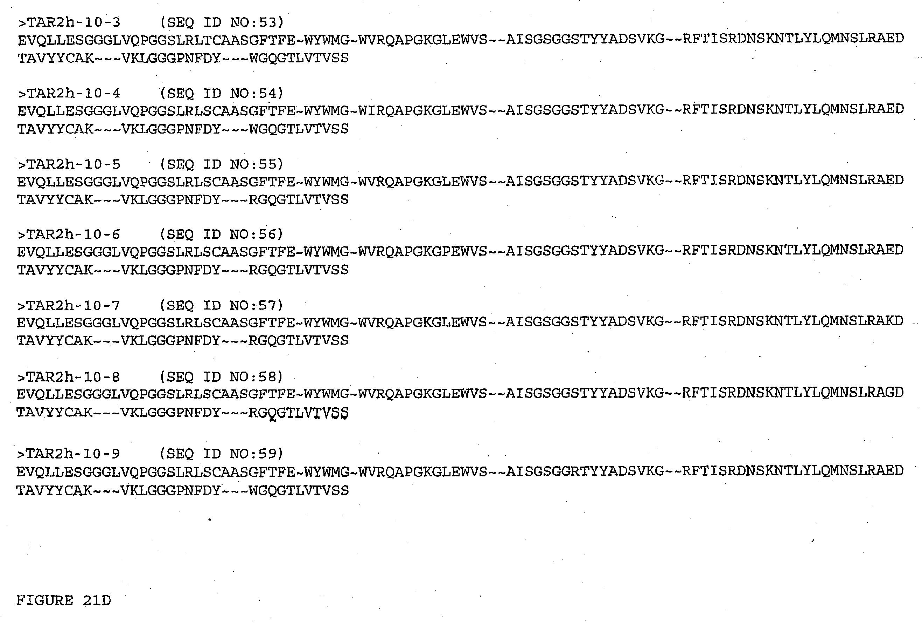

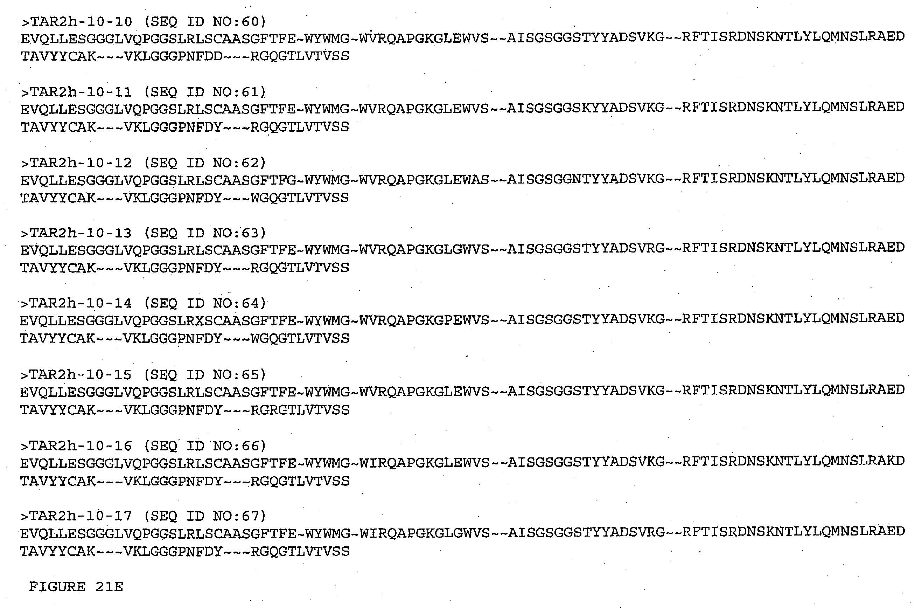

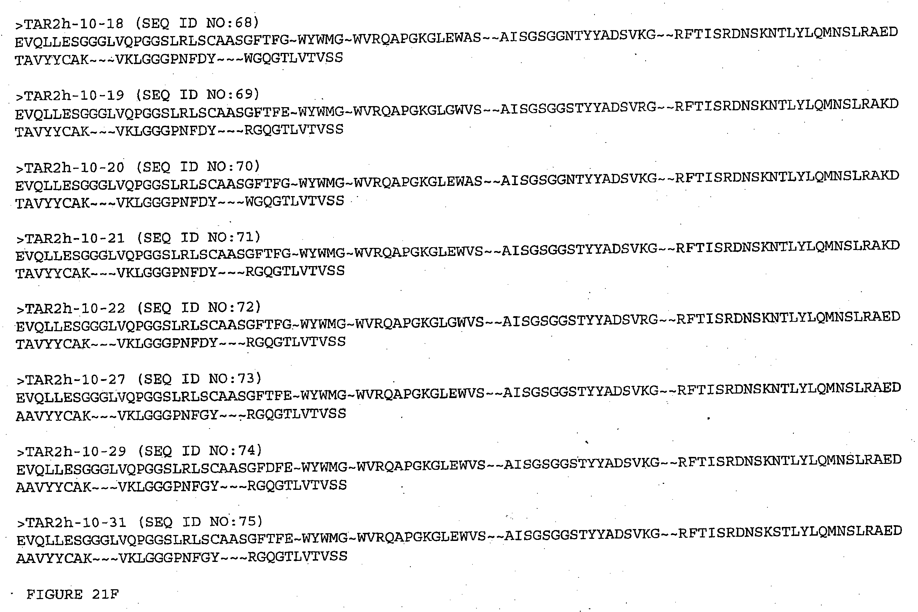

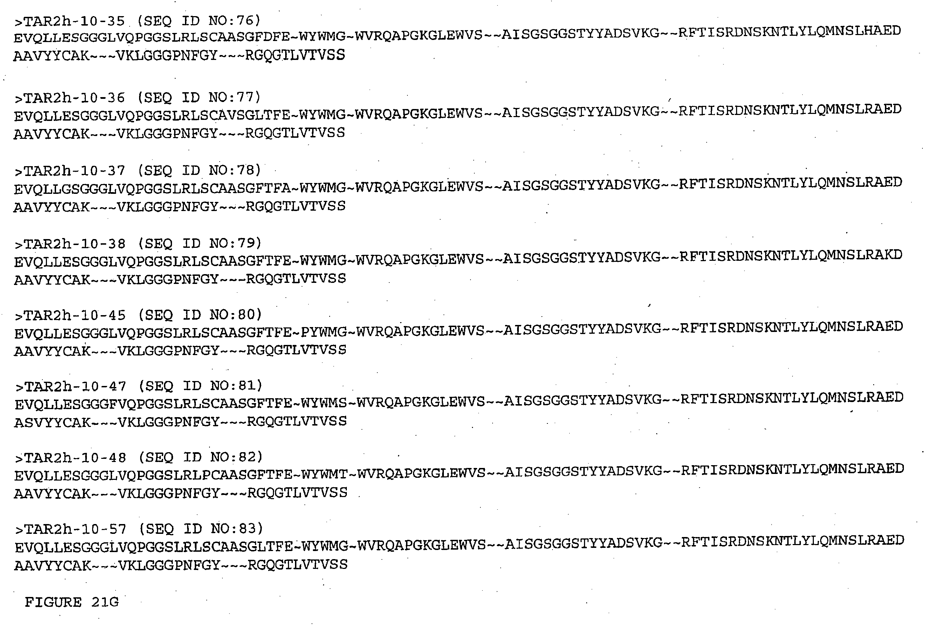

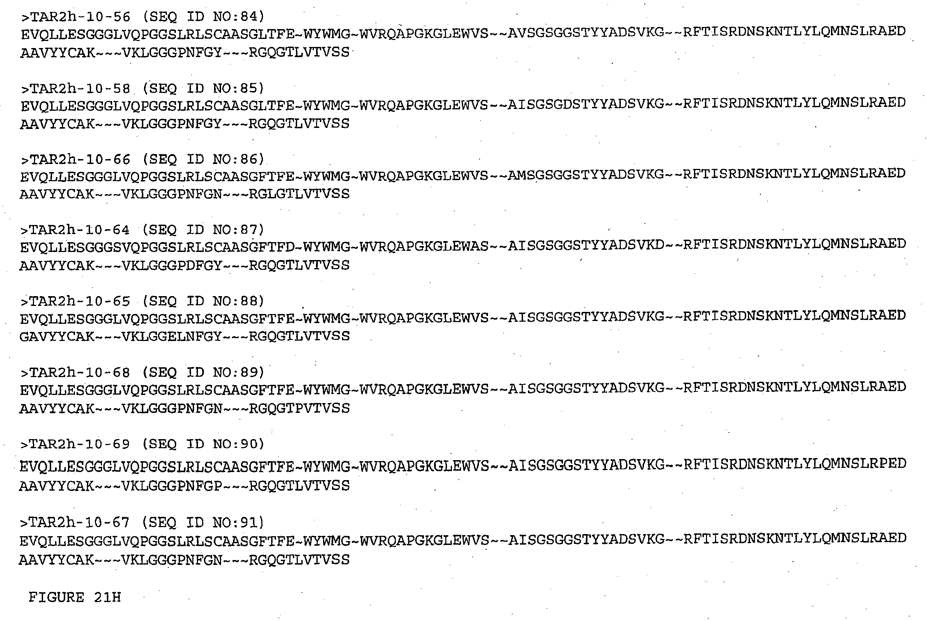

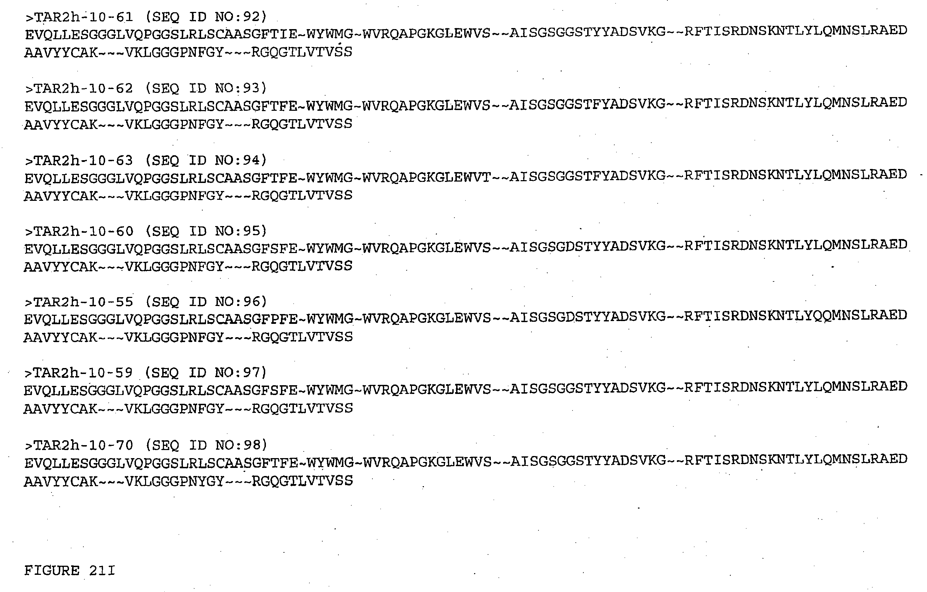

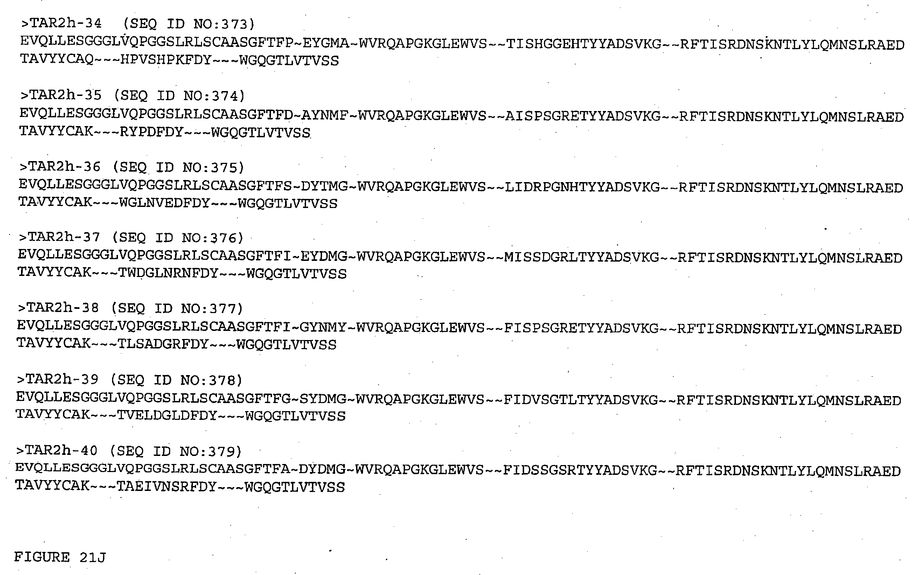

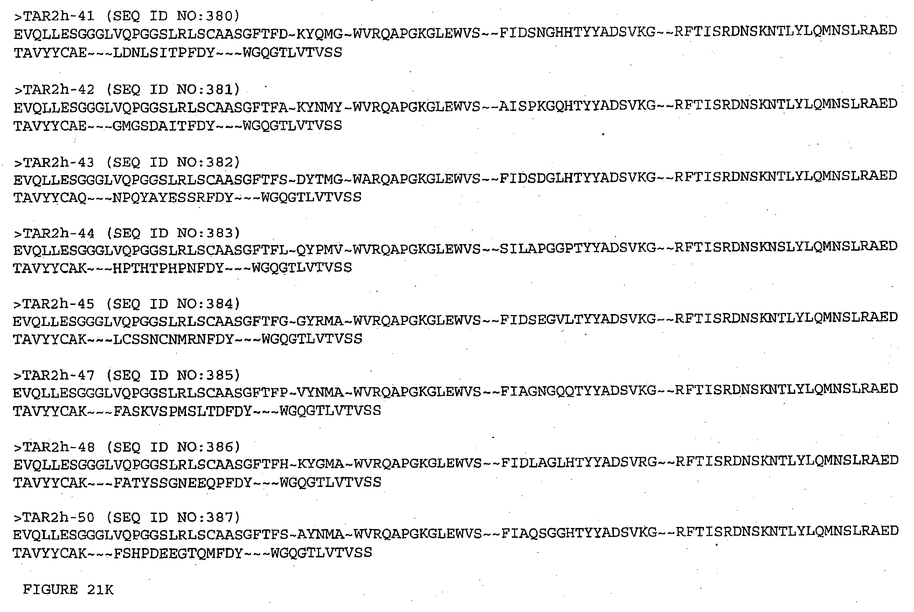

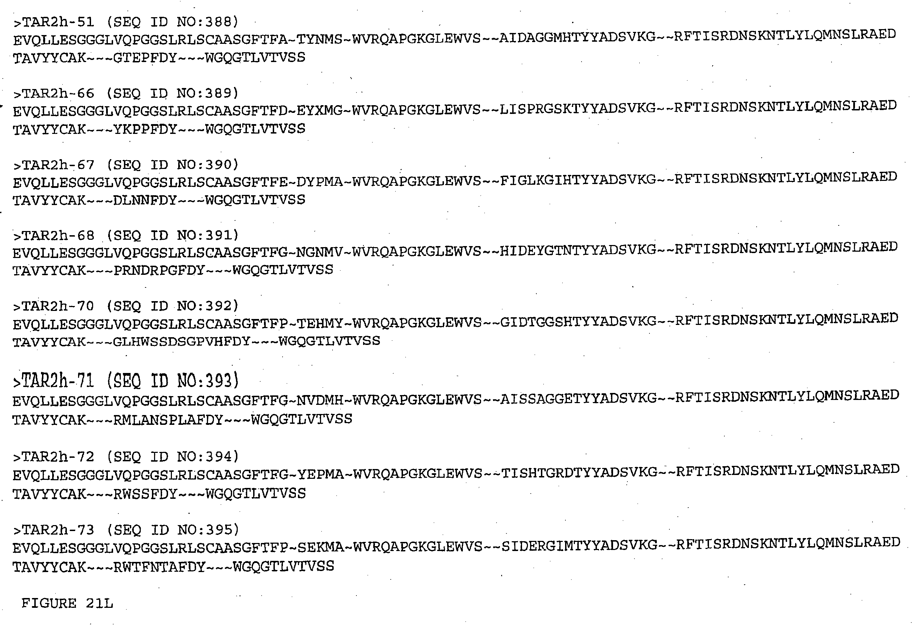

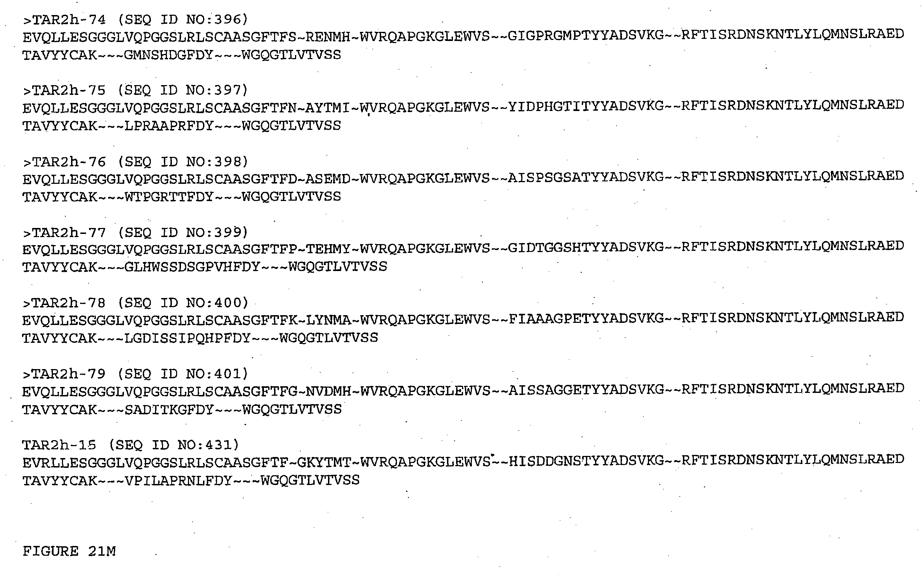

Figure 21A-21M shows the amino acid sequences (SEQ ID NOS:31-98 and SEQ ID NOS:373-401 and 431) of several human immunoglobulin variable domains that have binding specificity for human TNFR1. The presented amino acid sequences are continuous with no gaps; the symbol ~ has been inserted into the sequences to indicate the locations of the complementarity determining regions (CDRs). CDR1 is flanked by ~, CDR2 is flanked by ~~, and CDR3 is flanked by ~~~.

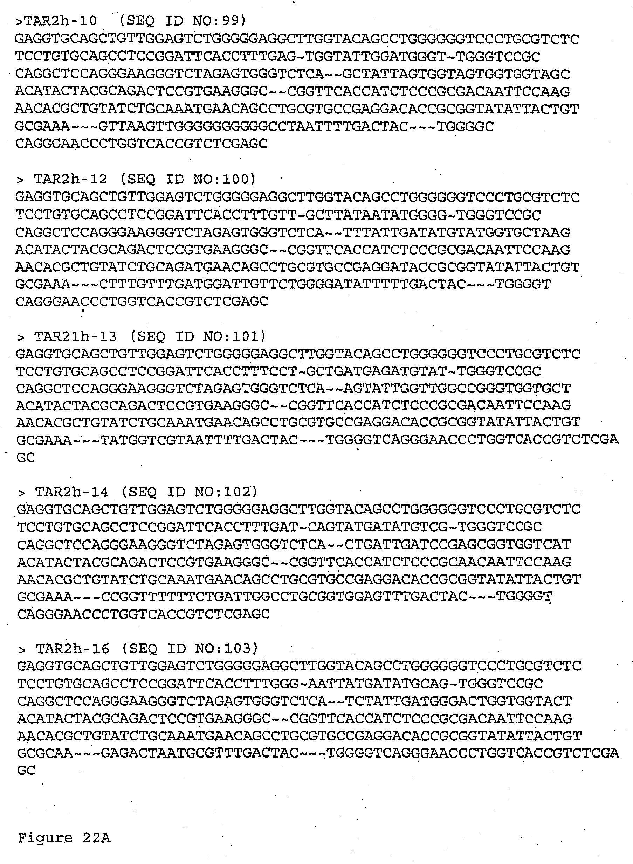

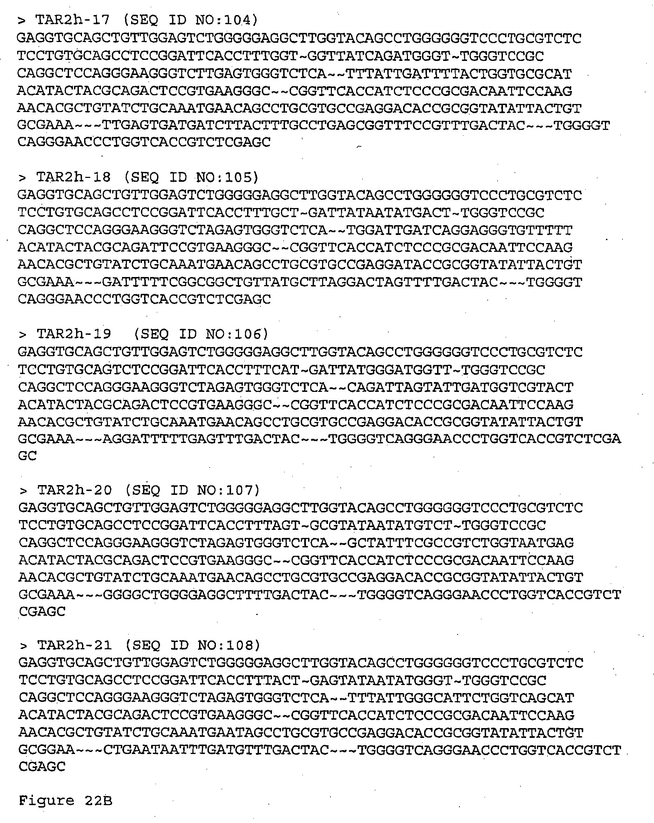

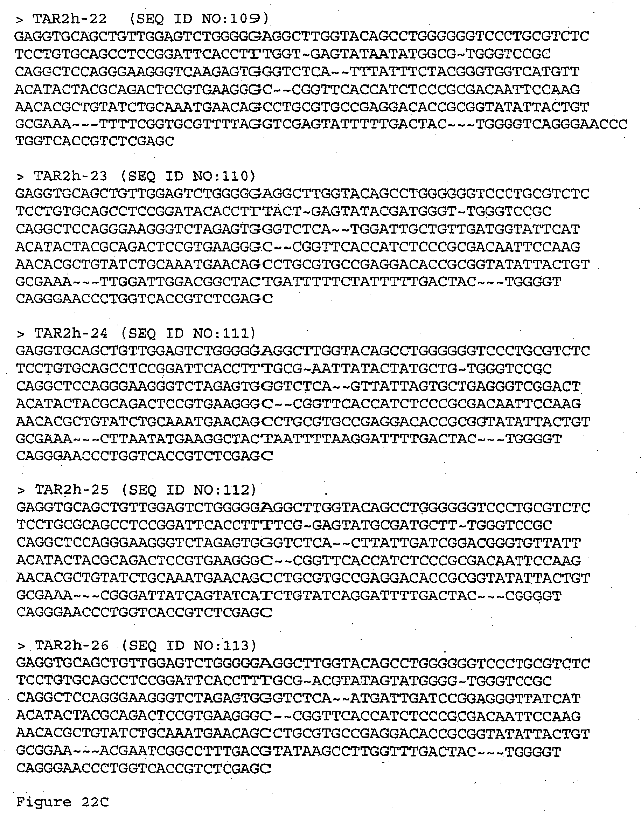

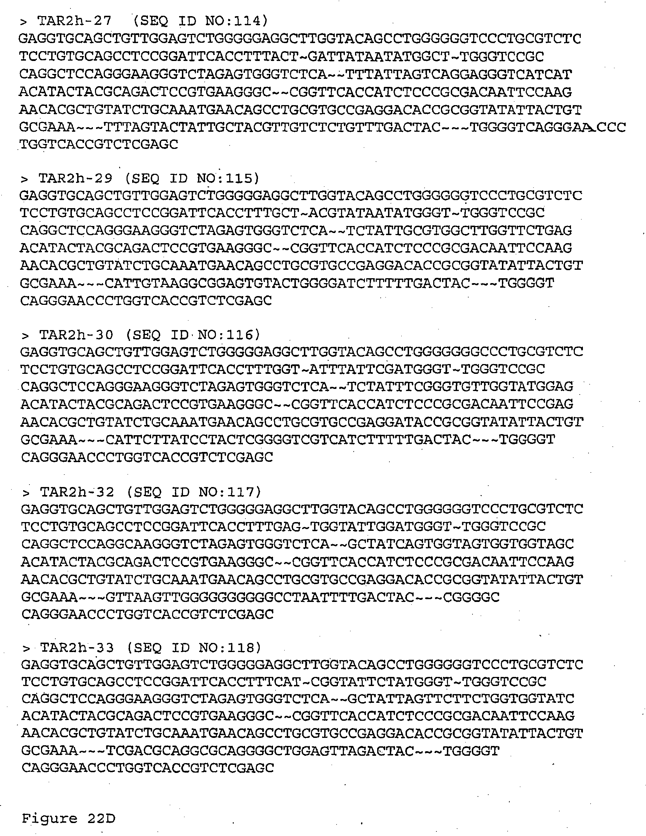

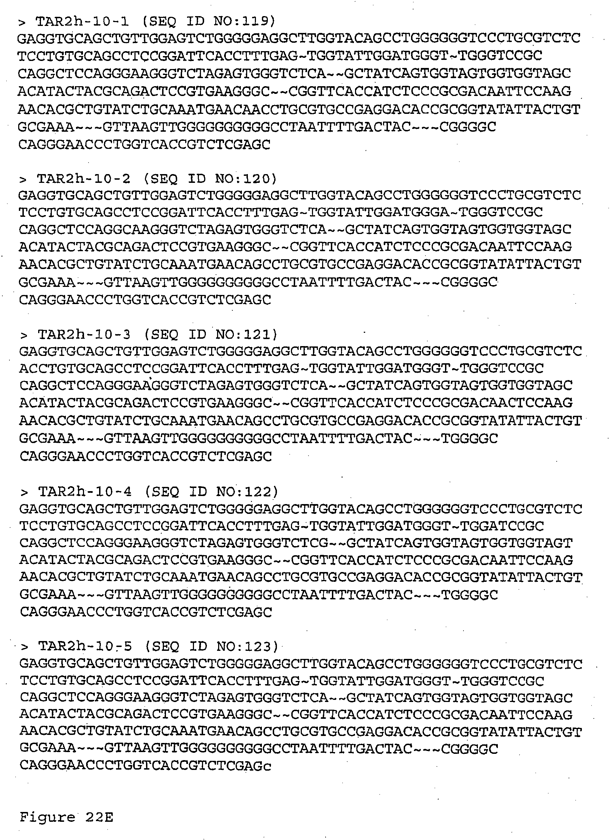

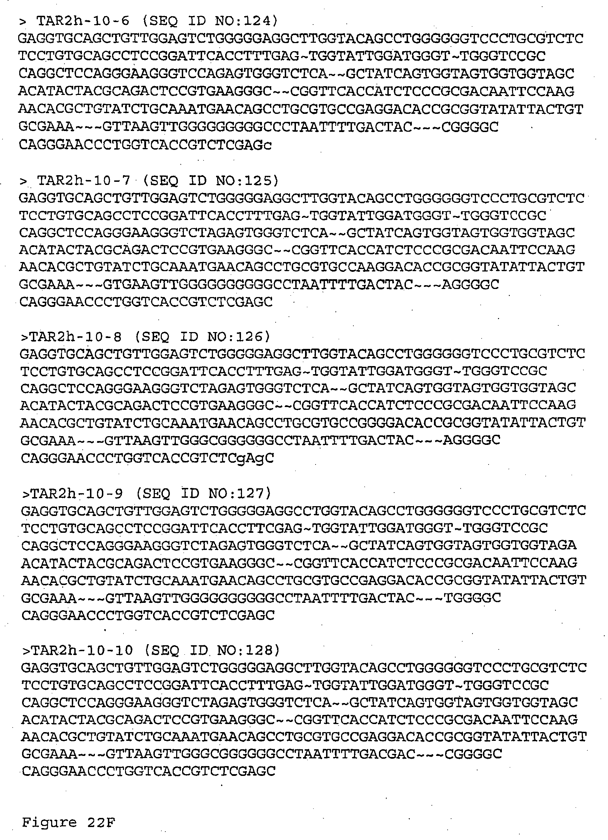

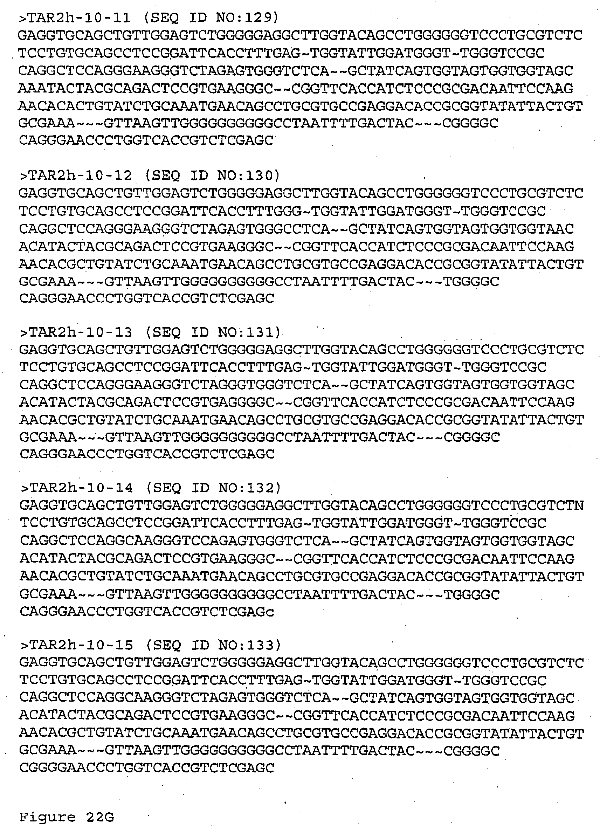

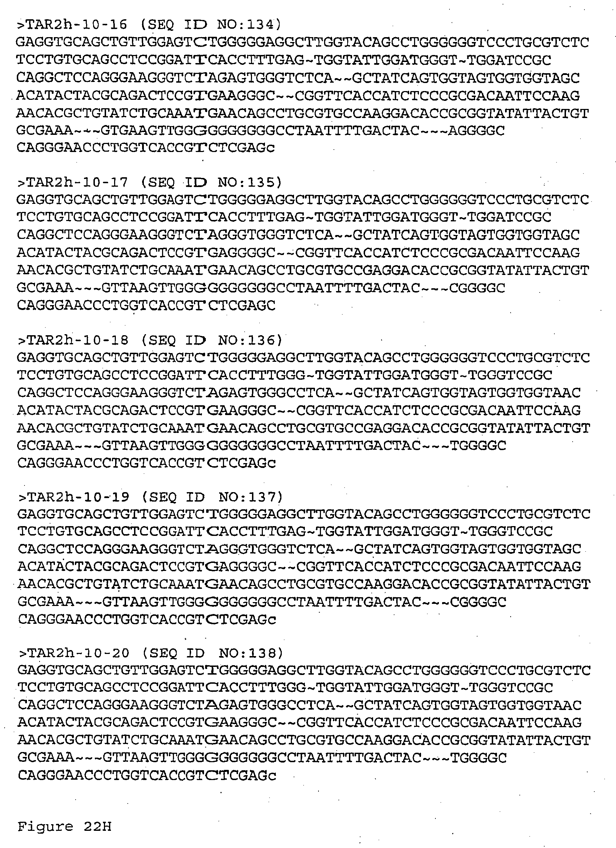

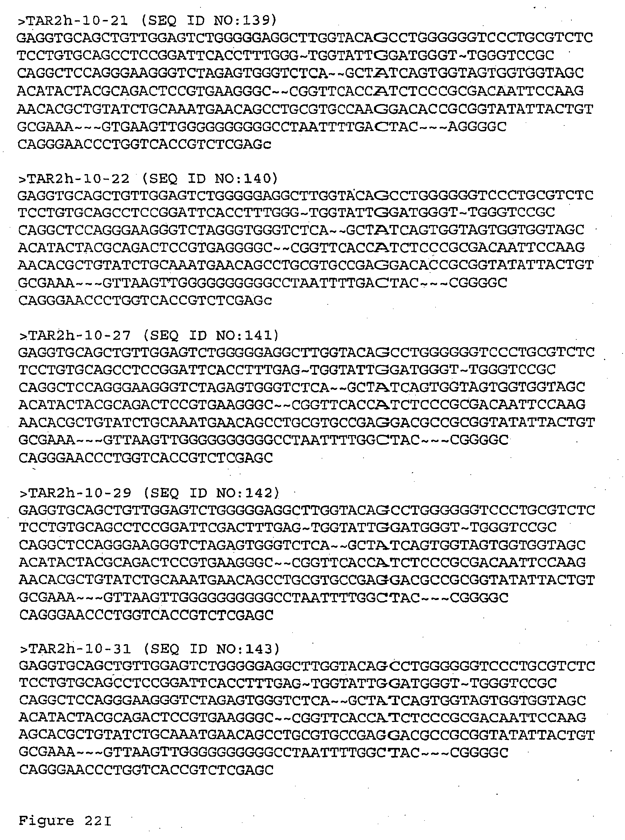

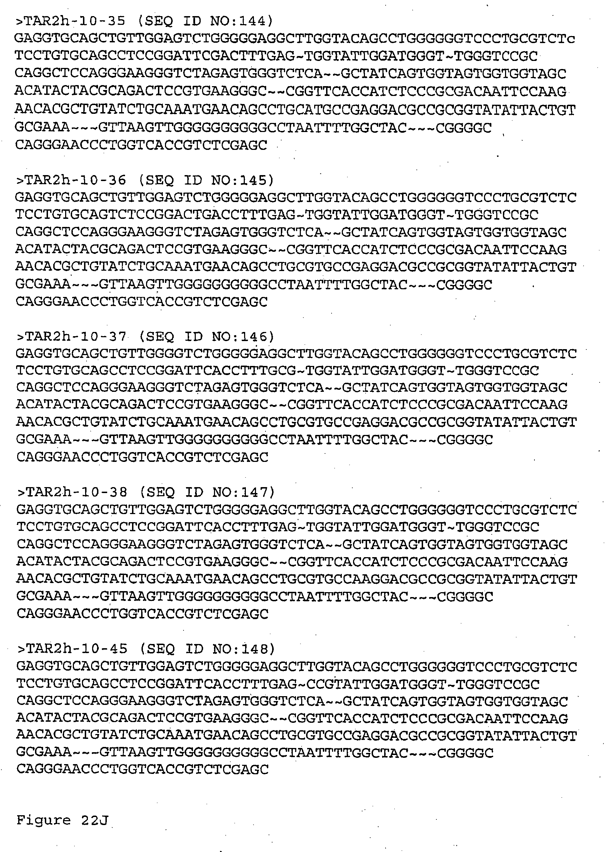

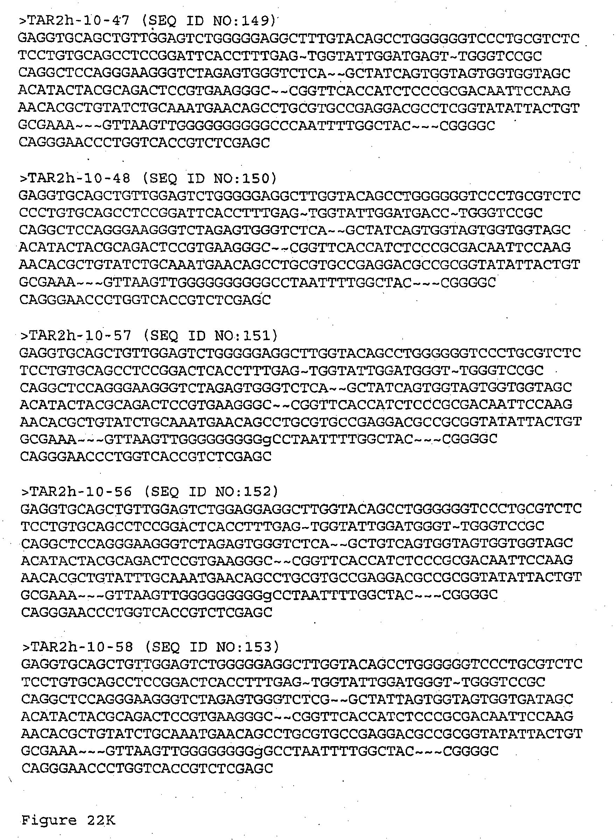

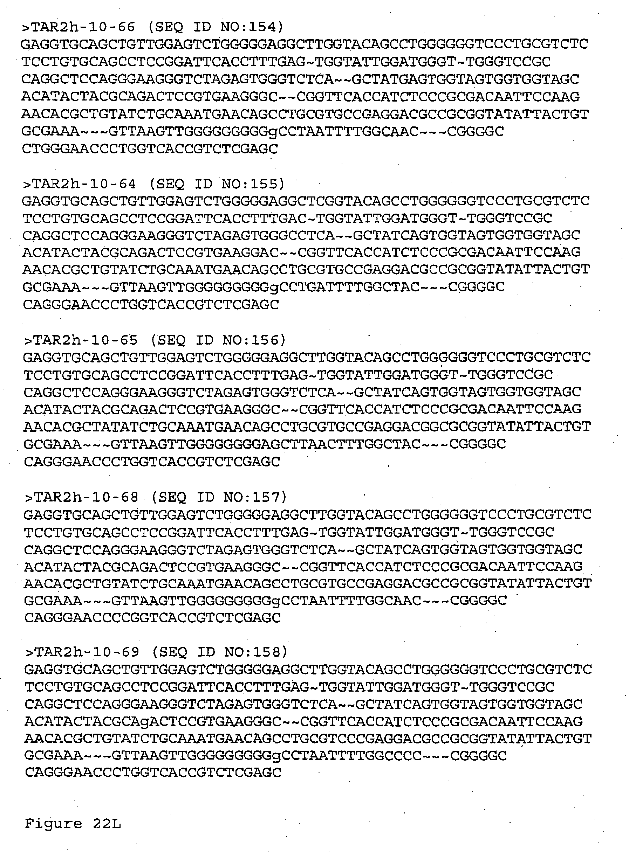

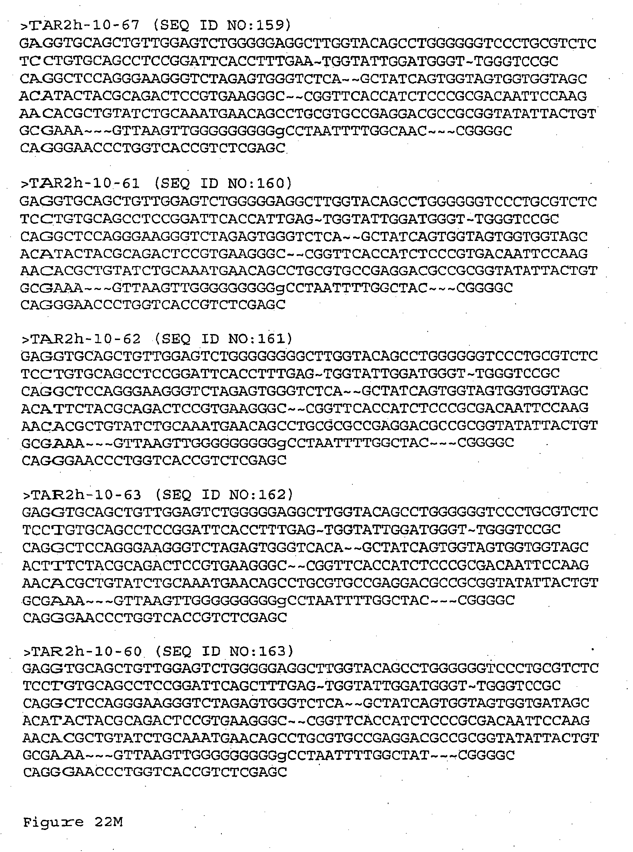

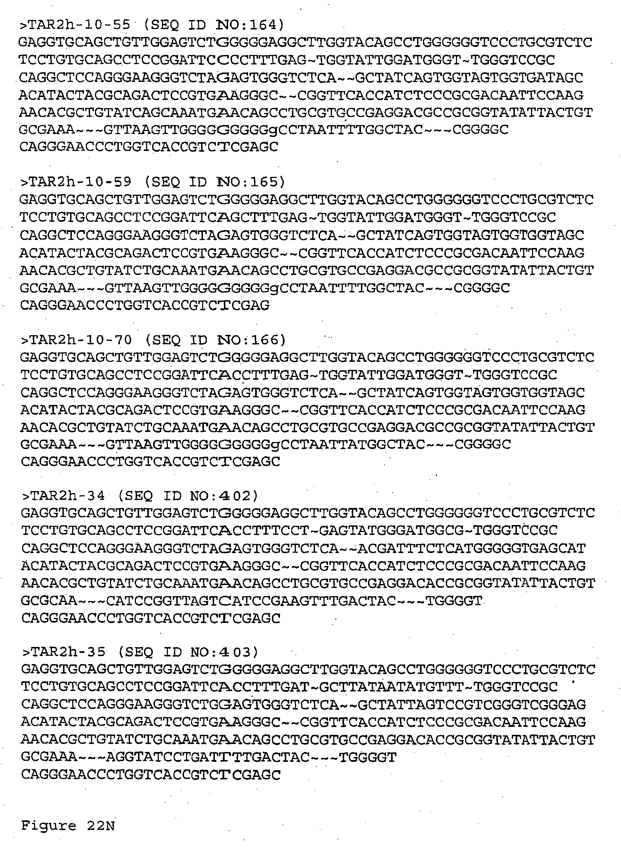

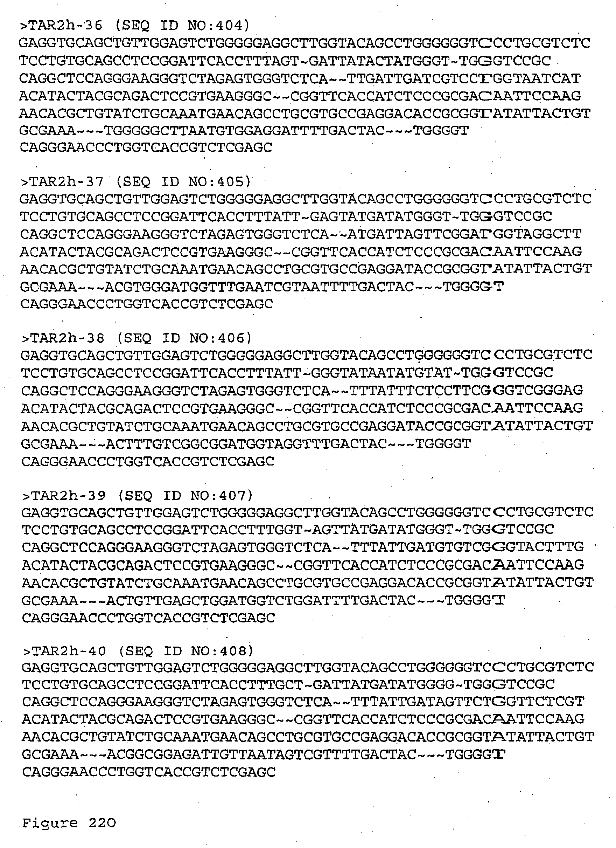

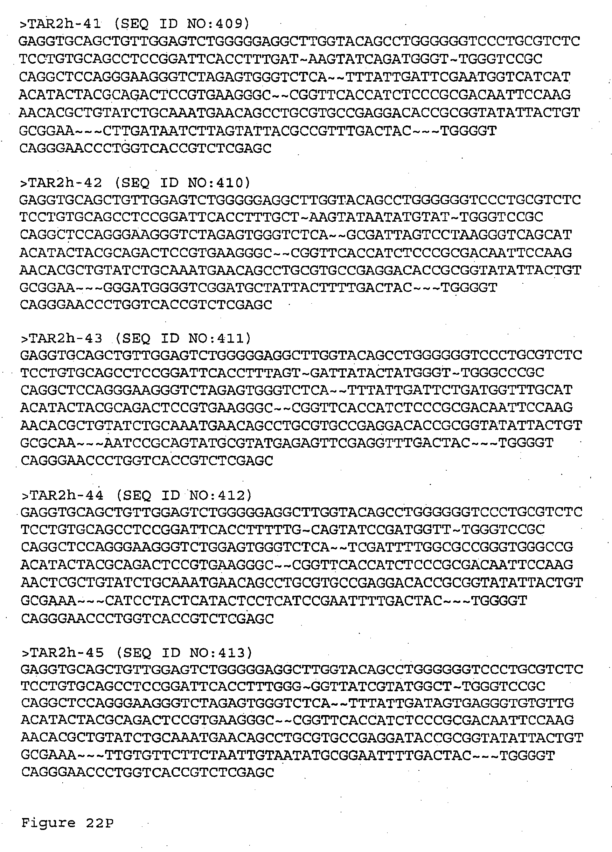

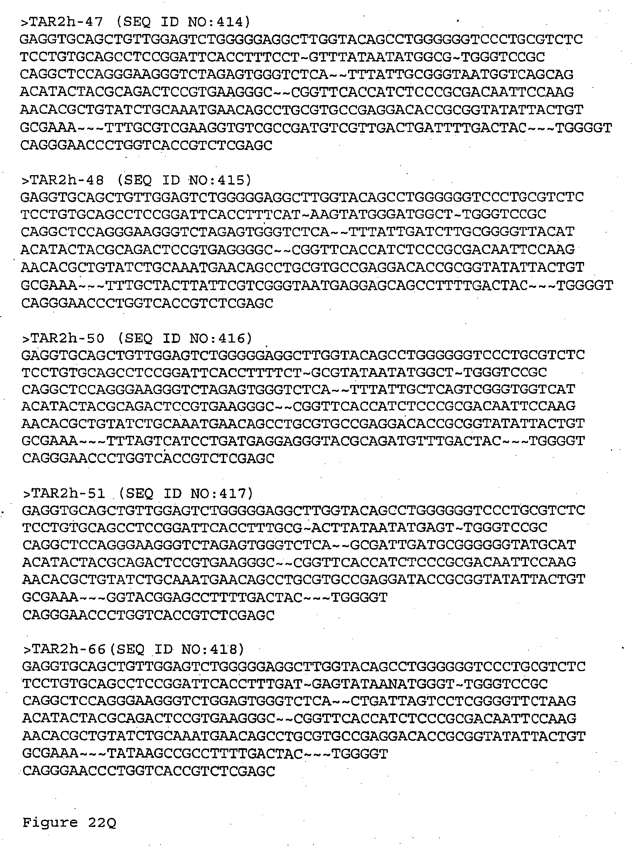

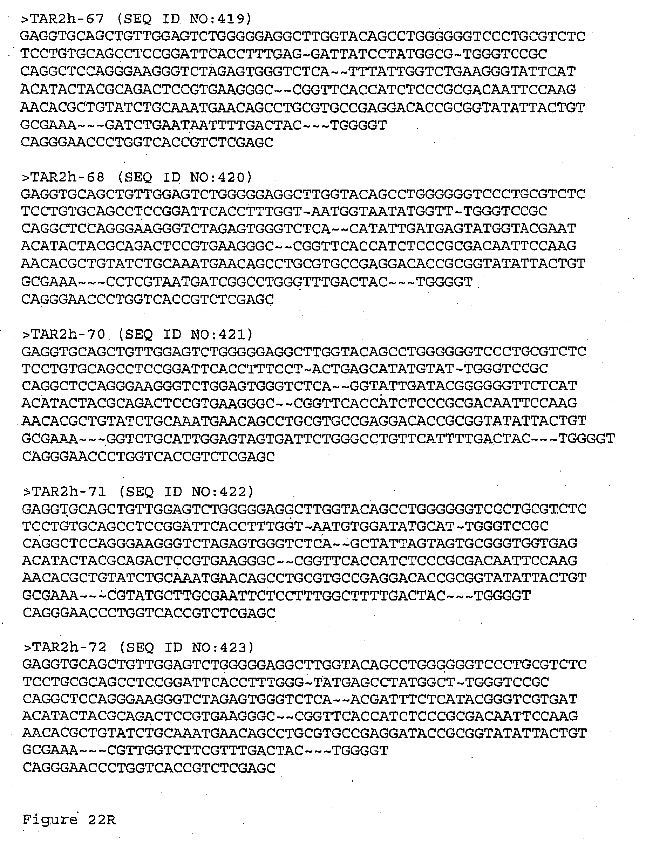

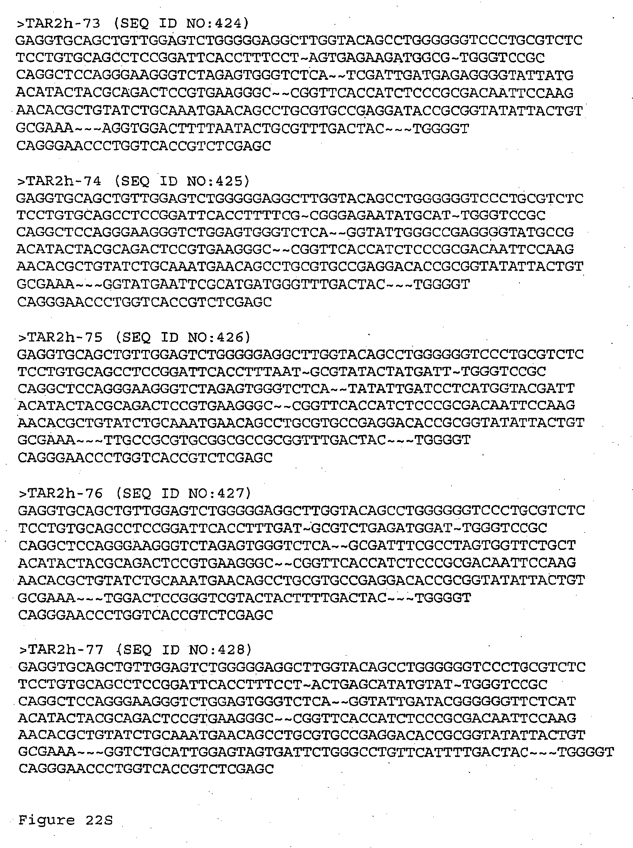

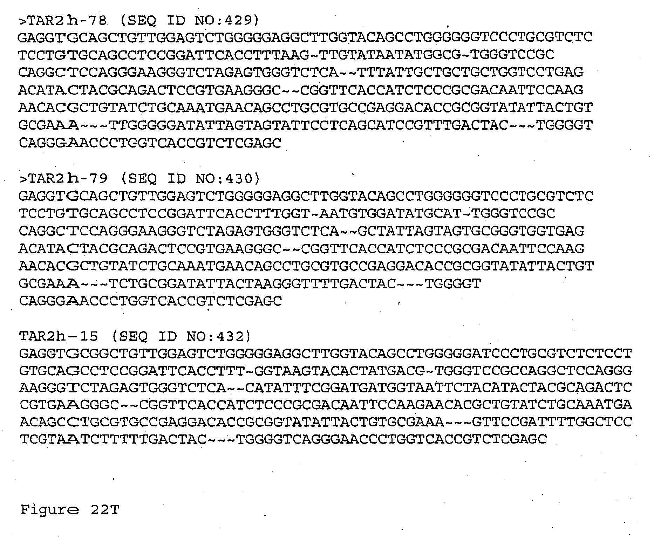

Figure 22A-22T shows the nucleotide sequences (SEQ ID NOS:99-166 and SEQ ID NOS:402-430 and 432) of several nucleic acids that encode the human immunoglobulin variable domains presented in Figure 21A-21M. The presented nucleotide sequences are continuous with no gaps; the symbol - has been inserted into the sequences to indicate the location of the sequences encoding the CDRs. The sequences encoding CDR1 are flanked by ~, the sequences encoding CDR2 are flanked by ~~, and the sequences encoding CDR3 are flanked by ~~~.

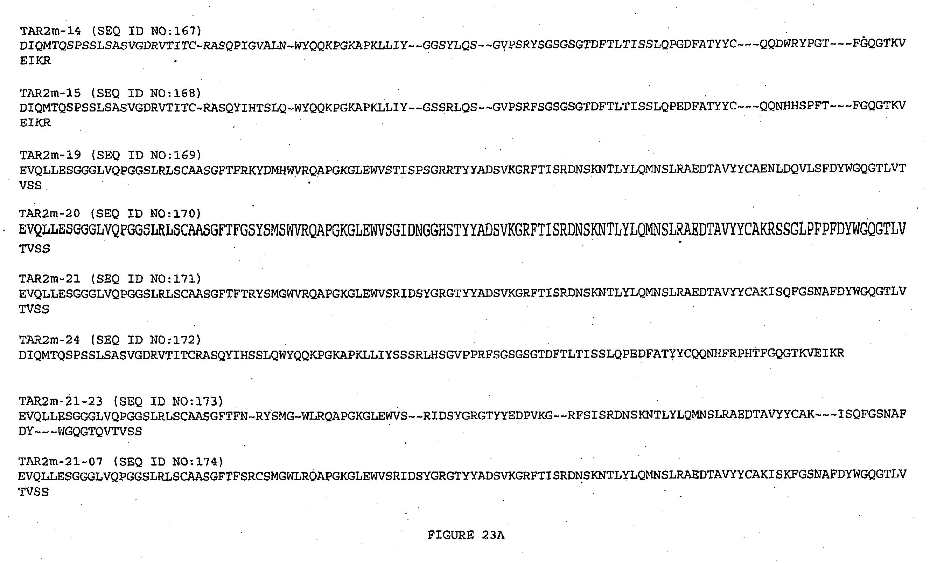

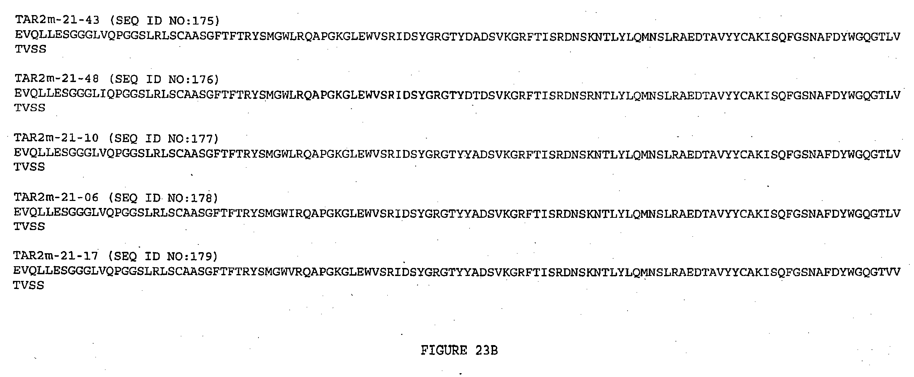

Figure 23A-23B shows the amino acid sequences (SEQ ID NOS:167-179) of several human immunoglobulin variable domains that have binding specificity for mouse TNFR1. The presented amino acid sequences are continuous with no gaps. In some of the sequences the symbol has been inserted to indicate the location of the complementarity determining regions (CDRs). CDR1 is flanked by ~, CDR2 is flanked by ~~, and CDR3 is flanked by ~~~.

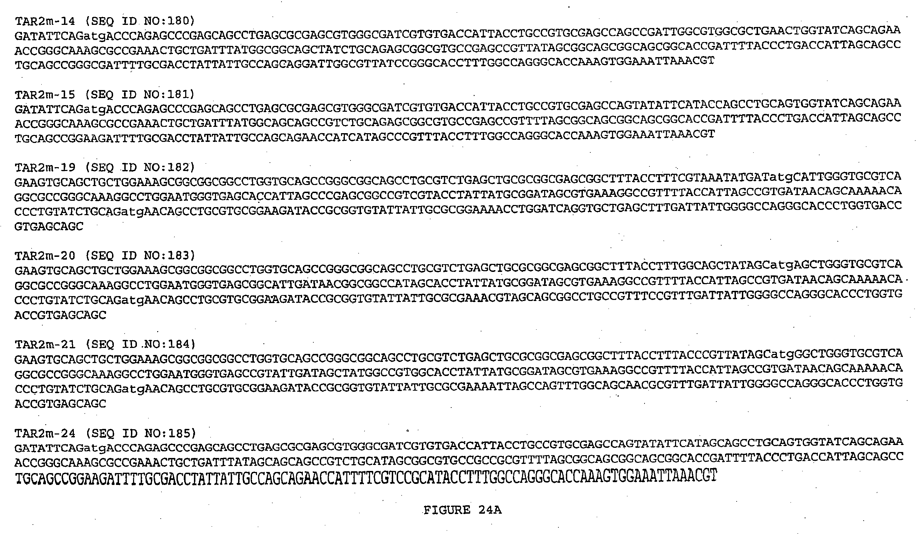

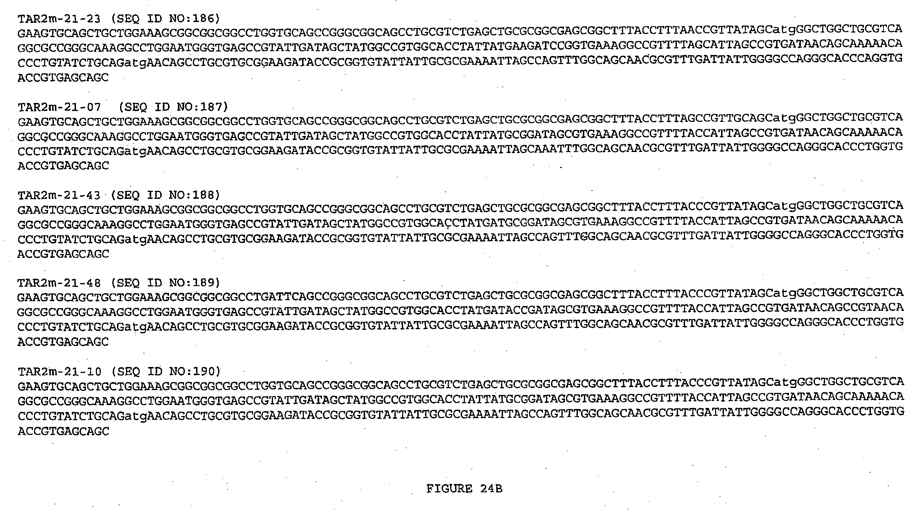

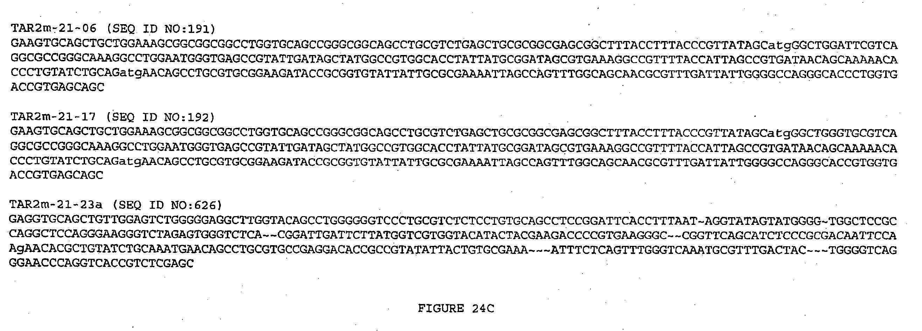

Figure 24A-24C shows the nucleotide sequences (SEQ ID NOS:180-192 and 626) of several nucleic acids that encode the human immunoglobulin variable domains presented in Figure 23A-23B. SEQ ID NO:186 and SEQ ID NO:626 both encode the amino acid sequence of SEQ ID NO:173. The sequences of SEQ ID NO:626 encoding CDR1 are flanked by ~, the sequences encoding CDR2 are flanked by ~~, and the sequences encoding CDR3 are flanked by ~~~.

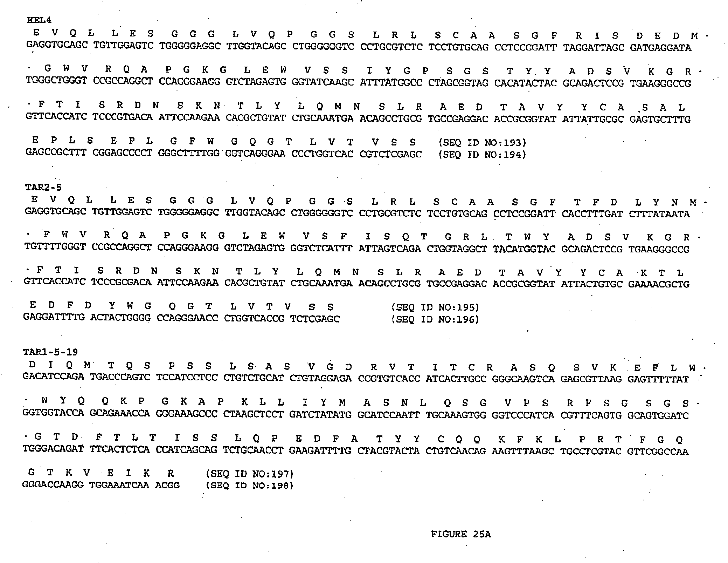

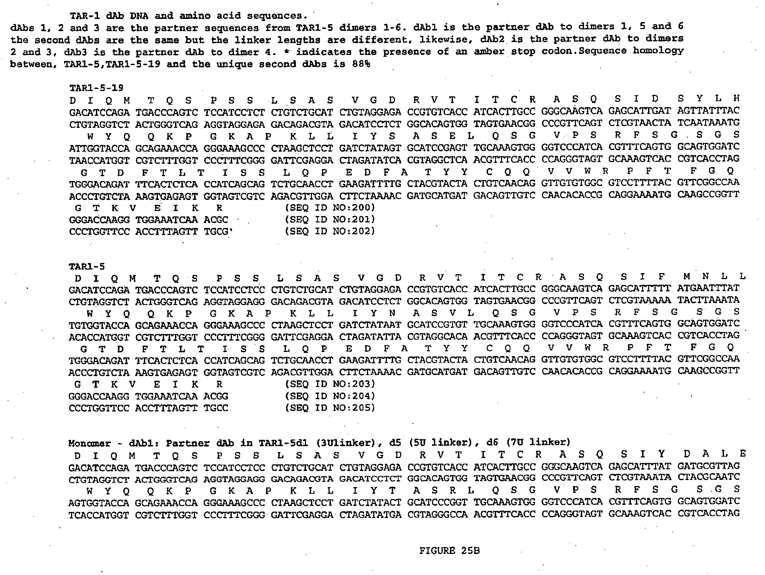

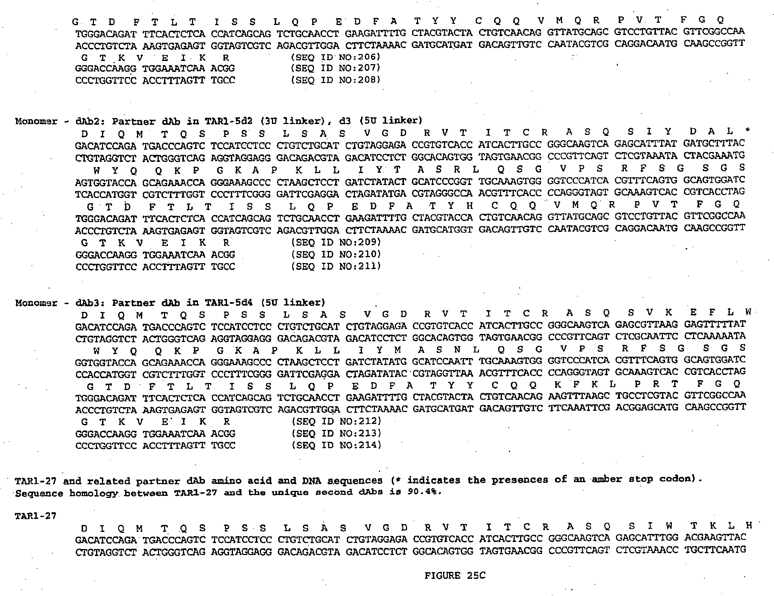

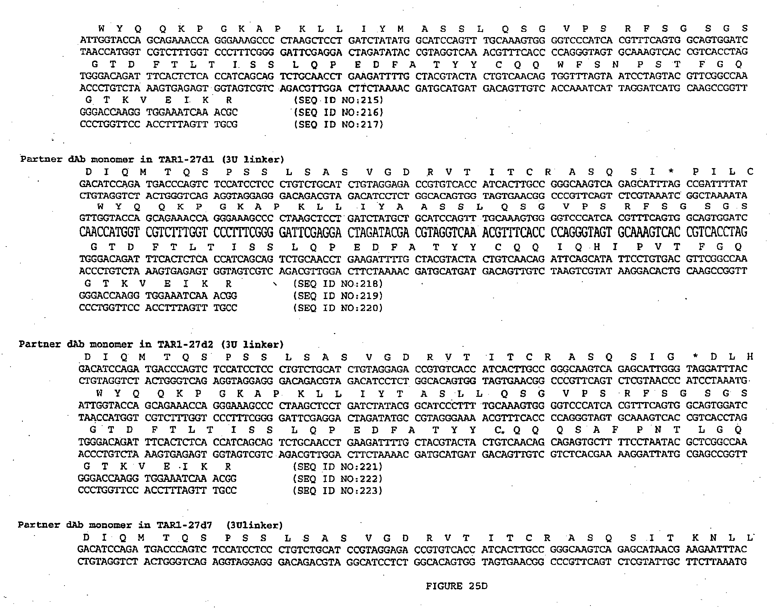

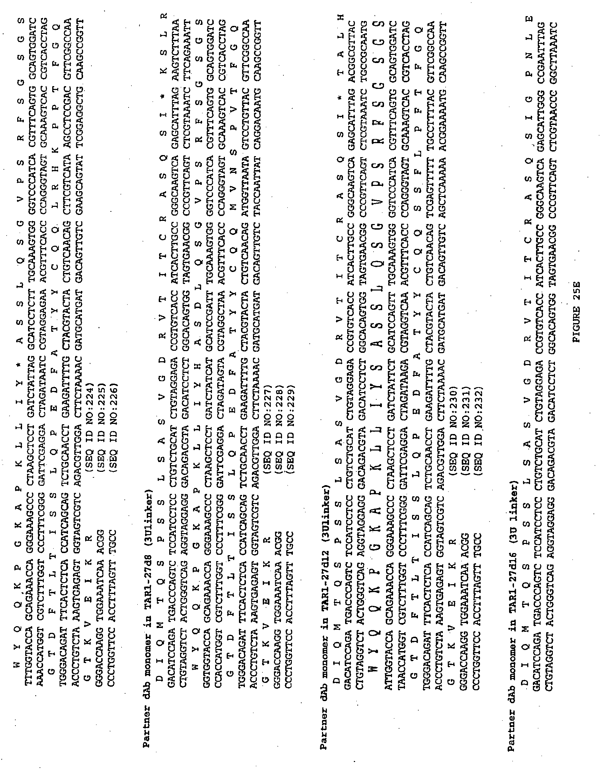

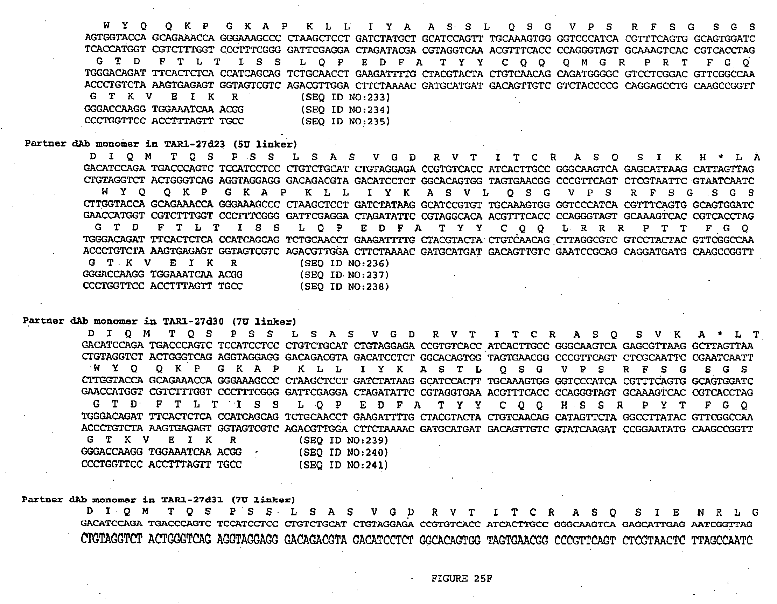

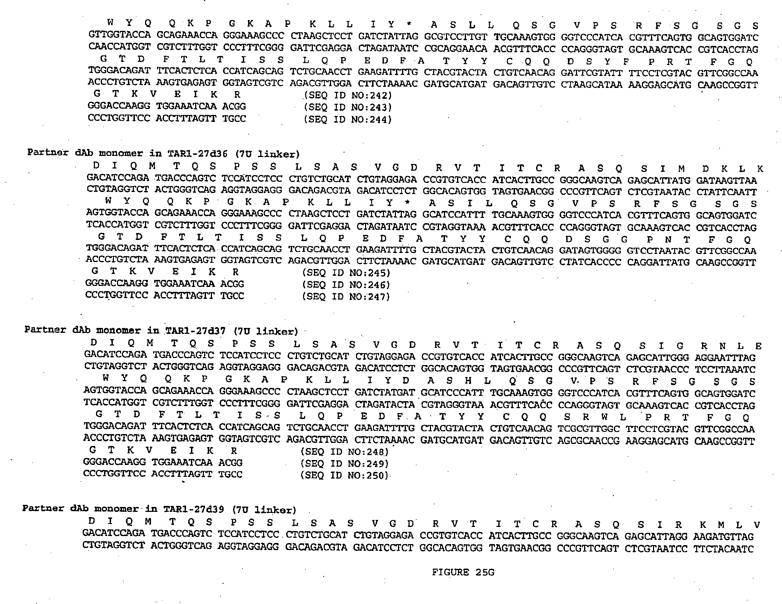

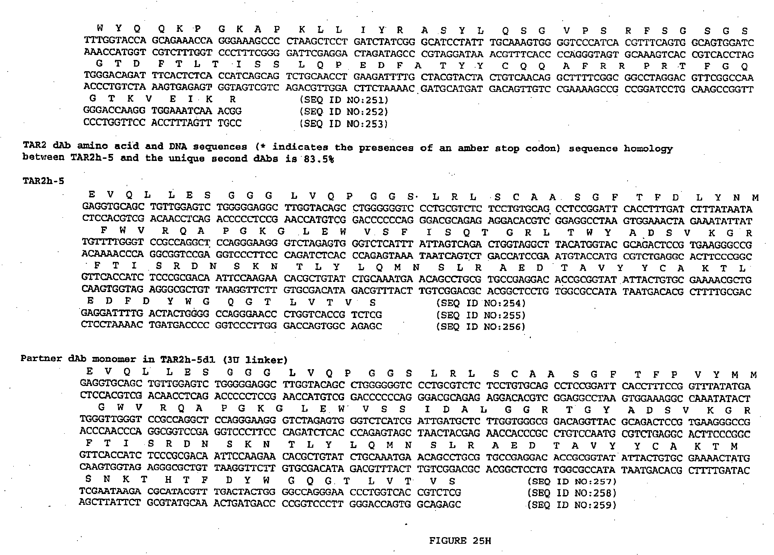

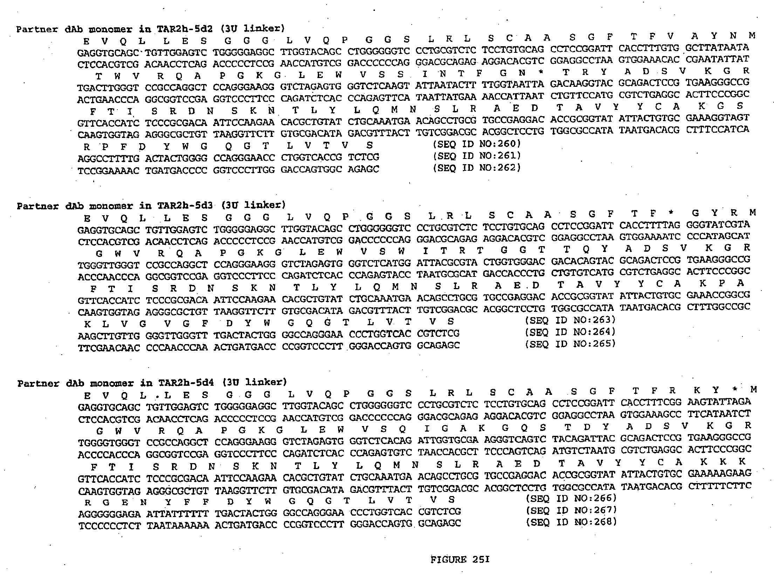

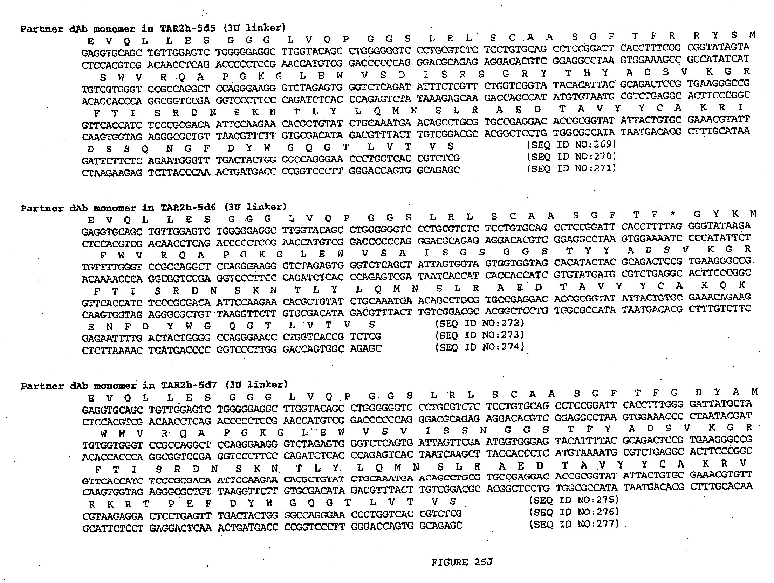

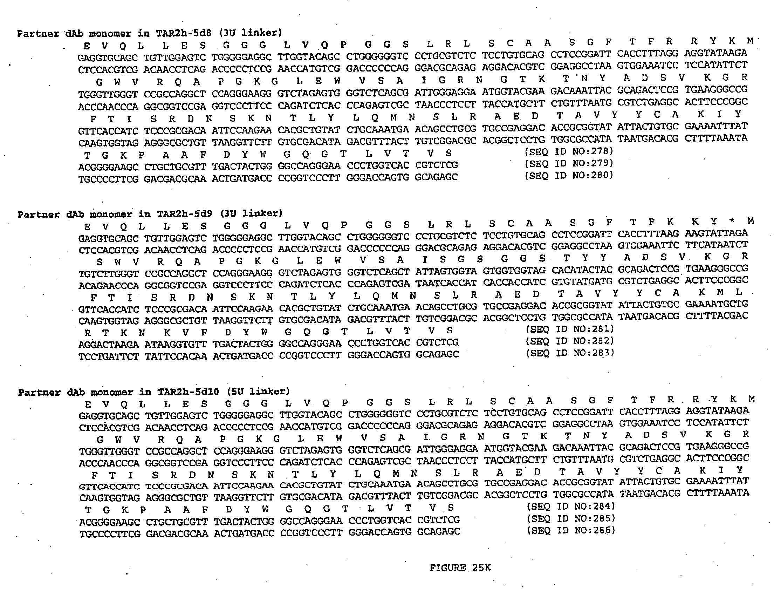

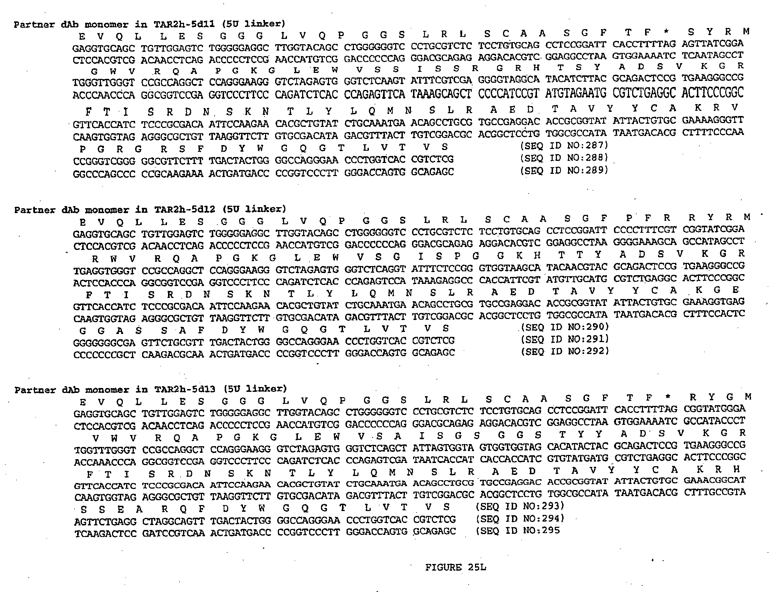

Figure 25A-25L shows the nucleotide sequences encoding several human immunoglobulin variable domains and the amino acid sequences of the encoded human immunoglobulin variable domains (SEQ ID NOS:193-198 and 200-295).

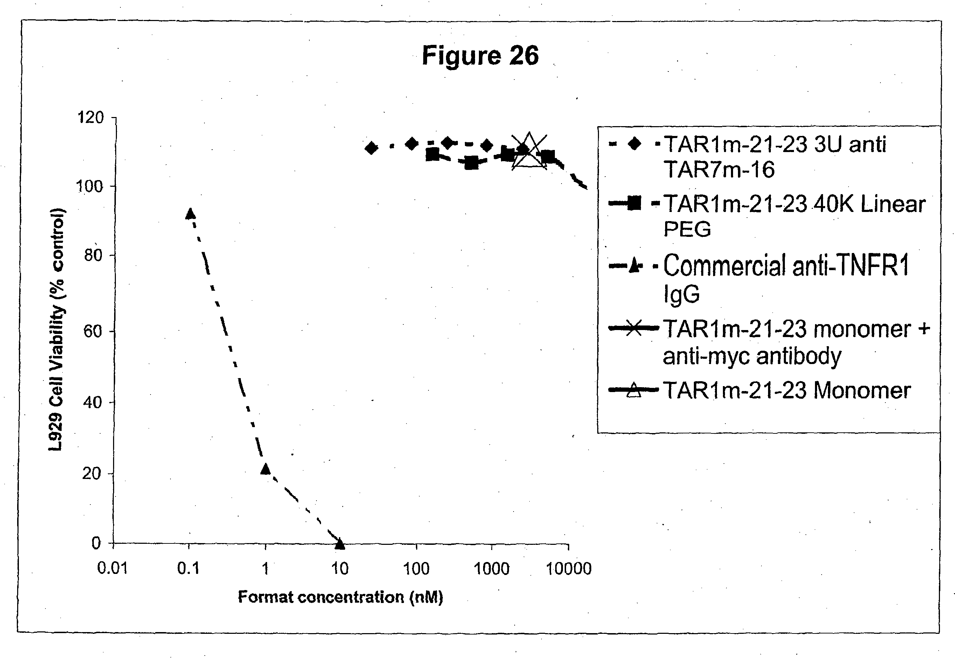

Figure 26 is a graph showing that anti-TNFR1 dAb formats do not substantially agonize TNFR1 in an L929 assay. L929 cells were cultured in media that contained a range of concentrations of anti-TNFR1 dAb monomer (TAR2m-21-23), TAR2m-21-23 monomer cross-linked by a commercially available anti-myc antibody (9E10), dual specific anti-TNFR1 dAb/anti-SA dAb (TAR2m-21-23 3U TAR7m-16), or pegylated anti-TNFR1 dAb monomer (TAR2m-21-23 40K PEG). In the case of TAR2m-21-23 monomer cross-linked by the anti-myc antibody, the dAb and antibody were mixed in a 2:1 ratio and pre-incubated for one hour at room-temperature to simulate the effects of *in vivo* immune cross-linking prior to culture. (The TAR2m-21-23 monomer includes a myc epitope.) TAR2m-21-23 monomer was incubated with the L929 cells at a concentration of 3,000 nM. TAR2m-21-23 monomer and anti-Myc antibody were incubated at a dAb concentration of 3,000 nM. TAR2m-21-23 3U TAR7m-16 was incubated with the cells at 25 nM, 83.3 nM, 250 nM, 833 nM and 2,500 nM concentrations. TAR2m-21-23 40K PEG was incubated with the cells at 158.25 nM, 527.5 nM, 1582.5 nM, 5,275 nM and 15,825 nM concentrations. After incubation overnight, cell viability was assessed. The results revealed that incubation of L929 cells with 10 nM, 1 nM or 0.1 nM of a commercially-available anti-TNFR1 IgG antibody that crosslinks and agonizes TNFR1 (Catalog No. AF-425-PB; R&D Systems, Minneapolis, MN) resulted in a dose-dependent increase in non-viable cells, thereby demonstrating the sensitivity of these cells to agonists of TNFR1. In contrast, incubation with various amounts of anti-TNFR1 formats did not antagonize TNFR1 and did not result in an increase in the number of non-viable cells in the cultures, even when used at more than 1000 times the concentration of the commercially-available anti-TNFR1 IgG antibody.

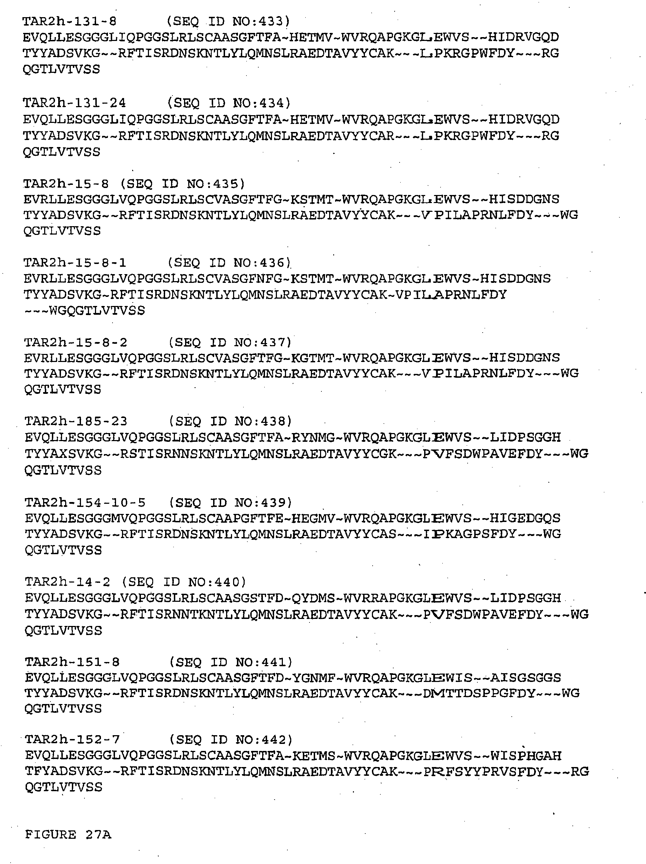

Figure 27A-27I shows the amino acid sequences (SEQ ID NOS:433-517 and 627) of several human immunoglobulin variable domains that have binding specificity for human TNFR11. The presented amino acid sequences are continuous with no gaps; the symbol ~ has been inserted into the sequences to indicate the locations of the complementarity determining regions (CDRs). CDR1 is flanked by ~, CDR2 is flanked by ~~, and CDR3 is flanked by ~~~.

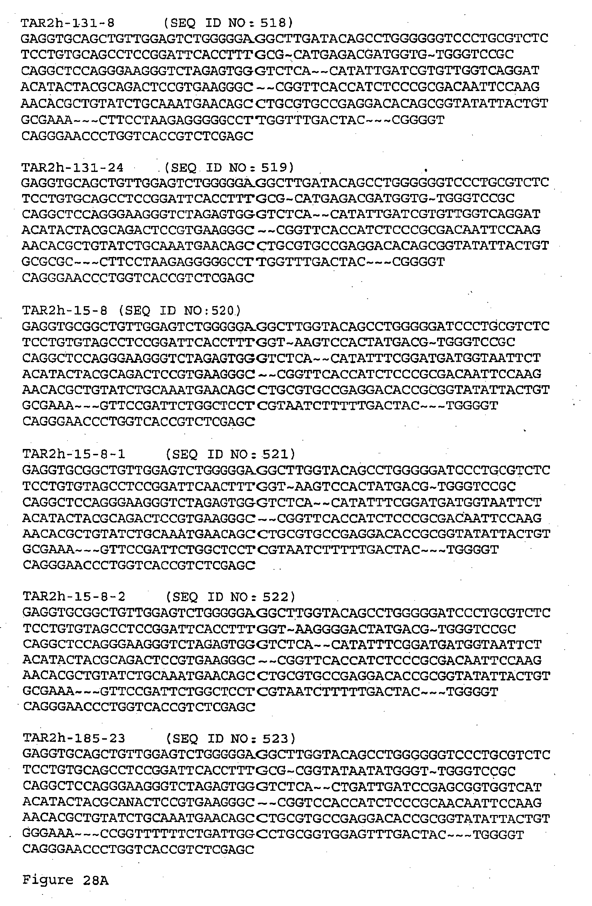

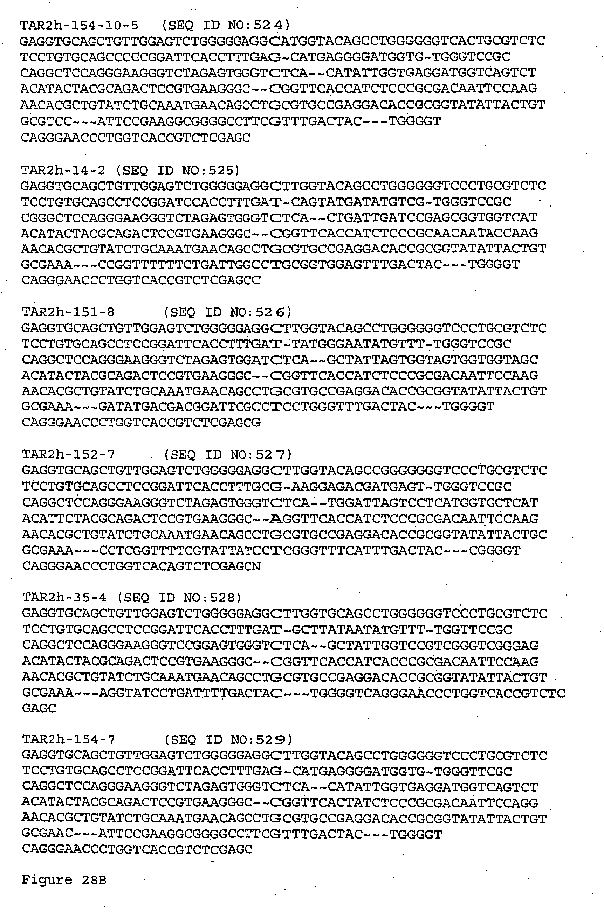

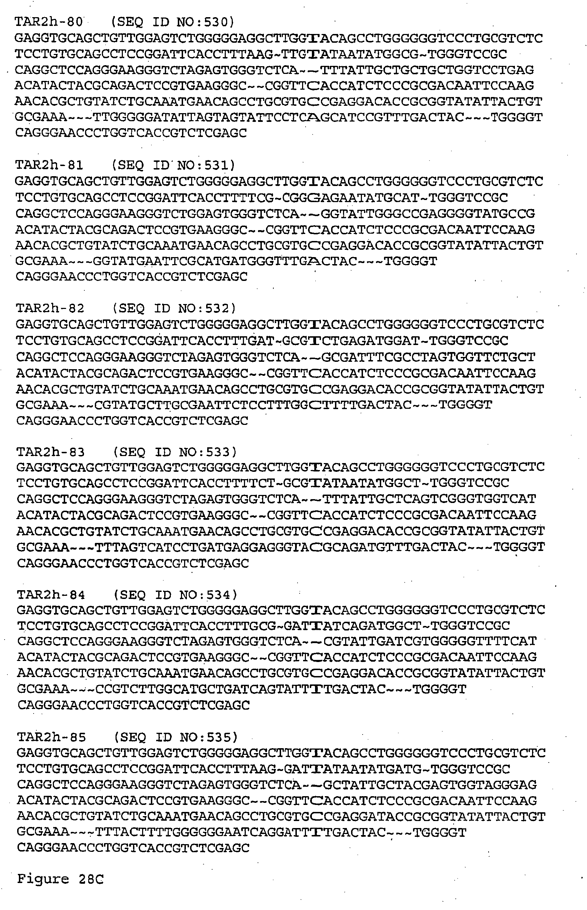

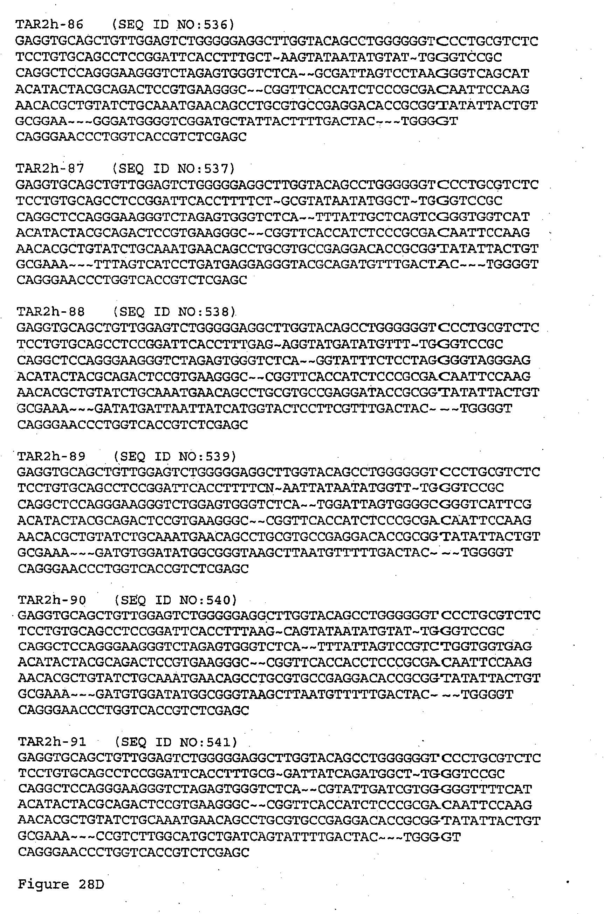

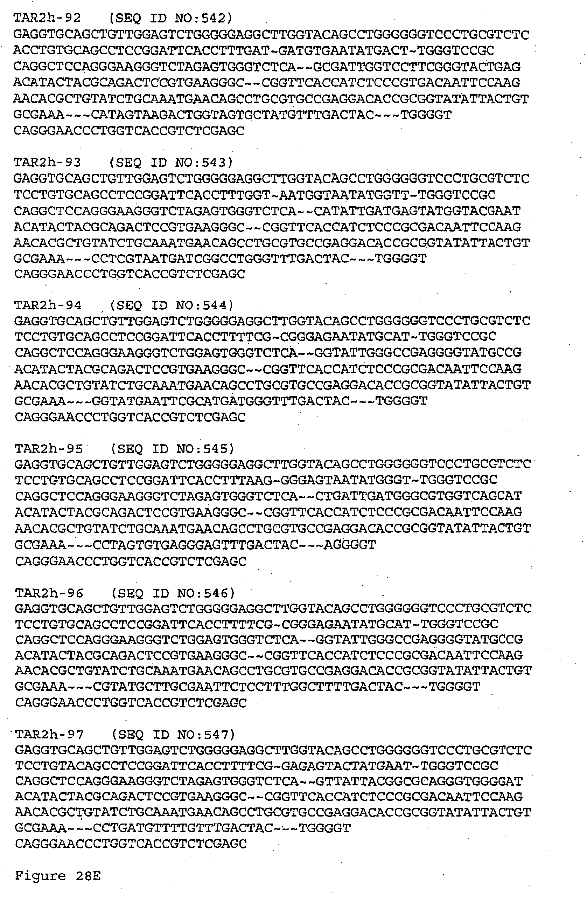

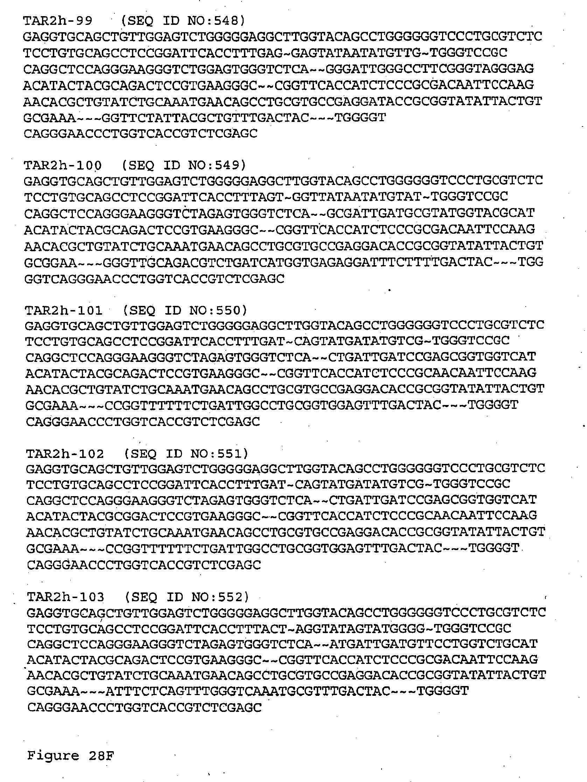

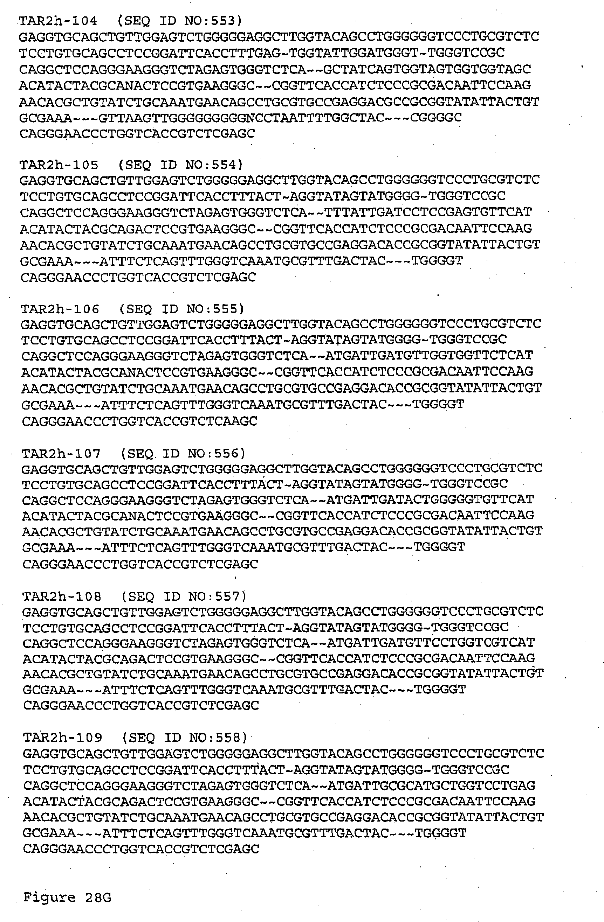

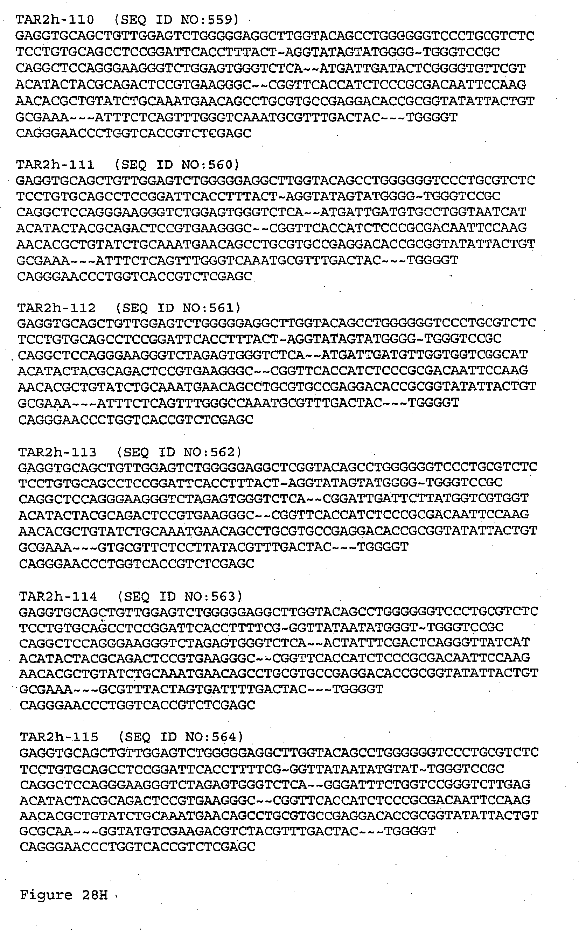

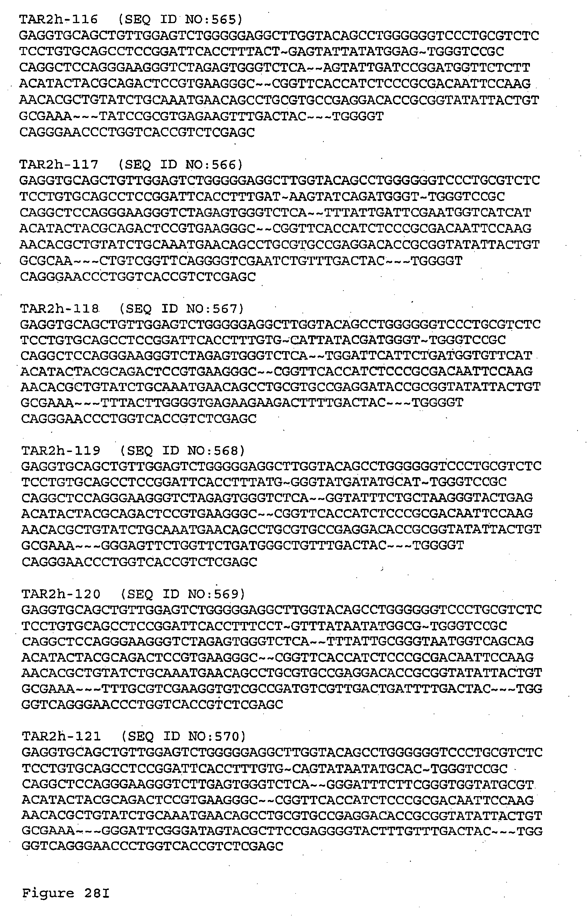

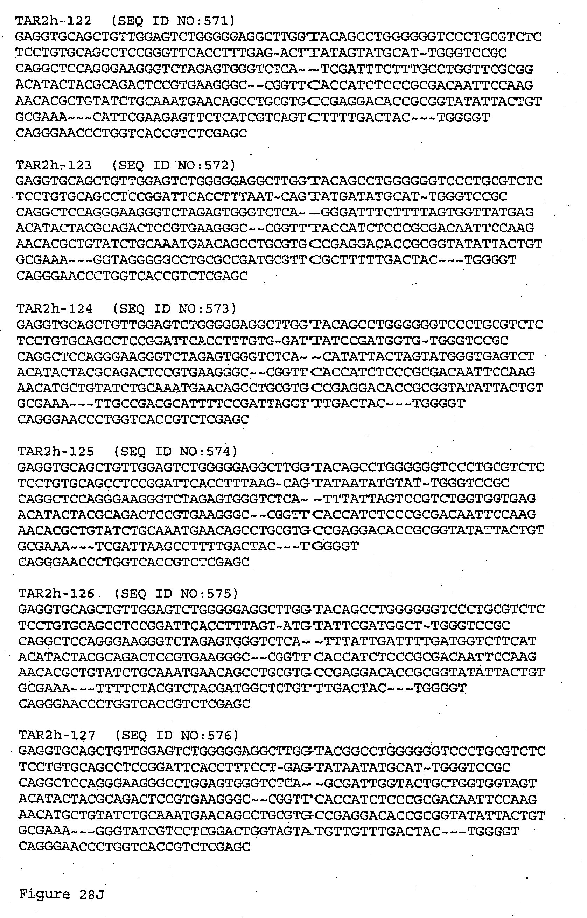

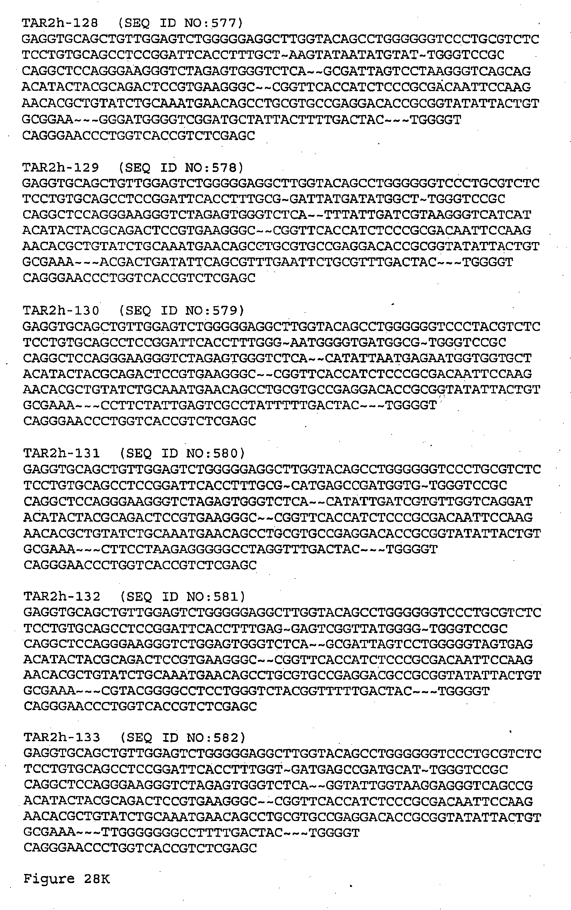

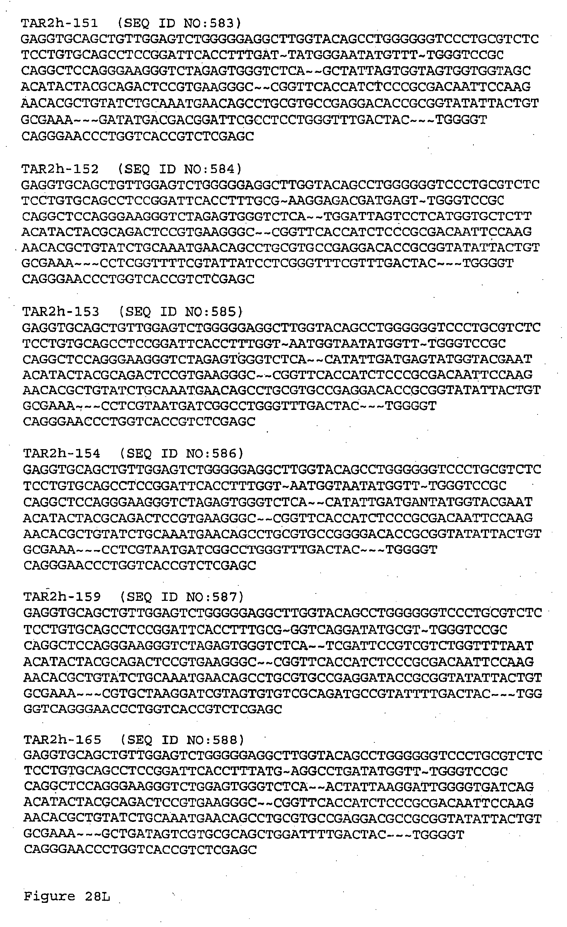

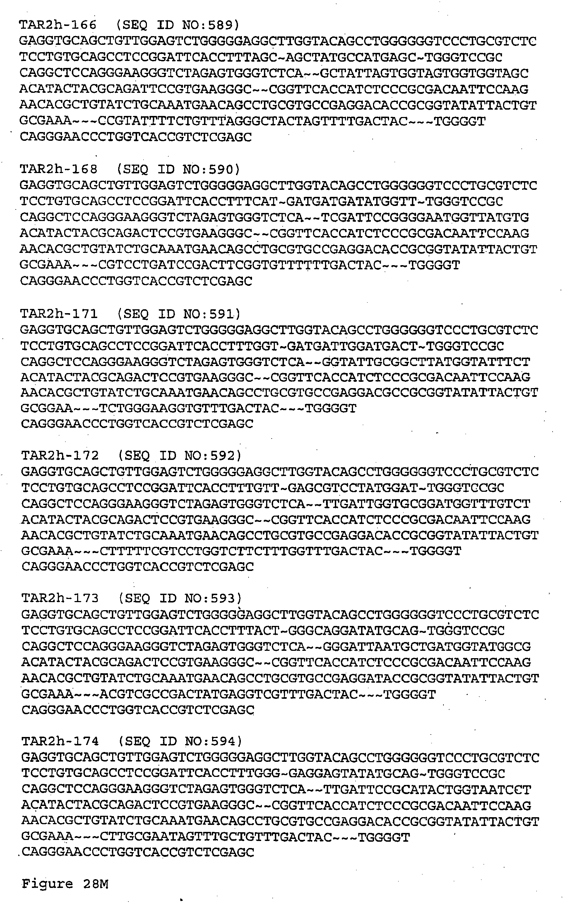

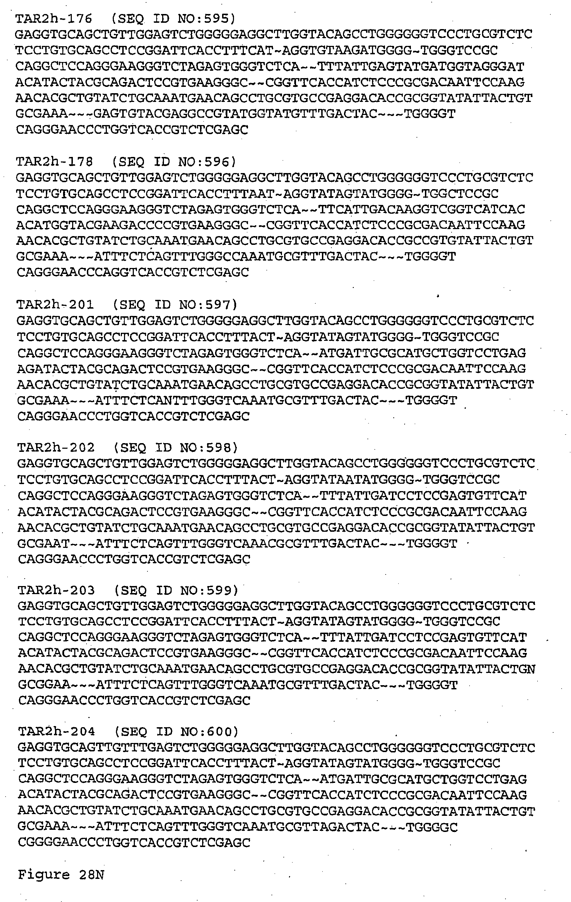

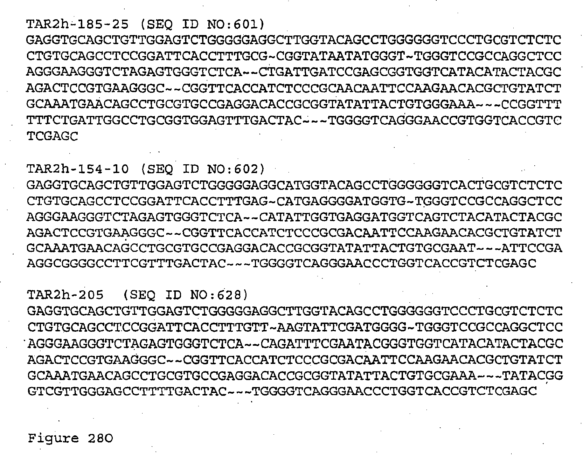

Figure 28A-28O shows the nucleotide sequences (SEQ ID NOS:518-602 and 628) of several nucleic acids that encode the human immunoglobulin variable domains presented in Figure 27A-27H. The presented nucleotide sequences are continuous with no gaps; the symbol ~ has been inserted into the sequences to indicate the location of the sequences encoding the CDRs. The sequences encoding CDR1 are flanked by ~, the sequences encoding CDR2 are flanked by ~~, and the sequences encoding CDR3 are flanked by ~~~.

### DETAILED DESCRIPTION OF THE INVENTION

Within this specification embodiments have been described in a way which enables a clear and concise specification to be written, but it is intended and will be appreciated that embodiments may be variously combined or separated without parting from the invention.

### Definitions

"Complementary" Two immunoglobulin domains are "complementary" where they belong to families of structures which form cognate pairs or groups or are derived from such families and retain this feature. For example, a V_{H} domain and a V_{L} domain of an antibody are complementary; two V_{H} domains are not complementary, and two V_{L} domains are not complementary. Complementary domains may be found in other members of the immunoglobulin superfamily, such as the V_{α} and V_{β} (or γ and δ) domains of the T-cell receptor. In the context of the second configuration of the present invention, non-complementary domains do not bind a target molecule cooperatively, but act independently on different target epitopes which may be on the same or different molecules. Domains which are artificial, such as domains based on protein scaffolds which do not bind epitopes unless engineered to do so, are non-complementary. Likewise, two domains based on (for example) an immunoglobulin domain and a fibronectin domain are not complementary.

"Immunoglobulin" This refers to a family of polypeptides which retain the immunoglobulin fold characteristic of antibody molecules, which contains two β sheets and, usually, a conserved disulphide bond. Members of the immunoglobulin superfamily are involved in many aspects of cellular and non-cellular interactions *in vivo*, including widespread roles in the immune system (for example, antibodies, T-cell receptor molecules and the like), involvement in cell adhesion (for example the ICAM molecules) and intracellular signalling (for example, receptor molecules, such as the PDGF receptor). The present invention is applicable to all immunoglobulin superfamily molecules which possess binding domains. Preferably, the present invention relates to antibodies.

"Combining" Variable domains according to the invention are combined to form a group of domains; for example, complementary domains may be combined, such as V_{L} domains being combined with V_{H} domains. Non-complementary domains may also be combined. Domains may be combined in a number of ways, involving linkage of the domains by covalent or non-covalent means.

"Domain" A domain is a folded protein structure which retains its tertiary structure independently of the rest of the protein. Generally, domains are responsible for discrete functional properties of proteins, and in many cases may be added, removed or transferred to other proteins without loss of function of the remainder of the protein and/or of the domain. By single antibody variable domain is meant a folded polypeptide domain comprising sequences characteristic of antibody variable domains. It therefore includes complete antibody variable domains and modified variable domains, for example in which one or more loops have been replaced by sequences which are not characteristic of antibody variable domains, or antibody variable domains which have been truncated or comprise N- or C-terminal extensions, as well as folded fragments of variable domains which retain at least in part the binding activity and specificity of the full-length domain.

"Repertoire" A collection of diverse variants, for example polypeptide variants which differ in their primary sequence. A library used in the present invention will encompass a repertoire of polypeptides comprising at least 1000 members.

"Library" The term library refers to a mixture of heterogeneous polypeptides or nucleic acids. The library is composed of members, each of which have a single polypeptide or nucleic acid sequence. To this extent, *library* is synonymous with *repertoire.* Sequence differences between library members are responsible for the diversity present in the library. The library may take the form of a simple mixture of polypeptides or nucleic acids, or may be in the form of organisms or cells, for example bacteria, viruses, animal or plant cells and the like, transformed with a library of nucleic acids. Preferably, each individual organism or cell contains only one or a limited number of library members. Advantageously, the nucleic acids are incorporated into expression vectors, in order to allow expression of the polypeptides encoded by the nucleic acids. In a preferred aspect, therefore, a library may take the form of a population of host organisms, each organism containing one or more copies of an expression vector containing a single member of the library in nucleic acid form which can be expressed to produce its corresponding polypeptide member. Thus, the population of host organisms has the potential to encode a large repertoire of genetically diverse polypeptide variants.

A "closed conformation multi-specific ligand" describes a multi-specific ligand as herein defined comprising at least two epitope binding domains as herein defined. The term 'closed conformation' (multi-specific ligand) means that the epitope binding domains of the ligand are arranged such that epitope binding by one epitope binding domain competes with epitope binding by another epitope binding domain. That is, cognate epitopes may be bound by each epitope binding domain individually but not simultaneously. The closed conformation of the ligand can be achieved using methods herein described.

"Antibody" An antibody (for example IgG, IgM, IgA, IgD or IgE) or fragment (such as a Fab , F(ab')₂, Fv, disulphide linked Fv, scFv, closed conformation multispecific antibody, disulphide-linked scFv, diabody) whether derived from any species naturally producing an antibody, or created by recombinant DNA technology; whether isolated from serum, B-cells, hybridomas, transfectomas, yeast or bacteria).

"Dual-specific ligand" A ligand comprising a first immunoglobulin single variable domain and a second immunoglobulin single variable domain as herein defined, wherein the variable regions are capable of binding to two different antigens or two epitopes on the same antigen which are not normally bound by a monospecific immunoglobulin. For example, the two epitopes may be on the same hapten, but are not the same epitope or sufficiently adjacent to be bound by a monospecific ligand. The dual specific ligands according to the invention are composed of variable domains which have different specificities, and do not contain mutually complementary variable domain pairs which have the same specificity.

"Antigen" A molecule that is bound by a ligand according to the present invention. Typically, antigens are bound by antibody ligands and are capable of raising an antibody response *in vivo*. It may be a polypeptide, protein, nucleic acid or other molecule. Generally, the dual specific ligands according to the invention are selected for target specificity against a particular antigen. In the case of conventional antibodies and fragments thereof, the antibody binding site defined by the variable loops (L1, L2, L3 and H1, H2, H3) is capable of binding to the antigen.

"Epitope" A unit of structure conventionally bound by an immunoglobulin V_{H}/V_{L} pair. Epitopes define the minimum binding site for an antibody, and thus represent the target of specificity of an antibody. In the case of a single domain antibody, an epitope represents the unit of structure bound by a variable domain in iso lation.

"Generic ligand" A ligand that binds to all members of a repertoire. Generally, not bound through the antigen binding site as defined above. Non-limiting examples include protein A, protein L and protein G.

"Selecting" Derived by screening, or derived by a Darwinian selection process, in which binding interactions are made between a domain and the antigen or epitope or between an antibody and an antigen or epitope. Thus a first variable domain may be selected for binding to an antigen or epitope in the presence or in the absence of a complementary variable domain.

"Universal framework" A single antibody framework sequence corresponding to the regions of an antibody conserved in sequence as defined by Kabat ("Sequences of Proteins of Immunological Interest", US Department of Health and Human Services) or corresponding to the human germline immunoglobulin repertoire or structure as defined by Chothia and Lesk, (1987) J. Mol. Biol. 196:910-917. The invention provides for the use of a single framework, or a set of such frameworks, which has been found to permit the derivation of virtually any binding specificity though variation in the hypervariable regions alone.

"Half-life" The time taken for the serum concentration of the ligand to reduce by 50%, *in vivo,* for example due to degradation of the ligand and/or clearance or sequestration of the ligand by natural mechanisms. The ligands of the invention are stabilised *in vivo* and their half-life increased by binding to molecules which resist degradation and/or clearance or sequestration. Typically, such molecules are naturally occurring proteins which themselves have a long half-life *in vivo.* The half-life of a ligand is increased if its functional activity persists, *in vivo,* for a longer period than a similar ligand which is not specific for the half-life increasing molecule. Thus, a ligand specific for HSA and a target molecule is compared with the same ligand wherein the specificity for HSA is not present, that it does not bind HSA but binds another molecule. For example, it may bind a second epitope on the target molecule. Typically, the half life is increased by 10%, 20%, 30%, 40%, 50% or more. Increases in the range of 2x, 3x, 4x, 5x, 10x, 20x, 30x, 40x, 50x or more of the half life are possible. Alternatively, or in addition, increases in the range of up to 30x, 40x, 50x, 60x, 70x, 80x, 90x, 100x, 150x of the half life are possible.

"Homogeneous immunoassay" An immunoassay in which analyte is detected without need for a step of separating bound and un-bound reagents.

"Substantially identical (or "substantially homologous")". A first amino acid or nucleotide sequence that contains a sufficient number of identical or equivalent (e.g., with a similar side chain, e.g., conserved amino acid substitutions) amino acid residues or nucleotides to a second amino acid or nucleotide sequence such that the first and second amino acid or nucleotide sequences have similar activities. In the case of antibodies, the second antibody has the same binding specificity and has at least 50% of the affinity of the same.

As used herein, the terms "low stringency," "medium stringency," "high stringency," or "very high stringency conditions" describe conditions for nucleic acid hybridization and washing. Guidance for performing hybridization reactions can be found in Current Protocols in Molecular Biology, John Wiley & Sons, N.Y. (1989), 6.3.1-6.3.6, which is incorporated herein by reference in its entirety. Aqueous and nonaqueous methods are described in that reference and either can be used. Specific hybridization conditions referred to herein are as follows: (1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); (2) medium stringency hybridization conditions in 6X SSC at about 45°C, followed by one or more washes in 0.2X SSC, 0.1% SDS at 60°C; (3) high stringency hybridization conditions in 6X SSC at about 45°C, followed by one or more washes in 0.2X SSC, 0.1% SDS at 65°C; and preferably (4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

As herein defined the term "closed conformation" (multi-specific ligand) means that the epitope binding domains of the ligand are attached to or associated with each other, optionally by means of a protein skeleton, such that epitope binding by one epitope binding domain competes with epitope binding by another epitope binding domain. That is, cognate epitopes may be bound by each epitope binding domain individually but not simultaneously. The closed conformation of the ligand can be achieved using methods herein described.

"Open conformation" means that the epitope binding domains of the ligand are attached to or associated with each other, optionally by means of a protein skeleton, such that epitope binding by one epitope binding domain does not compete with epitope binding by another epitope binding domain.

As referred to herein, the term "competes" means that the binding of a first epitope to its cognate epitope binding domain is inhibited when a second epitope is bound to its cognate epitope binding domain. For example, binding may be inhibited sterically, for example by physical blocking of a binding domain or by alteration of the structure or environment of a binding domain such that its affinity or avidity for an epitope is reduced.

The phrase "immunoglobulin single variable domain" refers to an antibody variable region (V_{H}, V_{HH}, V_{L}) that specifically binds an antigen or epitope independently of other V regions or domains; however, as the term is used herein, an immunoglobulin single variable domain can be present in a format (e.g., homo- or hetero-multimer) with other variable regions or variable domains where the other regions or domains are not required for antigen binding by the single immunoglobulin variable domain (i.e., where the immunoglobulin single variable domain binds antigen independently of the additional variable domains). "Immunoglobulin single variable domain" encompasses not only an isolated antibody single variable domain polypeptide, but also larger polypeptides that comprise one or more monomers of an antibody single variable domain polypeptide sequence. A "domain antibody" or "dAb" is the same as an "immunoglobulin single variable domain" polypeptide as the term is used herein. An immunoglobulin single variable domain polypeptide, as used herein refers to a mammalian immunoglobulin single variable domain polypeptide, preferably human, but also includes rodent (for example, as disclosed in WO 00/29004, the contents of which are incorporated herein by reference in their entirety) or camelid V_{HH} dAbs. Camelid dAbs are immunoglobulin single variable domain polypeptides which are derived from species including camel, llama, alpaca, dromedary, and guanaco, and comprise heavy chain antibodies naturally devoid of light chain: V_{HH}. V_{HH} molecules are about ten times smaller than IgG molecules, and as single polypeptides, they are very stable, resisting extreme pH and temperature conditions.

As used herein, the term "antagonist of Tumor Necrosis Factor Receptor 1 (TNFR1)" refers to an agent (e.g., a molecule, a compound) which binds TNFR1 and can inhibit a (i.e., one or more) function of TNFR1. For example, an antagonist of TNFR1 can inhibit the binding of TNFα to TNFR1 and/or inhibit signal transduction mediated through TNFR1. Accordingly, TNFR1-mediated processes and cellular responses (e.g., TNFα-induced cell death in a standard L929 cytotoxicity assay) can be inhibited with an antagonist of TNFR1. An antagonist of TNFR1 can be, for example, a small organic molecule, natural product, protein, peptide or peptidomimetic. Antagonists of TNFR1 can be identified, for example, by screening libraries or collections of molecules, such as, the Chemical Repository of the National Cancer Institute, as described herein or using other suitable methods. Preferred antagonists of TNFR1 are antibodies, antigen-binding fragments of antibodies, ligands and dAb monomers described herein.

Sequences similar or homologous to the sequences disclosed herein are also part of the invention. In some embodiments, the sequence identity at the amino acid level can be about 90%, 91%, 92%, 93%, 94%, 95%, 96%, 97%, 98%, 99% or higher. Calculations of "homology" or "sequence identity" or "similarity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "homology" is equivalent to amino acid or nucleic acid "identity"). The percent identity between the two sequences is a function of the number of identical positions shared by the sequences, taking into account the number of gaps, and the length of each gap, which need to be introduced for optimal alignment of the two sequences.

Advantageously, the BLAST algorithm (version 2.0) is employed for sequence alignment, with parameters set to default values. The BLAST algorithm is described in detail at the world wide web site ("www") of the National Center for Biotechnology Information (".ncbi") of the National Institutes of Health ("nih") of the U.S. government (".gov"), in the "/Blast/" directory, in the "blast_help.html" file. The search parameters are defined as follows, and are advantageously set to the defined default parameters.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul, 1990, Proc. Natl. Acad. Sci. USA 87(6):2264-8 (see the "blast_help.html" file, as described above) with a few enhancements. The BLAST programs were tailored for sequence similarity searching, for example to identify homologues to a query sequence. The programs are not generally useful for motif-style searching. For a discussion of basic issues in similarity searching of sequence databases, see Altschul et al. (1994).

The five BLAST programs available at the National Center for Biotechnology Information web site perform the following tasks:
"blastp" compares an amino acid query sequence against a protein sequence database;
"blastn" compares a nucleotide query sequence against a nucleotide sequence database;
"blastx" compares the six-frame conceptual translation products of a nucleotide query sequence (both strands) against a protein sequence database;
"tblastn" compares a protein query sequence against a nucleotide sequence database dynamically translated in all six reading frames (both strands).
"tblastx" compares the six-frame translations of a nucleotide query sequence against the six-frame translations of a nucleotide sequence database.

BLAST uses the following search parameters:
HISTOGRAM Display a histogram of scores for each search; default is yes. (See parameter H in the BLAST Manual).

DESCRIPTIONS Restricts the number of short descriptions of matching sequences reported to the number specified; default limit is 100 descriptions. (See parameter V in the manual page). See also EXPECT and CUTOFF.

ALIGNMENTS Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).

EXPECT The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).

CUTOFF Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.

MATRIX Specify an alternate scoring matrix for BLASTP, BLASTX, TBLASTN and TBLASTX. The default matrix is BLOSUM62 (Henikoff & Henikoff, 1992, Proc. Natl. Aacad. Sci. USA 89(22):10915-9). The valid alternative choices include: PAM40, PAM 120, PAM250 and IDENTITY. No alternate scoring matrices are available for BLASTN; specifying the MATRIX directive in BLASTN requests returns an error response.

STRAND Restrict a TBLASTN search to just the top or bottom strand of the database sequences; or restrict a BLASTN, BLASTX or TBLASTX search to just reading frames on the top or bottom strand of the query sequence.

FILTER Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States, 1993, Computers and Chemistry 17:191-201, or, for BLASTN, by the DUST program of Tatusov and Lipman (see the world wide web site of the NCBI). Filtering can eliminate statistically significant but biologically uninteresting reports from the blast output (e.g., hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "N" repeated 13 times) and the letter "X" in protein sequences (e.g., "X" repeated 9 times).

Filtering is only applied to the query sequence (or its translation products), not to database sequences. Default filtering is DUST for BLASTN, SEG for other programs.

It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi Causes NCBI gi identifiers to be shown in the output, in addition to the accession and/or locus name.

Most preferably, sequence comparisons are conducted using the simple BLAST search algorithm provided at the NCBI world wide web site described above, in the "/BLAST" directory.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc. which are incorporated herein by reference) and chemical methods.

TNFR1 is a transmembrane receptor containing an extracellular region that binds ligand and an intracellular domain that lacks intrinsic signal transduction activity but can associate with signal transduction molecules. The complex of TNFR1 with bound TNF contains three TNFR1 chains and three TNF chains. (Banner et al., Cell, 73(3) 431-445 (1993).) The TNF ligand is present as a trimer, which is bound by three TNFR1 chains. (Id.) The three TNFR1 chains are clustered closely together in the receptor-ligand complex, and this clustering is a prerequisite to TNFR1-mediated signal transduction. In fact, multivalent agents that bind TNFR1, such as anti-TNFR1 antibodies, can induce TNFR1 clustering and signal transduction in the absence of TNF and are commonly used as TNFR1 agonists. (See, *e.g*., Belka et al., EMBO, 14(6):1156-1165 (1995); Mandik-Nayak et al., J. Immunol, 167:1920-1928 (2001).) Accordingly, multivalent agents that bind TNFR1, are generally not effective antagonists of TNFR1 even if they block the binding of TNFα, to TNFR1.

The extracellular region of TNFR1 comprises a thirteen amino acid amino-terminal segment (amino acids 1-13 of SEQ ID NO:603 (human); amino acids 1-13 of SEQ ID NO:604 (mouse)), Domain 1 (amino acids 14-53 of SEQ ID NO:603 (human); amino acids 14-53 of SEQ ID NO:604 (mouse)), Domain 2 (amino acids 54-97 of SEQ ID NO: 603 (human); amino acids 54-97 of SEQ ID NO:604 (mouse)), Domain 3 (amino acids 98-138 of SEQ ID NO: 603 (human); amino acid 98-138 of SEQ ID NO:604 (mouse)), and Domain 4 (amino acids 139-167 of SEQ ID NO:603 (human); amino acids 139-167 of SEQ ID NO:604 (mouse)) which is followed by a membrane-proximal region (amino acids 168-182 of SEQ ID NO:603_(human); amino acids 168-183 SEQ ID NO: 604 (mouse)). (See, Banner et al., Cell 73(3) 431-445 (1993) and Loetscher et al., Cell 61(2) 351-359 (1990).) Domains 2 and 3 make contact with bound ligand (TNFβ, TNFα). (Banner et al., Cell, 73(3) 431-445 (1993).) The extracellular region of TNFR1 also contains a region referred to as the pre-ligand binding assembly domain or PLAD domain (amino acids 1-53 of SEQ ID NO:603_(human); amino acids 1-53 of SEQ ID NO:604 (mouse)) (The Government of the USA, WO 01/58953; Deng et al., Nature Medicine, doi: 10.1038/nm1304 (2005)).

TNFR1 is shed from the surface of cells *in vivo* through a process that includes proteolysis of TNFR1 in Domain 4 or in the membrane-proximal region (amino acids 168-182 of SEQ ID NO:603; amino acids 168-183 of SEQ ID NO:604), to produce a soluble form of TNFR1. Soluble TNFR1 retains the capacity to bind TNFα, and thereby functions as an endogenous inhibitor of the activity of TNFα.

The invention relates to a dAb) or ligand that binds TNFR1 but does not compete with TNF for binding to TNFR1. Such antibody or antigen-binding fragment thereof (*e.g*., dAb) or ligand provide advantages as diagnostic agents, and can be used to bind and detect, quantify or measure TNFR1 in a sample but will not compete with TNF in the sample for binding to TNFR1. Accordingly, an accurate determination of whether TNFR1 is present in the sample or how much TNFR1 is in the sample can be made.

The domain antibody of the invention could also be used in a diagnostic kit for determine whether TNFR1 is present in a sample or how much TNFR1 is present in a sample, comprising a dAb or ligand that binds TNFR1 but does not compete with TNF for binding to TNFR1 and instructions for use (e.g., to determine the presence and/or quantity of TNFR1 in the sample). The kit may further comprise one or more ancillary reagents, such as a suitable buffer or suitable detecting reagent (e.g., a detectably labeled antibody or antigen-binding fragment thereof that binds the dAb or ligand that binds TNFR1 but does not compete with TNF for binding to TNFR1).

The invention relates to antagonists of the invention which are antagonists of TNFR1 (e.g., ligands described herein) that have binding specificity for Tumor Necrosis Factor Receptor 1 (TNFR1; p55; CD120a). Preferably the antagonists of the inventions do not have binding specificity for Tumor Necrosis Factor 2 (TNFR2), or do not substantially antagonize TNFR2. An antagonist of TNFR1 does not substantially antagonize TNFR2 when the antagonist (1 nM, 10 nM, 100 nM, 1 µM, 10 µM or 100 µM) results in no more than about 5% inhibition of TNFR2-mediated activity induced by TNFα (100 pg/ml) in a standard cell assay. Particularly preferred antagonists of TNFR1 are effective therapeutics for treating chronic inflammatory disease (are efficacious, have therapeutic efficacy). For example, in some embodiments, the antagonist of TNFR1 is efficacious in a model of chronic inflammatory disease, such as the mouse collagen-induced arthritis model, mouse ΔARE model of arthritis, mouse dextran sulfate sodium-induced model of inflammatory bowel disease, mouse ΔARE model of inflammatory bowel disease, mouse tobacco smoke model of chronic obstructive pulmonary disease or a suitable primate model (e.g., primate collagen-induced arthritis).

Antagonists of TNFR1 can be monovalent or multivalent. In some embodiments, the antagonist is monovalent and contains one binding site that interacts with TNFR1. Monovalent antagonists bind one TNFR1 and do not induce cross-linking or clustering of TNFR1 on the surface of cells which can lead to activation of the receptor and signal transduction.

In other embodiments, the antagonist of TNFR1 is multivalent. Multivalent antagonists of TNFR1 can contain two or more copies of a particular binding site for TNFR1 or contain two or more different binding sites that bind TNFR1. For example, as described herein the antagonist of TNFR1 can be a dimer, trimer or multimer comprising two or more copies of a particular dAb that binds TNFR1, or two or more different dAbs that bind TNFR1. Preferably, a multivalent antagonist of TNFR1 does not substantially agonize TNFR1 (act as an agonist of TNFR1) in a standard cell assay (*i.e*., when present at a concentration of 1 nM, 10 nM, 100 nM, 1 µM, 10 µM, 100 µM, 1000 µM or 5,000 µM, results in no more than about 5% of the TNFR1-mediated activity induced by TNFα (100 pg/ml) in the assay).

In certain embodiments, the multivalent antagonist of TNFR1 contains two or more binding sites for a desired epitope or domain of TNFR1.

In other embodiments, the multivalent antagonist of TNFR1 contains two or more binding sites that bind to different epitopes or domains of TNFR1. In one example, the multivalent antagonist of TNFR1 can comprise binding sites that bind two or more desired epitopes or domains of TNFR1. For example, the multivalent antagonists of TNFR1 can comprise binding sites for Domains 1 and 4 of TNFR1. Preferably, such multivalent antagonists do not agonize TNFR1 when present at a concentration of about 1 nM, or about 10 nM, or about 100 nM, or about 1 µM, or about 10 µM, in a standard L929 cytotoxicity assay or a standard HeLa IL-8 assay as described herein.

The ligands of the invention do not inhibit binding of TNFα to TNFR1. Such ligands (and antagonists) provide advantages as diagnostic agents, because they can be used to bind and detect, quantify or measure TNFR1 in a sample and will not compete with TNF in the sample for binding to TNFR1. Accordingly, an accurate determination of whether or how much TNFR1 is in the sample can be made.

The ligands of the invention do not inhibit binding of TNFα to TNFR1, but do inhibit signal transduction mediated through TNFR1. For example, an antagonist of TNFR1 can inhibit TNFα-induced clustering of TNFR1, which precedes signal transduction through TNFR1. Such antagonists provide several advantages. For example, in the presence of such an antagonist, TNFα can bind TNFR1 expressed on the surface of cells and be removed from the cellular environment, but TNFR1 mediated signal transduction will not be activated. Thus, TNFR1 signal-induced production of additional TNFα and other mediators of inflammation will be inhibited. Similarly, antagonists of TNFR1 that bind TNFR1 and inhibit signal transduction mediated through TNFR1, but do no inhibit binding of TNFα to TNFR1, will not inhibit the TNFα-binding and inhibiting activity of endogenously produced soluble TNFR1. Accordingly, administering such an antagonist to a mammal in need thereof can complement the endogenous regulatory pathways that inhibit the activity TNFα and the activity of TNFR1 *in vivo.* The ligands of the invention (i) bind TNFR1, (ii) do not antagonize the activation of TNFR1 mediated signal transduction, and (iii) do not inhibit the binding of TNFα to TNFR1. Such a ligand binds soluble TNFR1 and do not prevent the soluble receptor from binding TNFα, and thus administering such an antagonist to a mammal in need thereof can complement the endogenous regulatory pathways that inhibit the activity TNFα *in vivo* by increasing the half-life of the soluble receptor in the serum. These advantages are particularly relevant to ligands that have been formatted to have a larger hydrodynamic size, for example, by attachment of a PEG group, serum albumin, transferrin, transferrin receptor or at least the transferrin-binding portion thereof, an antibody Fc region, or by conjugation to an antibody domain. For example, an agent (e.g., polypeptide) that i) bind TNFR1, (ii) does not antagonize the activation of TNFR1 mediated signal transduction, and (iii) does not inhibit the binding of TNFα to TNFR1, such as a dAb monomer, can be formatted as a larger antigen-binding fragment of an antibody or as and antibody (e.g., formatted as a Fab, Fab', F(ab)₂, F(ab')₂, IgG, scFv). The hydrodynaminc size of a ligand and its serum half-life can also be increased by conjugating or linking a TNFR1 binding agent to a binding domain (e.g., antibody or antibody fragment) that binds an antigen or epitope that increases half-live *in vivo,* as described herein (see, Annex 1). For example, the TNFR1 binding agent (e.g., polypeptide) can be conjugated or linked to an anti-serum albumin or anti-neonatal Fc receptor antibody or antibody fragment, eg an anti-SA or anti-neonatal Fc receptor dAb, Fab, Fab' or scFv, or to an anti-SA affibody or anti-neonatal Fc receptor affibody.

Examples of suitable albumin, albumin fragments or albumin variants for use in a TNFR1-binding ligand according to the invention are described in WO 2005/077042A2. In particular, the following albumin, albumin fragments or albumin variants can be used in the present invention:
- SEQ ID NO:1 as disclosed in WO 2005/077042A2.
- Albumin fragment or variant comprising or consisting of amino acids 1-387 of SEQ ID NO:1 in WO 2005/077042A2;
- Albumin, or fragment or variant thereof, comprising an amino acid sequence selected from the group consisting of: (a) amino acids 54 to 61 of SEQ ID NO: 1 in WO 2005/077042A2; (b) amino acids 76 to 89 of SEQ ID NO: 1 in WO 2005/077042A2; (c) amino acids 92 to 100 of SEQ ID NO:1 in WO 2005/077042A2; (d) amino acids 170 to 176 of SEQ ID NO:1 in WO 2005/077042A2; (e) amino acids 247 to 252 of SEQ ID NO:1 in WO 2005/077042A2; (f) amino acids 266 to 277 of SEQ ID NO:1 in WO 2005/077042A2; (g) amino acids 280 to 288 of SEQ ID NO:1 in WO 2005/077042A2; (h) amino acids 362 to 368 of SEQ ID NO:1 in WO 2005/077042A2; (i) amino acids 439 to 447 of SEQ ID NO:1 in WO 2005/077042A2 (j) amino acids 462 to 475 of SEQ ID NO: in WO 2005/077042A2; (k) amino acids 478 to 486 of SEQ ID NO:1 in WO 2005/077042A2; and (1) amino acids 560 to 566 of SEQ ID NO:1 in WO 2005/077042A2.

Further examples of suitable albumin, fragments and analogs for use in a TNFR1-binding ligand according to the invention are described in WO 03/076567A2, which is incorporated herein by reference in its entirety. In particular, the following albumin, fragments or variants can be used in the present invention:
- Human serum albumin as described in WO 03/076567A2, eg, in figure 3
- Human serum albumin (HA) consisting of a single non-glycosylated polypeptide chain of 585 amino acids with a formula molecular weight of 66,500 (See, Meloun, et al., FEBSLetters 58:136 (1975); Behrens, et al., Fed. Proc. 34:591 (1975); Lawn, et al., Nucleic Acids Research 9:6102-6114 (1981); Minghetti, et al., J. Biol. Chem. 261:6747 (1986));
- A polymorphic variant or analog or fragment of albumin as described in Weitkamp, et al., Ann. Hum. Genet. 37:219 (1973);
- An albumin fragment or variant as described in EP 322094, eg, HA(1-373., HA(1-388), HA(1-389), HA(1-369), and HA(1-419) and fragments between 1-369 and 1-419;
- An albumin fragment or variant as described in EP 399666, eg, HA(1-177) and HA(1-200) and fragments between HA(1-X), where X is any number from 178 to 199.

Where a (one or more) half-life extending moeity (eg, albumin, transferrin and fragments and analogues thereof) is used in the TNFR1-binding ligands of the invention, it can be conjugated using any suitable method, such as, by direct fusion to the TNFR1-binding moeity (eg, anti-TNFR1 dAb or antibody fragment), for example by using a single nucleotide construct that encodes a fusion protein, wherein the fusion protein is encoded as a single polypeptide chain with the half-life extending moeity located N- or C-terminally to the TNFR1 binding moeity. Alternatively, conjugation can be achieved by using a peptide linker between moeities, eg, a peptide linker as described in WO 03/076567A2 or WO 2004/003019

Some ligands of the invention bind human TNFR1 and mouse TNFR1. Such ligands (e.g., antagonists, dAb monomers) provide the advantage of allowing preclinical and clinical studies using the same ligand and obviate the need to conduct preclinical studies with a suitable surrogate ligand.

Preferably, the antagonist of TNFR1 is a ligand as described herein. The ligands comprise a domain antibody (dAb) that has binding specificity for TNFR1. The ligand can be a polypeptide that consists of such a dAb, or consists essentially of such a dAb. The ligand can also be a polypeptide that comprises a dAb in a suitable format, such as an antibody format (e.g., IgG-like format, scFv, Fab, Fab', F(ab')₂), a dual specific ligand that comprises a dAb that binds TNFR1 and a second dAb that binds another target protein, antigen or epitope (e.g., serum albumin), or a multispecific ligand as described herein.

Antagonists of TNFR1 of the disclosure, including ligands according to any aspect of the present invention, as well as dAb monomers useful in constructing such ligands, may advantageously dissociate from their cognate target(s) with a K_{d} of 300 nM to 5 pM (ie, 3 x 10⁻⁷ to 5 x 10⁻¹²M), preferably 50 nM to 20 pM, or 5 nM to 200 pM or 1 nM to 100 pM, 1 x 10⁻⁷ M or less, 1 x 10⁻⁸ M or less, 1 x 10⁻⁹ M or less, 1 x 10⁻¹⁰ M or less, 1 x 10⁻¹¹ M or less; and/or a K_{off} rate constant of 5 x 10⁻¹ s⁻¹ to 1 x 10⁻⁷ s⁻¹, preferably 1 x 10⁻² s⁻¹ to 1 x 10⁻⁶ s⁻¹, or 5 x 10⁻³ s⁻¹ to 1 x 10⁻⁵ s⁻¹, or 5 x 10⁻¹ s⁻¹ or less, or 1 x 10⁻² s⁻¹ or less, or 1 x 10⁻³ s⁻¹ or less, or 1 x 10⁻⁴ s⁻¹ or less, or 1 x 10⁻⁵ s⁻¹ or less, or 1 x 10⁻⁶ s⁻¹ or less as determined by surface plasmon resonance. The K_{d} rate constant is defined as K_{off}/Kₒₙ.

In other embodiments, the antagonist of the disclosure binds TNFR1 and inhibits a (i.e., one or more) function of TNFR1 (e.g., receptor clustering, receptor signaling or binding of TNFα to TNFR1), and also binds to another member of the TNF receptor superfamily. Preferably, this type of antagonist also inhibits a function (e.g., member clustering, signaling or binding of the member to its cognate ligand) of the other member of the TNF receptor superfamily. The TNF receptor superfamily is an art recognized group of proteins that includes TNFR1 (p55, CD120a, p60, TNF receptor superfamily member 1A, TNFRSF1A), TNFR2 (p75, p80, CD120b, TNF receptor superfamily member 1B, TNFRSF1B), CD 18 (TNFRSF3, LTBR, TNFR2-RP, TNFR-RP, TNFCR, TNF-R-III), OX40 (TNFRSF4, ACT35, TXGP1L), CD40 (TNFRSF5, p50, Bp50), Fas (CD95, TNFRSF6, APO-1, APTI), DcR3 (TNFRSF6B), CD27 (TNFRSF7, Tp55, S152), CD30 (TNFRSF8, Ki-1, D1S166E), CD137 (TNFRSF9,4-1BB, ILA), TRAILR-1 (TNFRSF10A, DR4, Apo2), TRAILR2 (TNFRSF10B, DR5, KILLER, TRICK2A, TRICKB), TRAILR3 (TNFRSF10C, DcR1, LIT, TRID), TRAILR4 (TNFRSF10D, DcR2, TRUNDD), RANK (TNFRSF11A), OPG (TNFRSF11B, OCIF, TR1), DR3 (TNFRSF12, TRAMP, WSL-1, LARD, WSL-LR, DDR3, TR3, APO-3), DR3L (TNFRSF12L), TAC1 (TNFRSF13B), BAFFR (TNFRSF13C), HVEM (TNFRSF14, ATAR, TR2, LIGHTR, HVEA), NGFR (TNFRSF16), BCMA (TNFRSF 17, BCM), AITR (TNFRSF18, GITR), TNFRSF19, FLJ14993 (TNFRSF19L, RELT), DR6 (TNFRSF21), SOBa (TNFRSF22, Tnfrh2, 2810028K06Rik), mSOB (THFRSF23, Tnfrh1). In some embodiments, the antagonist comprises a first dAb that binds TNFR1 and inhibits a function of TNFR1 and a second dAb that binds another member of the TNF receptor superfamily, such as TNFR2 (CD120b), OX40, CD40, Fas (CD95), TRAILR-1, TRAILR-2, TAC1, BCMA and the like as listed above. In another embodiment, the antagonist comprises a dAb monomer that binds TNFR1 and inhibits a function (eg, receptor clustering, receptor signaling or binding of TNFα to TNFR1) of TNFR1 and also binds to another member of the TNF receptor superfamily, such as TNFR2 (CD120b), OX40, CD40, Fas (CD95), TRAILR-1, TRAILR-2, TAC1, BCMA and the like as listed above.

### Ligands and dAb Monomers that Bind TNFR1

The disclosure provides ligands that comprise an anti-TNFRI dAb monomer (e.g., dual specific ligand comprising such a dAb) that binds to TNF Receptor I with a K_{d} of 300 nM to 5 pM (ie, 3 x 10⁻⁷ to 5 x 10⁻¹²M), preferably 50 nM to 20 pM, more preferably 5 nM to 200 pM and most preferably 1 nM to 100 pM, for example 1 x 10⁻⁷ M or less, preferably 1 x 10⁻⁸ M or less, more preferably 1 x 10⁻⁹ M or less, advantageously 1 x 10⁻¹⁰ M or less and most preferably 1 x 10⁻¹¹ M or less; and/or a K_{off} rate constant of 5 x 10⁻¹ s⁻¹ to 1 x 10⁻⁷ s⁻¹, preferably 1 x 10⁻² s⁻¹ to 1 x 10⁻⁶ s⁻¹, more preferably 5 x 10⁻³ s⁻¹ to 1 x 10⁻⁵ s⁻¹, for example 5 x 10⁻¹ s⁻¹ or less, preferably 1 x 10⁻² s⁻¹ or less, advantageously 1 x 10⁻³ s⁻¹ or less, more preferably 1 x 10⁻⁴ s or less, still more preferably 1 x 10⁻⁵ s⁻¹ or less, and most preferably 1 x 10⁻⁶ s⁻¹ or less as determined by surface plasmon resonance.

Preferably, the ligand or dAb monomer inhibits binding of TNF alpha to TNF alpha Receptor I (p55 receptor) with an inhibitory concentration 50 (IC50) of 500 nM to 50 pM, preferably 100 nM to 50 pM, more preferably 10 nM to 100 pM, advantageously 1 nM to 100 pM; for example 50 nM or less, preferably 5 nM or less, more preferably 500 pM or less, advantageously 200 pM or less, and most preferably 100 pM or less. Preferably, the TNF Receptor I target is Human TNFα.

Preferably, the ligand or dAb binds human TNFR1 and inhibits binding of human TNF alpha to human TNFR1, or inhibits signaling through TNFR1 in response to TNF alpha binding. For example, in certain embodiments, a ligand or dAb monomer can bind TNFR1 and inhibit TNFR1-mediated signaling, but does not substantially inhibit binding of TNFα to TNFR1. In some embodiments, the ligand or dAb monomer inhibits TNFα-induced crosslinking or clustering of TNFR1 on the surface of a cell. Such ligands or dAbs (e.g., TAR2m-21-23 described herein) are advantageous because they can antagonize cell surface TNFR1 but do not substantially reduce the inhibitory activity of endogenous soluble TNFR1. For example, the ligand or dAb can bind TNFR1, but inhibit binding of TNFα to TNFR1 in a receptor binding assay by no more that about 10%, no more that about 5%, no more than about 4%, no more than about 3%, no more than about 2%, or no more than about 1%. Also, in these embodiments, the ligand or dAb inhibits TNFα-induced crosslinking of TNFR1 and/or TNFR1-mediated signaling in a standard cell assay by at least about 10%, at least about 20%, at least about 30%, at least about 40%, at least about 50%, at least about 60%, at least about 70%, at least about 80%, at least about 90%, at least about 95%, or at least about 99%.

The ligand can be monovalent (e.g., a dAb monomer) or multivalent (e.g., dual specific, multi-specific) as described herein.

In other embodiments, the ligand of the invention is multivalent and comprises two or more dAb monomers that bind TNFR1. Multivalent ligands can contain two or more copies of a particular dAb that binds TNFR1 or contain two or more dAbs that bind TNFR1. For example, as described herein, the ligand can be a dimer, trimer or multimer comprising two or more copies of a particular dAb that binds TNFR1, or two or more different dAbs that bind TNFR1. In some examples, the ligand is a homo dimer or homo trimer that comprises two or three copies of a particular dAb that binds TNFR1, respectively. Preferably, a multivalent ligand does not substantially agonize TNFR1 (act as an agonist of TNFR1) in a standard cell assay (*i.e.,* when present at a concentration of 1 nM, 10 nM, 100 nM, 1 µM, 10 µM, 100 µM, 1000 µM or 5,000 µM, results in no more than about 5% of the TNFR1-mediated activity induced by TNFα (100 pg/ml) in the assay).

In certain embodiments, the multivalent ligand contains two or more dAbs that bind desired epitope or domain of TNFR1.

In other embodiments, the multivalent ligand contains two or more dAbs that bind to different epitopes or domains of TNFR1.

Other ligands or dAb monomers do not inhibit binding of TNFα to TNFR1. Such antagonists provide advantages as diagnostic agents, because they can be used to bind and detect, quantify or measure TNFR1 in a sample and will not compete with TNF in the sample for binding to TNFR1. Accordingly, an accurate determination of whether or how much TNFR1 is in the sample can be made.

Some ligands and dAb monomers do not inhibit binding of TNFα to TNFR1, but do inhibit signal transduction mediated through TNFR1. For example, a ligand or dAb monomer can inhibit TNFα-induced clustering of TNFR1, which precedes signal transduction through TNFR1. Such ligands or dAb monomers provide several advantages, as discussed herein with respect to antagonists that have these properties. In particular embodiments, the ligand or dAb monomer of this type binds Domain 1 of TNFR1 or Domain 4 of TNFR1. In certain embodiments, the ligand is a dAb monomer that binds Domain 4 of TNFR1.

Preferably, the ligand or dAb monomer neutralizes (inhibits the activity of) TNFα or TNFR1 in a standard assay (*e.g.*, the standard L929 or standard HeLa IL-8 assays described herein) with a neutralizing dose 50 (ND50) of 500 nM to 50 pM, preferably 100 nM to 50 pM, more preferably 10 nM to 100 pM, advantageously 1 nM to 100 pM; for example 50 nM or less, preferably 5 nM or less, more preferably 500 pM or less, advantageously 200 pM or less, and most preferably 100 pM or less.

In certain embodiments, the ligand or dAb monomer specifically binds human Tumor Necrosis Factor Receptor 1 (TNFR1; p55), and dissociates from human TNFR1 with a dissociation constant (K_{d}) of 50 nM to 20 pM, and a K_{off} rate constant of 5x10⁻¹ s⁻¹ to 1x10⁻⁷ s⁻¹, as determined by surface plasmon resonance.

In other embodiments, the ligand or dAb monomer specifically binds TNFR1 with a K_{d} described herein and inhibits lethality in a standard mouse LPS/D-galactosamine-induced septic shock model (i.e., prevents lethality or reduces lethality by at least about 10%, as compared with a suitable control). Preferably, the dAb monomer inhibits lethality by at least about 25%, or by at least about 50%, as compared to a suitable control in a standard mouse LPS/D-galactosamine-induced septic shock model when administered at about 5 mg/kg or more preferably about 1 mg/kg.

In other embodiments, the ligand or dAb monomer binds TNFRI and antagonizes the activity of the TNFR1 in a standard cell assay with an ND₅₀ of ≤ 100 nM, and at a concentration of ≤ 10µM the dAb agonizes the activity of the TNFR1 by ≤ 5% in the assay.

In particular embodiments, ligand or dAb monomer does not substantially agonize TNFR1 (act as an agonist of TNFR1) in a standard cell assay (*i.e.*, when present at a concentration of 1 nM, 10 nM, 100 nM, 1 µM, 10 µM, 100 µM, 1000 µM or 5,000 µM, results in no more than about 5% of the TNFR1-mediated activity induced by TNFα (100 pg/ml) in the assay).

In certain embodiments, the ligand or dAb monomer is substantially resistant to aggregation. For example, in some embodiments, less than about 10%, less than about 9%, less than about 8%, less than about 7%, less than about 6%, less than about 5%, less than about 4%, less than about 3%, less than about 2% or less than about 1% of the ligand or dAb monomer aggregates when a 1-5 mg/ml, 5-10 mg/ml, 10-20 mg/ml, 20-50 mg/ml, 50-100 mg/ml, 100-200 mg/ml or 200 -500 mg/ml solution of ligand or dAb in a solvent that is routinely used for drug formulation such as saline, buffered saline, citrate buffer saline, water, an emulsion, and, any of these solvents with an acceptable excipient such as those approved by the FDA, is maintained at about 22°C, 22-25°C, 25-30°C, 30-37°C, 37-40°C, 40-50°C, 50-60°C, 60-70°C, 70-80°C, 15-20°C, 10-15°C, 5-10°C, 2-5°C, 0-2°C, -10°C to 0°C, -20°C to -10°C, -40°C to -20°C, -60°C to -40°C, or -80°C to -60°C, for a period of about time, for example, 10 minutes, 1 hour , 8 hours, 24 hours, 2 days, 3 days, 4 days, 1 week, 2 weeks, 3 weeks, 1 month, 2 months, 3 months, 4 months, 6 months, 1 year, or 2 years.

Aggregation can be assessed using any suitable method, such as, by microscopy, assessing turbidity of a solution by visual inspection or spectroscopy or any other suitable method. Preferably, aggregation is assessed by dynamic light scattering. Ligands or dAb monomers that are resistant to aggregation provide several advantages. For example, such ligands or dAb monomers can readily be produced in high yield as soluble proteins by expression using a suitable biological production system, such as *E*. *coli,* and can be formulated and/or stored at higher concentrations than conventional polypeptides, and with less aggregation and loss of activity.

In addition, ligands or dAb monomers that are resistant to aggregation can be produced more economically than other antigen- or epitope-binding polypeptides (e.g., conventional antibodies). For example, generally, preparation of antigen- or epitope-binding polypeptides intended for *in vivo* applications includes processes (e.g., gel filtration) that remove aggregated polypeptides. Failure to remove such aggregates can result in a preparation that is not suitable for *in vivo* applications because, for example, aggregates of an antigen-binding polypeptide that is intended to act as an antagonist can function as an agonist by inducing cross-linking or clustering of the target antigen. Protein aggregates can also reduce the efficacy of therapeutic polypeptide by inducing an immune response in the subject to which they are administered.

In contrast, the aggregation resistant ligands or dAb monomers of the invention can be prepared for *in vivo* applications without the need to include process steps that remove aggregates, and can be used in *in vivo* applications without the aforementioned disadvantages caused by polypeptide aggregates.

In some embodiments, the ligand or dAb monomer unfolds reversibly when heated to a temperature (Ts) and cooled to a temperature (Tc), wherein Ts is greater than the melting temperature (Tm) of the dAb, and Tc is lower than the melting temperature of the dAb. For example, the dAb monomer can unfold reversibly when heated to 80°C and cooled to about room temperature. A polypeptide that unfolds reversibly loses function when unfolded but regains function upon refolding. Such polypeptides are distinguished from polypeptides that aggregate when unfolded or that improperly refold (misfolded polypeptides), *i.e.,* do not regain function.

Polypeptide unfolding and refolding can be assessed, for example, by directly or indirectly detecting polypeptide structure using any suitable method. For example, polypeptide structure can be detected by circular dichroism (CD) (*e.g*., far-UV CD, near-UV CD), fluorescence (*e.g*., fluorescence of tryptophan side chains), susceptibility to proteolysis, nuclear magnetic resonance (NMR), or by detecting or measuring a polypeptide function that is dependent upon proper folding (e.g., binding to target ligand, binding to generic ligand). In one example, polypeptide unfolding is assessed using a functional assay in which loss of binding function (*e.g.*, binding a generic and/or target ligand, binding a substrate) indicates that the polypeptide is unfolded.

The extent of unfolding and refolding of a ligand or dAb monomer can be determined using an unfolding or denaturation curve. An unfolding curve can be produced by plotting temperature as the ordinate and the relative concentration of folded polypeptide as the abscissa. The relative concentration of folded ligand or dAb monomer can be determined directly or indirectly using any suitable method (*e.g.,* CD, fluorescence, binding assay). For example, a ligand or dAb monomer solution can be prepared and ellipticity of the solution determined by CD. The ellipticity value obtained represents a relative concentration of folded ligand or dAb monomer of 100%. The ligand or dAb monomer in the solution is then unfolded by incrementally raising the temperature of the solution and ellipticity is determined at suitable increments (*e.g*., after each increase of one degree in temperature). The ligand or dAb monomer in solution is then refolded by incrementally reducing the temperature of the solution and ellipticity is determined at suitable increments. The data can be plotted to produce an unfolding curve and a refolding curve. The unfolding and refolding curves have a characteristic sigmoidal shape that includes a portion in which the ligand or dAb monomer molecules are folded, an unfolding/refolding transition in which ligand or dAb monomer molecules are unfolded to various degrees, and a portion in which the ligand or dAb monomer molecules are unfolded. The y-axis intercept of the refolding curve is the relative amount of refolded ligand or dAb monomer recovered. A recovery of at least about 50%, or at least about 60%, or at least about 70%, or at least about 75%, or at least about 80%, or at least about 85%, or at least about 90%, or at least about 95% is indicative that the ligand or dAb monomer unfolds reversibly.

In a preferred embodiment, reversibility of unfolding of the ligand or dAb monomer is determined by preparing a ligand or dAb monomer solution and plotting heat unfolding and refolding curves. The ligand or dAb monomer solution can be prepared in any suitable solvent, such as an aqueous buffer that has a pH suitable to allow the ligand or dAb monomer to dissolve (*e.g*., pH that is about 3 units above or below the isoelectric point (pI)). The ligand or dAb monomer solution is concentrated enough to allow unfolding/folding to be detected. For example, the ligand or dAb monomer solution can be about 0.1 µM to about 100 µM, or preferably about 1 µM to about 10 µM.

If the melting temperature (Tm) of the ligand or dAb monomer is known, the solution can be heated to about ten degrees below the Tm (Tm-10) and folding assessed by ellipticity or fluorescence (e.g., far-UV CD scan from 200 nm to 250 nm, fixed wavelength CD at 235 nm or 225 nm; tryptophan fluorescent emission spectra at 300 to 450 nm with excitation at 298 nm) to provide 100% relative folded ligand or dAb monomer. The solution is then heated to at least ten degrees above Tm (Tm+10) in predetermined increments (*e.g*., increases of about 0.1 to about 1 degree), and ellipticity or fluorescence is determined at each increment. Then, the ligand or dAb monomer is refolded by cooling to at least Tm-10 in predetermined increments and ellipticity or fluorescence determined at each increment. If the melting temperature of the ligand or dAb monomer is not known, the solution can be unfolded by incrementally heating from about 25°C to about 100°C and then refolded by incrementally cooling to at least about 25°C, and ellipticity or fluorescence at each heating and cooling increment is determined. The data obtained can be plotted to produce an unfolding curve and a refolding curve, in which the y-axis intercept of the refolding curve is the relative amount of refolded protein recovered.

In certain embodiments, the ligands or dAb monomers of the invention are efficacious in models of chronic inflammatory diseases when an effective amount is administered. Generally an effective amount is about 1 mg/kg to about 10 mg/kg (e.g., about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, or about 10 mg/kg). The models of chronic inflammatory disease described herein are recognized by those skilled in the art as being predictive of therapeutic efficacy in humans. The prior art does not suggest using antagonists of TNFR1 (e.g., monovalent antagonists, ligands as described herein) in these models, or that they would be efficacious.

In particular embodiments, the ligand or dAb monomer is efficacious in the standard mouse collagen-induced arthritis model (Example 15A). For example, administering an effective amount of the ligand can reduce the average arthritic score of the summation of the four limbs in the standard mouse collagen-induced arthritis model, for example, by about 1 to about 16, about 3 to about 16, about 6 to about 16, about 9 to about 16, or about 12 to about 16, as compared to a suitable control. In another example, administering an effective amount of the ligand can delay the onset of symptoms of arthritis in the standard mouse collagen-induced arthritis model, for example, by about 1 day, about 2 days, about 3 days, about 4 days, about 5 days, about 6 days, about 7 days, about 10 days, about 14 days, about 21 days or about 28 days, as compared to a suitable control. In another example, administering an effective amount of the ligand can result in an average arthritic score of the summation of the four limbs in the standard mouse collagen-induced arthritis model of 0 to about 3, about 3 to about 5, about 5 to about 7, about 7 to about 15, about 9 to about 15, about 10 to about 15, about 12 to about 15, or about 14 to about 15.

In other embodiments, the ligand or dAb monomer is efficacious in the mouse ΔARE model of arthritis (Example 15B). For example, administering an effective amount of the ligand can reduce the average arthritic score in the mouse ΔARE model of arthritis, for example, by about 0.1 to about 2.5, about 0.5 to about 2.5, about 1 to about 2.5, about 1.5 to about 2.5, or about 2 to about 2.5, as compared to a suitable control. In another example, administering an effective amount of the ligand can delay the onset of symptoms of arthritis in the mouse ΔARE model of arthritis by, for example, about 1 day, about 2 days, about 3 days, about 4 days, about 5 days, about 6 days, about 7 days, about 10 days, about 14 days, about 21 days or about 28 days, as compared to a suitable control. In another example, administering an effective amount of the ligand can result in an average arthritic score in the mouse ΔARE model of arthritis of 0 to about 0.5, about 0.5 to about 1, about 1 to about 1.5, about 1.5 to about 2, or about 2 to about 2.5.

In other embodiments, the ligand or dAb monomer is efficacious in the mouse ΔARE model of inflammatory bowel disease (IBD) (Example 15B). For example, administering an effective amount of the ligand can reduce the average acute and/or chronic inflammation score in the mouse ΔARE model of IBD, for example, by about 0.1 to about 2.5, about 0.5 to about 2.5, about 1 to about 2.5, about 1.5 to about 2.5, or about 2 to about 2.5, as compared to a suitable control. In another example, administering an effective amount of the ligand can delay the onset of symptoms of IBD in the mouse ΔARE model of IBD by, for example, about 1 day, about 2 days, about 3 days, about 4 days, about 5 days, about 6 days, about 7 days, about 10 days, about 14 days, about 21 days or about 28 days, as compared to a suitable control. In another example, administering an effective amount of the ligand can result in an average acute and/or chronic inflammation score in the mouse ΔARE model of IBD of 0 to about 0.5, about 0.5 to about 1, about 1 to about 1.5, about 1.5 to about 2, or about 2 to about 2.5.

In other embodiments, the ligand or dAb monomer is efficacious in the mouse dextran sulfate sodium (DSS) induced model of IBD (Example 15C). For example, administering an effective amount of the ligand can reduce the average severity score in the mouse DSS model of IBD, for example, by about 0.1 to about 2.5, about 0.5 to about 2.5, about 1 to about 2.5, about 1.5 to about 2.5, or about 2 to about 2.5, as compared to a suitable control. In another example, administering an effective amount of the ligand can delay the onset of symptoms of IBD in the mouse DSS model of IBD by, for example, about 1 day, about 2 days, about 3 days, about 4 days, about 5 days, about 6 days, about 7 days, about 10 days, about 14 days, about 21 days or about 28 days, as compared to a suitable control. In another example, administering an effective amount of the ligand can result in an average severity score in the mouse DSS model of IBD of 0 to about 0.5, about 0.5 to about 1, about 1 to about 1.5, about 1.5 to about 2, or about 2 to about 2.5.

In particular embodiments, the ligand or dAb monomer is efficacious in the mouse tobacco smoke model of chronic obstructive pulmonary disease (COPD) (Example 15D). For example, administering an effective amount of the ligand can reduce or delay onset of the symptoms of COPD, as compared to a suitable control.

In other embodiments, the ligand comprises a domain antibody (dAb) monomer that specifically binds Tumor Necrosis Factor Receptor I (TNFR1, p55, CD120a) with a K_{d} of 300 nM to 5 pM, and comprises an amino acid sequence that is at least 90%, at least about 91%, at least about 92%, at least about 93%, at least about 94%, at least about 95%, at least about 96%, at least about 97%, at least about 98%, or at least about 99% homologous to the amino acid sequence or a dAb which is TAR2h-205 (SEQ ID NO:627).

Also described herein are ligands comprising a dAb monomer that binds TNFR1 and competes with any of the dAbs disclosed herein for binding to TNFR1 (e.g., mouse and/or human TNFR1).

Preferably, the ligand or dAb monomer is secreted in a quantity of at least about 0.5 mg/L when expressed in *E*. *coli* or in *Pichia* species (e.g., *P. pastoris).* In other preferred embodiments, the dAb monomer is secreted in a quantity of at least about 0.75 mg/L, at least about 1 mg/L, at least about 4 mg/L, at least about 5 mg/L, at least about 10 mg/L, at least about 15 mg/L, at least about 20 mg/L, at least about 25 mg/L, at least about 30 mg/L, at least about 35 mg/L, at least about 40 mg/L, at least about 45 mg/L, or at least about 50 mg/L, or at least about 100 mg/L, or at least about 200 mg/L, or at least about 300 mg/L, or at least about 400 mg/L, or at least about 500 mg/L, or at least about 600 mg/L, or at least about 700 mg/L, or at least about 800 mg/L, at least about 900 mg/L, or at least about 1g/L when expressed in *E. coli* or in *Pichia* species (e.g., *P. pastoris).* In other preferred embodiments, the dAb monomer is secreted in a quantity of at least about 1 mg/L to at least about 1g/L, at least about 1 mg/L to at least about 750 mg/L, at least about 100 mg/L to at least about 1 g/L, at least about 200 mg/L to at least about 1 g/L, at least about 300 mg/L to at least about 1 g/L, at least about 400 mg/L to at least about 1 g/L, at least about 500 mg/L to at least about 1g/L, at least about 600 mg/L to at least about 1 g/L, at least about 700 mg/L to at least about 1 g/L, at least about 800 mg/L to at least about 1g/L, or at least about 900 mg/L to at least about 1g/L when expressed in *E. coli* or in *Pichia* species (e.g., *P. pastoris).* Although, the ligands and dAb monomers described herein can be secretable when expressed in *E. coli* or in *Pichia* species (e.g., *P. pastoris),* they can be produced using any suitable method, such as synthetic chemical methods or biological production methods that do not employ *E. coli* or *Pichia* species.

The dAb monomer can comprise any suitable immunoglobulin variable domain, and preferably comprises a human variable domain or a variable domain that comprises human framework regions. In certain embodiments, the dAb monomer comprises a universal framework, as described herein.

The universal framework can be a V_{L} framework (Vλ or Vκ), such as a framework that comprises the framework amino acid sequences encoded by the human germline DPK1, DPK2, DPK3, DPK4, DPK5, DPK6, DPK7, DPK8, DPK9, DPK10, DPK12, DPK13, DPK15, DPK16, DPK18, DPK19, DPK20, DPK21, DPK22, DPK23, DPK24, DPK25, DPK26 or DPK 28 immunoglobulin gene segment. If desired, the V_{L} framework can further comprises the framework amino acid sequence encoded by the human germline J_{κ}1, J_{κ}2, J_{κ}3, J_{κ}4, or J_{κ}5 immunoglobulin gene segment.

In other embodiments the universal framework can be a V_{H} framework, such as a framework that comprises the framework amino acid sequences encoded by the human germline DP4, DP7, DP8, DP9, DP10, DP31, DP33, DP38, DP45, DP46, DP47, DP49, DP50, DP51, DP53, DP54, DP65, DP66, DP67, DP68 or DP69 immunoglobulin gene segment. If desired, the V_{H} framework can further comprises the framework amino acid sequence encoded by the human germline J_{H}1, J_{H}2, J_{H}3, J_{H}4, J_{H}4b, J_{H}5 and J_{H}6 immunoglobulin gene segment.

In particular embodiments, the dAb monomer ligand comprises the DPK9 V_{L} framework, or a V_{H} framework selected from the group consisting of DP47, DP45 and DP38.

The dAb monomer can comprises a binding site for a generic ligand, such as protein A, protein L and protein G.

In certain embodiments, the dAb monomer comprises one or more framework regions comprising an amino acid sequence that is the same as the amino acid sequence of a corresponding framework region encoded by a human germline antibody gene segment, or the amino acid sequences of one or more of said framework regions collectively comprise up to 5 amino acid differences relative to the amino acid sequence of said corresponding framework region encoded by a human germline antibody gene segment.

In other embodiments, the amino acid sequences of FW1, FW2, FW3 and FW4 of the dAb monomer are the same as the amino acid sequences of corresponding framework regions encoded by a human germline antibody gene segment, or the amino acid sequences of FW1, FW2, FW3 and FW4 collectively contain up to 10 amino acid differences relative to the amino acid sequences of corresponding framework regions encoded by said human germline antibody gene segment.

In other embodiments, the dAb monomer comprises FW1, FW2 and FW3 regions, and the amino acid sequence of said FW1, FW2 and FW3 regions are the same as the amino acid sequences of corresponding framework regions encoded by human germline antibody gene segments.

In some embodiments, the dAb monomer does not comprise a *Camelid* immunoglobulin variable domain, or one or more framework amino acids that are unique to immunoglobulin variable domains encoded by *Camelid* germline antibody gene segments.

### Ligands and dAb Monomers that Bind Serum Albumin

The invention provides a ligand or dAb monomer (*e.g*., dual specific ligand comprising such a dAb) that binds to serum albumin (SA) with a K_{d} of 1nM to 500 µM (ie, x 10⁻⁹ to 5 x 10⁻⁴), preferably 100 nM to 10 µM. Preferably, for a dual specific ligand comprising a first anti-SA dAb and a second dAb to another target, the affinity (eg K_{d} and/or K_{off} as measured by surface plasmon resonance, eg using BiaCore) of the second dAb for its target is from 1 to 100000 times (preferably 100 to 100000, more preferably 1000 to 100000, or 10000 to 100000 times) the affinity of the first dAb for SA. For example, the first dAb binds SA with an affinity of approximately 10 µM, while the second dAb binds its target with an affinity of 100 pM. Preferably, the serum albumin is human serum albumin (HSA). In one embodiment, the first dAb (or a dAb monomer) binds SA (eg, HSA) with a K_{d} of approximately 50, preferably 70, and more preferably 100, 150 or 200 nM.

In certain embodiments, the dAb monomer specific for SA comprises the amino acid sequence of MSA-16 (SEQ ID NO:28) or a sequence that is at least 80%, at least 85%, at least 90%, at least 91%, at least 92%, at least 93%, at least 94%, at least 95%, at least 96%, at least 97%, at least 98%, or at least 99% homologous thereto.

In other embodiments, the dAb monomer specific for SA comprises the amino acid sequence of MSA-26 (SEQ ID NO:30) or a sequence that is at least 80%, at least 85%, at least 90%, at least 91%, at least 92%, at least 93%, at least 94%, at least 95%, at least 96%, at least 97%, at least 98%, or at least 99% homologous thereto.

In certain embodiments, the dAb monomer that binds SA resists aggregation, unfolds reversibly and/or comprises a framework region as described above for dAb monomers that bind TNFR1.

### Nucleic Acid Molecules, Vectors and Host Cells

The invention also provides isolated and/or recombinant nucleic acid molecules that encode the ligands and dAb monomers described herein.

The invention also provides a vector comprising a recombinant nucleic acid molecule of the invention. In certain embodiments, the vector is an expression vector comprising one or more expression control elements or sequences that are operably linked to the recombinant nucleic acid of the invention. Suitable vectors (e.g., plasmids, phagmids) and expression control elements are further described below.

The invention also provides a recombinant host cell comprising a recombinant nucleic acid molecule or vector of the invention. Suitable host cells and methods for producing the recombinant host cell of the invention are further described below.

The invention also provides a method for producing a ligand (e.g., dAb monomer, dual-specific ligand, multispecific ligand) of the invention, comprising maintaining a recombinant host cell comprising a recombinant nucleic acid of the invention under conditions suitable for expression of the recombinant nucleic acid, whereby the recombinant nucleic acid is expressed and a ligand is produced. In some embodiments, the method further comprises isolating the ligand.

### Ligand Formats

Ligands according to the invention can be formatted as mono or multispecific antibodies or antibody fragments or into mono or multispecific non-immunoglobulin structures. Suitable formats include, any suitable polypeptide structure in which an antibody variable domain or the CDR thereof can be incorporated so as to confer binding specificity for antigen on the structure. A variety of suitable antibody formats are known in the art, such as, IgG-like formats, chimeric antibodies, humanized antibodies, human antibodies, single chain antibodies, bispecific antibodies, antibody heavy chains, antibody light chains, homodimers and heterodimers of antibody heavy chains and/or light chains, antigen-binding fragments of any of the foregoing (*e.g*., a Fv fragment (*e.g*., single chain Fv (scFv), a disulfide bonded Fv), a Fab fragment, a Fab' fragment, a F(ab')2 fragment), a single variable domain (*e.g*., V_{H}, V_{L}), a dAb, and modified versions of any of the foregoing (*e.g*., modified by the covalent attachment ofpolyalkylene glycol (*e.g*., polyethylene glycol, polypropylene glycol, polybutylene glycol) or other suitable polymer). See, PCT/GB03/002804, filed June 30, 2003, which designated the United States, (WO 2004/081026) regarding PEGylated of single variable domains and dAbs, suitable methods for preparing same, increased *in vivo* half life of the PEGylated single variable domains and dAb monomers and multimers, suitable PEGs, preferred hydrodynamic sizes of PEGs, and preferred hydrodynamic sizes of PEGylated single variable domains and dAb monomers and multimers.

In some embodiments, the ligand is an IgG-like format. Such formats have the conventional four chain structure of an IgG molecule (2 heavy chains and two light chains), in which one or more of the variable regions (V_{H} and or V_{L}) have been replaced with a dAb or single variable domain of a desired specificity. Preferably, each of the variable regions (2 V_{H} regions and 2 V_{L} regions) is replaced with a dAb or single variable domain. The dAb(s) or single variable domain(s) that are included in an IgG-like format can have the same specificity or different specificities. In some embodiments, the IgG-like format is tetravalent and can have one, two, three or four specificities. For example, the IgG-like format can be monospecific and comprises 4 dAbs that have the same specificity; bispecific and comprises 3 dAbs that have the same specificity and another dAb that has a different specificity; bispecific and comprise two dAbs that have the same specificity and two dAbs that have a common but different specificity; trispecific and comprises first and second dAbs that have the same specificity, a third dAbs with a different specificity and a fourth dAb with a different specificity from the first, second and third dAbs; or tetraspecific and comprise four dAbs that each have a different specificity. Antigen-binding fragments of IgG-like formats (e.g., Fab, F(ab')₂, Fab', Fv, scFv) can be prepared. Preferably, the IgG-like formats or antigen-binding fragments thereof do not crosslink TNFR1.

Ligands, including dAb monomers, dimers and trimers, can be linked to an antibody Fc region, comprising one or both of C_{H}2 and C_{H}3 domains, and optionally a hinge region. For example, vectors encoding ligands linked as a single nucleotide sequence to an Fc region may be used to prepare such polypeptides.

The disclosure moreover provides dimers, trimers and polymers of the aforementioned dAb monomers, in accordance with the following aspects of the present invention.

Ligands may be combined and/or formatted into non-immunoglobulin multi-ligand structures to form multivalent complexes, which bind target molecules with the same antigen, thereby providing superior avidity. In some embodiments, at least one variable domain binds an antigen to increase the half life of the multimer. For example natural bacterial receptors such as SpA have been used as scaffolds for the grafting of CDRs to generate ligands which bind specifically to one or more epitopes. Details of this procedure are described in US 5,831,012. Other suitable scaffolds include those based on fibronectin and affibodies. Details of suitable procedures are described in WO 98/58965. Other suitable scaffolds include lipocallin and CTLA4, as described in van den Beuken et al., J. Mol. Biol. (2001) 310, 591-601, and scaffolds such as those described in WO 00/69907 (Medical Research Council), which are based for example on the ring structure of bacterial GroEL or other chaperone polypeptides.

Protein scaffolds may be combined; for example, CDRs may be grafted on to a CTLA4 scaffold and used together with immunoglobulin V_{H} or V_{L} domains to form a ligand. Likewise, fibronectin, lipocallin and other scaffolds may be combined.

### Dual- and Multi-Specific Ligands

The inventors have described, in their copending international patent application WO 03/002609 as well as copending unpublished UK patent application 0230203.2, dual specif immunoglobulin ligands which comprise immunoglobulin single variable domains which each have different specificities. The domains may act in competition with each other or independently to bind antigens or epitopes on target molecules.

Antigens or epitopes which increase the half-life of a ligand as described herein are advantageously present on proteins or polypeptides found in an organism *in vivo.* Examples include extracellular matrix proteins, blood proteins, and proteins present in various tissues in the organism. The proteins act to reduce the rate of ligand clearance from the blood, for example by acting as bulking agents, or by anchoring the ligand to a desired site of action. Examples of antigens/epitopes which increase half-life *in vivo* are given in Annex 1 below.

Increased half-life is useful *in in vivo* applications of immunoglobulins, especially antibodies and most especially antibody fragments of small size. Such fragments (Fvs, disulphide bonded Fvs, Fabs, scFvs, dAbs) suffer from rapid clearance from the body; thus, whilst they are able to reach most parts of the body rapidly, and are quick to produce and easier to handle, their *in vivo* applications have been limited by their only brief persistence *in vivo.* The invention solves this problem by providing increased half-life of the ligands *in vivo* and consequently longer persistence times in the body of the functional activity of the ligand.

Methods for pharmacokinetic analysis and determination of ligand half-life will be familiar to those skilled in the art. Details may be found in Kenneth, A et al: Chemical Stability of Pharmaceuticals: A Handbook for Pharmacists and in Peters et al, Pharmacokinete analysis: A Practical Approach (1996). Reference is also made to "Pharmacokinetics", M Gibaldi & D Perron, published by Marcel Dekker, 2nd Rev. ex edition (1982), which describes pharmacokinetic parameters such as t alpha and t beta half lives and area under the curve (AUC).

Half lives (t½ alpha and t½ beta) and AUC can be determined from a curve of serum concentration of ligand against time. The WinNonlin analysis package (available from Pharsight Corp., Mountain View, CA94040, USA) can be used, for example, to model the curve. In a first phase (the alpha phase) the ligand is undergoing mainly distribution in the patient, with some elimination. A second phase (beta phase) is the terminal phase when the ligand has been distributed and the serum concentration is decreasing as the ligand is cleared from the patient. The t alpha half life is the half life of the first phase and the t beta half life is the half life of the second phase. Thus, advantageously, the present invention provides a ligand or a composition comprising a ligand according to the invention having a tα half-life in the range of 15 minutes or more. In one embodiment, the lower end of the range is 30 minutes, 45 minutes, 1 hour, 2 hours, 3 hours, 4 hours, 5 hours, 6 hours, 7 hours, 10 hours, 11 hours or 12 hours. In addition, or alternatively, a ligand or composition according to the invention will have a tα half life in the range of up to and including 12 hours. In one embodiment, the upper end of the range is 11, 10, 9, 8, 7, 6 or 5 hours. An example of a suitable range is 1 to 6 hours, 2 to 5 hours or 3 to 4 hours.

Advantageously, the present disclosure provides a ligand or a composition comprising a ligand according to the disclosure having a tβ half-life in the range of 2.5 hours or more. In one embodiment, the lower end of the range is 3 hours, 4 hours, 5 hours, 6 hours, 7 hours, 10 hours , 11 hours, or 12 hours. In addition, or alternatively, a ligand or composition has a tβ half-life in the range of up to and including 21 days. In one embodiment, the upper end of the range is 12 hours, 24 hours, 2 days, 3 days, 5 days, 10 days, 15 days or 20 days. Advantageously a ligand or composition according to the invention will have a tβ half life in the range 12 to 60 hours. In a further embodiment, it will be in the range 12 to 48 hours. In a further embodiment still, it will be in the range 12 to 26 hours.

In addition, or alternatively to the above criteria, the present disclosure provides a ligand or a composition comprising a ligand according to the invention having an AUC value (area under the curve) in the range of 1 mg.min/ml or more. In one embodiment, the lower end of the range is 5, 10, 15, 20, 30, 100, 200 or 300 mg.min/ml. In addition, or alternatively, a ligand or composition has an AUC in the range of up to 600 mg.min/ml. In one embodiment, the upper end of the range is 500, 400, 300, 200, 150, 100, 75 or 50 mg.min/ml. Advantageously a ligand will have a AUC in the range selected from the group consisting of the following: 15 to 150 mg.min/ml, 15 to 100 mg.min/ml, 15 to 75 mg.min/ml, and 15 to 50mg.min/ml.

The dual specific ligands of the invention do not include ligands as described in WO 02/02773. Thus, the ligands of the present invention do not comprise complementary V_{H}/V_{L} pairs which bind any one or more antigens or epitopes cooperatively. Instead, the ligands according to the first aspect of the invention comprise a V_{H}/V_{L} complementary pair, wherein the V domains have different specificities. Moreover, the ligands according to the first aspect of the invention comprise V_{H}/V_{L} complementary pairs having different specificities for non-structurally related epitopes or antigens. Structurally related epitopes or antigens are epitopes or antigens which possess sufficient structural similarity to be bound by a conventional V_{H}/V_{L} complementary pair which acts in a co-operative manner to bind an antigen or epitope; in the case of structurally related epitopes, the epitopes are sufficiently similar in structure that they "fit" into the same binding pocket formed at the antigen binding site of the V_{H}/V_{L} dimer.

The present invention also provides a ligand comprising a first immunoglobulin variable domain having a first antigen or epitope binding specificity and a second immunoglobulin variable domain having a second antigen or epitope binding specificity wherein one of said first and second variable domains bind to an antigen which increases the half-life of the ligand *in vivo,* and the variable domains are not complementary to one another.

The first and the second antigen binding domains may be associated either covalently or non-covalently. In the case that the domains are covalently associated, then the association may be mediated for example by disulphide bonds or by a polypeptide linker such as (Gly₄Ser)ₙ, where n = from 1 to 8, e.g., 2, 3, 4, 5 or 7.

In the case that the variable domains are selected from V-gene repertoires selected for instance using phage display technology as herein described, then these variable domains can comprise a universal framework region, such that they may be recognised by a specific generic ligand as herein defined. The use of universal frameworks, generic ligands and the like is described in WO 99/20749. In the present invention, reference to phage display includes the use of both phage and/or phagemids.

Where V-gene repertoires are used variation in polypeptide sequence is preferably located within the structural loops of the variable domains. The polypeptide sequences of either variable domain may be altered by DNA shuffling or by mutation in order to enhance the interaction of each variable domain with its complementary pair. DNA shuffling is known in the art and taught, for example, by Stemmer, Nature 370:389-391 (1994) and U.S. Patent No. 6,297,053, both of which are incorporated herein by reference. Other methods of mutagenesis are well known to those of skill in the art.

In a preferred embodiment the 'dual-specific ligand' is a single chain Fv fragment. In an alternative embodiment of the invention, the 'dual-specific ligand' consists of a Fab region of an antibody. The term "Fab region" includes a Fab-like region where two VH or two VL domains are used.

The variable regions may be derived from antibodies directed against target antigens or epitopes. Alternatively they may be derived from a repertoire of single antibody domains such as those expressed on the surface of filamentous bacteriophage. Selection may be performed as described below.

The present disclosure provides one or more nucleic acid molecules encoding at least a dual-specific ligand as herein defined. The dual specific ligand may be encoded on a single nucleic acid molecule; alternatively, each domain may be encoded by a separate nucleic acid molecule. Where the ligand is encoded by a single nucleic acid molecule, the domains may be expressed as a fusion polypeptide, in the manner of a scFv molecule, or may be separately expressed and subsequently linked together, for example using chemical linking agents. Ligands expressed from separate nucleic acids will be linked together by appropriate means.

The nucleic acid may further encode a signal sequence for export of the polypeptides from a host cell upon expression and may be fused with a surface component of a filamentous bacteriophage particle (or other component of a selection display system) upon expression.

In a further aspect the present disclosure provides a vector comprising nucleic acid encoding a dual specific ligand according to the present invention.

In a yet further aspect, the present disclosure provides a host cell transfected with a vector encoding a dual specific ligand according to the present invention.

Expression from such a vector may be configured to produce, for example on the surface of a bacteriophage particle, variable domains for selection. This allows selection of displayed variable regions and thus selection of 'dual-specific ligands' using the method of the present invention.

Also described herein is a method for producing a ligand comprising a first immunoglobulin single variable domain having a first binding specificity and a second single immunoglobulin single variable domain having a second (different) binding specificity, one or both of the binding specificities being specific for an antigen which increases the half-life of the ligand *in vivo,* the method comprising the steps of:
a) selecting a first variable domain by its ability to bind to a first epitope,
b) selecting a second variable region by its ability to bind to a second epitope,
c) combining the variable domains; and
d) selecting the ligand by its ability to bind to said first epitope and to said second epitope.

The ligand can bind to the first and second epitopes either simultaneously or, where there is competition between the binding domains for epitope binding, the binding of one domain may preclude the binding of another domain to its cognate epitope. In one embodiment, therefore, step (d) above requires simultaneous binding to both first and second (and possibly further) epitopes; in another embodiment, the binding to the first and second epitopes is not simultaneous.

The epitopes are preferably on separate antigens.

Ligands advantageously comprise V_{H}/V_{L} combinations, or V_{H}/V_{H} or V_{L}/V_{L} combinations of immunoglobulin variable domains, as described above. The ligands may moreover comprise camelid V_{HH} domains, provided that the V_{HH} domain which is specific for an antigen which increases the half-life of the ligand *in vivo* does not bind Hen egg white lysozyme (HEL), porcine pancreatic alpha-amylase or NmC-A; hcg, BSA-linked RR6 azo dye or *S*. *mutans* HG982 cells, as described in Conrath et al., (2001) JBC 276:7346-7350 and WO99/23221, neither of which describe the use of a specificity for an antigen which increases half-life to increase the half life of the ligand *in vivo.*

In one embodiment, said first variable domain is selected for binding to said first epitope in absence of a complementary variable domain. In a further embodiment, said first variable domain is selected for binding to said first epitope/antigen in the presence of a third variable domain in which said third variable domain is different from said second variable domain and is complementary to the first domain. Similarly, the second domain may be selected in the absence or presence of a complementary variable domain.

In one embodiment, the variable domains are derived from a respective antibody directed against the antigen or epitope. In a preferred embodiment the variable domains are derived from a repertoire of single variable antibody domains.

### Combining Single Variable Domains

Domains useful in the invention, once selected, may be combined by a variety of methods known in the art, including covalent and non-covalent methods.

Preferred methods include the use of polypeptide linkers, as described, for example, in connection with scFv molecules (Bird et al., (1988) Science 242:423-426). Discussion of suitable linkers is provided in Bird et al. Science 242, 423-426; Hudson et al, Journal Immunol Methods 231 (1999) 177-189; Hudson et al, Proc Nat Acad Sci USA 85, 5879-5883. Linkers are preferably flexible, allowing the two single domains to interact. One linker example is a (Gly₄ Ser)ₙ linker, where n=1 to 8, eg, 2, 3, 4, 5 or 7. The linkers used in diabodies, which are less flexible, may also be employed (Holliger et al., (1993) PNAS (USA) 90:6444-6448).

In one embodiment, the linker employed is not an immunoglobulin hinge region.

Variable domains may be combined using methods other than linkers. For example, the use of disulphide bridges, provided through naturally-occurring or engineered cysteine residues, may be exploited to stabilise V_{H}-V_{H'}V_{L}-V_{L} or V_{H}-V_{L} dimers (Reiter et al., (1994) Protein Eng. 7:697-704) or by remodelling the interface between the variable domains to improve the "fit" and thus the stability of interaction (Ridgeway et al., (1996) Protein Eng. 7:617-621; Zhu et al., (1997) Protein Science 6:781-788).

Other techniques for joining or stabilising variable domains of immunoglobulins, and in particular antibody V_{H} domains, may be employed as appropriate.

In accordance with the present invention, dual specific ligands can be in "closed" conformations in solution. A "closed" configuration is that in which the two domains (for example V_{H} and V_{L}) are present in associated form, such as that of an associated V_{H}-V_{L} pair which forms an antibody binding site. For example, scFv may be in a closed conformation, depending on the arrangement of the linker used to link the V_{H} and V_{L} domains. If this is sufficiently flexible to allow the domains to associate, or rigidly holds them in the associated position, it is likely that the domains will adopt a closed conformation.

Similarly, V_{H} domain pairs and V_{L} domain pairs may exist in a closed conformation. Generally, this will be a function of close association of the domains, such as by a rigid linker, in the ligand molecule. Ligands in a closed conformation will be unable to bind both the molecule which increases the half-life of the ligand and a second target molecule. Thus, the ligand will typically only bind the second target molecule on dissociation from the molecule which increases the half-life of the ligand. Moreover, the construction of V_{H}/V_{H}, V_{L}/V_{L} or V_{H}/V_{L} dimers without linkers provides for competition between the domains.

Ligands may moreover be in an open conformation. In such a conformation, the ligands will be able to simultaneously bind both the molecule which increases the half-life of the ligand and the second target molecule. Typically, variable domains in an open configuration are (in the case of V_{H}-V_{L} pairs) held far enough apart for the domains not to interact and form an antibody binding site and not to compete for binding to their respective epitopes. In the case of V_{H}/V_{H} or V_{L}/V_{L} dimers, the domains are not forced together by rigid linkers. Naturally, such domain pairings will not compete for antigen binding or form an antibody binding site.

Fab fragments and whole antibodies will exist primarily in the closed conformation, although it will be appreciated that open and closed dual specific ligands are likely to exist in a variety of equilibria under different circumstances. Binding of the ligand to a target is likely to shift the balance of the equilibrium towards the open configuration. Thus, certain ligands according to the invention can exist in two conformations in solution, one of which (the open form) can bind two antigens or epitopes independently, whilst the alternative conformation (the closed form) can only bind one antigen or epitope; antigens or epitopes thus compete for binding to the ligand in this conformation.

Although the open form of the dual specific ligand may thus exist in equilibrium with the closed form in solution, it is envisaged that the equilibrium will favour the closed form; moreover, the open form can be sequestered by target binding into a closed conformation. Preferably, therefore, certain dual specific ligands of the invention are present in an equilibrium between two (open and closed) conformations.

Dual specific ligands may be modified in order to favour an open or closed conformation. For example, stabilisation of V_{H}-V_{L} interactions with disulphide bonds stabilises the closed conformation. Moreover, linkers used to join the domains, including V_{H} domain and V_{L} domain pairs, may be constructed such that the open from is favoured; for example, the linkers may sterically hinder the association of the domains, such as by incorporation of large amino acid residues in opportune locations, or the designing of a suitable rigid structure which will keep the domains physically spaced apart.

### Characterisation of the Dual-specific Ligand

The binding of the dual-specific ligand to its specific antigens or epitopes can be tested by methods which will be familiar to those skilled in the art and include ELISA. In a preferred embodiment of the invention binding is tested using monoclonal phage ELISA.

Phage ELISA may be performed according to any suitable procedure: an exemplary protocol is set forth below.

Populations of phage produced at each round of selection can be screened for binding by ELISA to the selected antigen or epitope, to identify "polyclonal" phage antibodies. Phage from single infected bacterial colonies from these populations can then be screened by ELISA to identify "monoclonal" phage antibodies. It is also desirable to screen soluble antibody fragments for binding to antigen or epitope, and this can also be undertaken by ELISA using reagents, for example, against a C- or N-terminal tag (see for example Winter et al. (1994) Ann. Rev. Immunology 12, 433-55 and references cited therein.

The diversity of the selected phage monoclonal antibodies may also be assessed by gel electrophoresis of PCR products (Marks *et al.* 1991, *supra; Nissim et al.* 1994 *supra*)*,* probing (Tomlinson et al., 1992) J. Mol. Biol. 227, 776) or by sequencing of the vector DNA.

### Structure of Dual-specific Ligands

As described above, an antibody is herein defined as an antibody (for example IgG, IgM, IgA, IgA, IgE) or fragment (Fab, Fv, disulphide linked Fv, scFv, diabody) which comprises at least one heavy and a light chain variable domain, at least two heavy chain variable domains or at least two light chain variable domains. It may be at least partly derived from any species naturally producing an antibody, or created by recombinant DNA technology; whether isolated from serum, B-cells, hybridomas, transfectomas, yeast or bacteria).

In a preferred embodiment the dual-specific ligand comprises at least one single heavy chain variable domain of an antibody and one single light chain variable domain of an antibody, or two single heavy or light chain variable domains. For example, the ligand may comprise a V_{H}/V_{L} pair, a pair of V_{H} domains or a pair of V_{L} domains.

The first and the second variable domains of such a ligand may be on the same polypeptide chain. Alternatively they may be on separate polypeptide chains. In the case that they are on the same polypeptide chain they may be linked by a linker, which is preferentially a peptide sequence, as described above.

The first and second variable domains may be covalently or non-covalently associated. In the case that they are covalently associated, the covalent bonds may be disulphide bonds.

In the case that the variable domains are selected from V-gene repertoires selected for instance using phage display technology as herein described, then these variable domains comprise a universal framework region, such that is they may be recognised by a specific generic ligand as herein defined. The use of universal frameworks, generic ligands and the like is described in WO99/20749.

Where V-gene repertoires are used variation in polypeptide sequence is preferably located within the structural loops of the variable domains. The polypeptide sequences of either variable domain may be altered by DNA shuffling or by mutation in order to enhance the interaction of each variable domain with its complementary pair. DNA shuffling is known in the art and taught, for example, by Stemmer, 1994, Nature 370: 389-391 and U.S. Patent No. 6,297,053, both of which are incorporated herein by reference. Other methods of mutagenesis are well known to those of skill in the art.

In a preferred embodiment the 'dual-specific ligand' is a single chain Fv fragment. In an alternative embodiment of the invention, the 'dual-specific ligand' consists of a Fab format.

In a further aspect, the present disclosure provides nucleic acid encoding at least a 'dual-specific ligand' as herein defined.

One skilled in the art will appreciate that, both antigens or epitopes may bind simultaneously to the same antibody molecule. Alternatively, they may compete for binding to the same antibody molecule. For example, where both epitopes are bound simultaneously, both variable domains of a dual specific ligand are able to independently bind their target epitopes. Where the domains compete, the one variable domain is capable of binding its target, but not at the same time as the other variable domain binds its cognate target; or the first variable domain is capable of binding its target, but not at the same time as the second variable domain binds its cognate target.

The variable regions may be derived from antibodies directed against target antigens or epitopes. Alternatively they may be derived from a repertoire of single antibody domains such as those expressed on the surface of filamentous bacteriophage. Selection may be performed as described below.

In general, the nucleic acid molecules and vector constructs required for the performance of the present invention may be constructed and manipulated as set forth in standard laboratory manuals, such as Sambrook et al. (1989) Molecular Cloning: A Laboratory Manual, Cold Spring Harbor, USA.

The manipulation of nucleic acids useful in the present invention is typically carried out in recombinant vectors.

Thus in a further aspect, the present disclosure provides a vector comprising nucleic acid encoding at least a 'dual-specific ligand' as herein defined.

As used herein, vector refers to a discrete element that is used to introduce heterologous DNA into cells for the expression and/or replication thereof. Methods by which to select or construct and, subsequently, use such vectors are well known to one of ordinary skill in the art. Numerous vectors are publicly available, including bacterial plasmids, bacteriophage, artificial chromosomes and episomal vectors. Such vectors may be used for simple cloning and mutagenesis; alternatively gene expression vector is employed. A vector of use according to the invention may be selected to accommodate a polypeptide coding sequence of a desired size, typically from 0.25 kilobase (kb) to 40 kb or more in length A suitable host cell is transformed with the vector after *in vitro* cloning manipulations. Each vector contains various functional components, which generally include a cloning (or "polylinker") site, an origin of replication and at least one selectable marker gene. If given vector is an expression vector, it additionally possesses one or more of the following: enhancer element, promoter, transcription termination and signal sequences, each positioned in the vicinity of the cloning site, such that they are operatively linked to the gene encoding a ligand according to the invention.

Both cloning and expression vectors generally contain nucleic acid sequences that enable the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses. The origin of replication from the plasmid pBR322 is suitable for most Gram-negative bacteria, the 2 micron plasmid origin is suitable for yeast, and various viral origins (e.g. SV 40, adenovirus) are useful for cloning vectors in mammalian cells. Generally, the origin of replication is not needed for mammalian expression vectors unless these are used in mammalian cells able to replicate high levels of DNA, such as COS cells.

Advantageously, a cloning or expression vector may contain a selection gene also referred to as selectable marker. This gene encodes a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins, e.g. ampicillin, neomycin, methotrexate or tetracycline; complement auxotrophic deficiencies, or supply critical nutrients not available in the growth media.

Since the replication of vectors encoding a ligand according to the present invention is most conveniently performed in *E. coli,* an *E. coli*-selectable marker, for example, the β-lactamase gene that confers resistance to the antibiotic ampicillin, is of use. These can be obtained from *E. coli* plasmids, such as pBR322 or a pUC plasmid such as pUC 18 or pUC19.

Expression vectors usually contain a promoter that is recognised by the host organism and is operably linked to the coding sequence of interest. Such a promoter may be inducible or constitutive. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences.

Promoters suitable for use with prokaryotic hosts include, for example, the β-lactamase and lactose promoter systems, alkaline phosphatase, the tryptophan (trp) promoter system and hybrid promoters such as the tac promoter. Promoters for use in bacterial systems will also generally contain a Shine-Delgarno sequence operably linked to the coding sequence.

The preferred vectors are expression vectors that enables the expression of a nucleotide sequence corresponding to a polypeptide library member. Thus, selection with the first and/or second antigen or epitope can be performed by separate propagation and expression of a single clone expressing the polypeptide library member or by use of any selection display system. As described above, the preferred selection display system is bacteriophage display. Thus, phage or phagemid vectors may be used, eg pIT1 or pIT2. Leader sequences useful in the invention include pelB, stII, ompA, phoA, bla and pelA. One example are phagemid vectors which have an *E. coli.* origin of replication (for double stranded replication) and also a phage origin of replication (for production of single-stranded DNA). The manipulation and expression of such vectors is well known in the art (Hoogenboom and Winter (1992) supra; Nissim *et al.* (1994) supra). Briefly, the vector contains a β-lactamase gene to confer selectivity on the phagemid and a lac promoter upstream of a expression cassette that consists (N to C terminal) of a pelB leader sequence (which directs the expressed polypeptide to the periplasmic space), a multiple cloning site (for cloning the nucleotide version of the library member), optionally, one or more peptide tag (for detection), optionally, one or more TAG stop codon and the phage protein pIII. Thus, using various suppressor and non-suppressor strains of *E. coli* and with the addition of glucose, iso-propyl thio-β-D-galactoside (IPTG) or a helper phage, such as VCS M13, the vector is able to replicate as a plasmid with no expression, produce large quantities of the polypeptide library member only or produce phage, some of which contain at least one copy of the polypeptide-pIII fusion on their surface.

Construction of vectors encoding ligands according to the invention employs conventional ligation techniques. Isolated vectors or DNA fragments are cleaved, tailored, and religated in the form desired to generate the required vector. If desired, analysis to confirm that the correct sequences are present in the constructed vector can be performed in a known fashion. Suitable methods for constructing expression vectors, preparing *in vitro* transcripts, introducing DNA into host cells, and performing analyses for assessing expression and function are known to those skilled in the art. The presence of a gene sequence in a sample is detected, or its amplification and/or expression quantified by conventional methods, such as Southern or Northern analysis, Western blotting, dot blotting of DNA, RNA or protein, *in situ* hybridisation, immunocytochemistry or sequence analysis of nucleic acid or protein molecules. Those skilled in the art will readily envisage how these methods may be modified, if desired.

### Antigens Capable of Increasing Ligand Half-life

The dual specific ligands according to the invention, in one configuration thereof, are capable of binding to one or more molecules which can increase the half-life of the ligand *in vivo.* Typically, such molecules are polypeptides which occur naturally *in vivo* and which resist degradation or removal by endogenous mechanisms which remove unwanted material from the organism. For example, the molecule which increases the half-life of the organism may be selected from the following:
Proteins from the extracellular matrix; for example collagen, laminins, integrins and fibronectin. Collagens are the major proteins of the extracellular matrix. About 15 types of collagen molecules are currently known, found in different parts of the body, eg type I collagen (accounting for 90% of body collagen) found in bone, skin, tendon, ligaments, cornea, internal organs or type II collagen found in cartilage, invertebral disc, notochord, vitreous humour of the eye.

Proteins found in blood, including:
Plasma proteins such as fibrin, α-2 macroglobulin, serum albumin, fibrinogen A, fibrinogen B, serum amyloid protein A, heptaglobin, profilin, ubiquitin, uteroglobulin and β-2-microglobulin;

Enzymes and inhibitors such as plasminogen, lysozyme, cystatin C, alpha-1-antitrypsin and pancreatic trypsin inhibitor. Plasminogen is the inactive precursor of the trypsin-like serine protease plasmin. It is normally found circulating through the blood stream. When plasminogen becomes activated and is converted to plasmin, it unfolds a potent enzymatic domain that dissolves the fibrinogen fibers that entangle the blood cells in a blood clot. This is called fibrinolysis.

Immune system proteins, such as IgE, IgG, IgM.

Transport proteins such as retinol binding protein, α-1 microglobulin.

Defensins such as beta-defensin 1, Neutrophil defensins 1,2 and 3.

Proteins found at the blood brain barrier or in neural tissues, such as melanocortin receptor, myelin, ascorbate transporter.

Transferrin receptor specific ligand-neuropharmaceutical agent fusion proteins (see US5977307);

brain capillary endothelial cell receptor, transferrin, transferrin receptor, insulin, insulin-like growth factor 1 (IGF 1) receptor, insulin-like growth factor 2 (IGF 2) receptor, insulin receptor.

Proteins localised to the kidney, such as polycystin, type IV collagen, organic anion transporter K1, Heymann's antigen.

Proteins localised to the liver, for example alcohol dehydrogenase, G250.

Blood coagulation factor X
α1 antitrypsin
TNF 1α

Proteins localised to the lung, such as secretory component (binds IgA).

Proteins localised to the Heart, for example HSP 27. This is associated with dilated cardiomyopathy.

Proteins localised to the skin, for example keratin.

Bone specific proteins, such as bone morphogenic proteins (BMPs), which are a subset of the transforming growth factor β superfamily that demonstrate osteogenic activity. Examples include BMP-2, -4, -5, -6, -7 (also referred to as osteogenic protein (OP-1) and -8 (OP-2).

Tumour specific proteins, including human trophoblast antigen, herceptin receptor, oestrogen receptor, cathepsins eg cathepsin B (found in liver and spleen).

Disease-specific proteins, such as antigens expressed only on activated T-cells: including LAG-3 (lymphocyte activation gene), osteoprotegerin ligand (OPGL) see Nature 402, 304-309; 1999, OX40 (a member of the TNF receptor family, expressed on activated T cells and the only costimulatory T cell molecule known to be specifically up-regulated in human T cell leukaemia virus type-I (HTLV-I)-producing cells.) *See* J Immunol. 2000 Jul 1;165(1):263-70*;* Metalloproteases (associated with arthritis/cancers), including CG6512 Drosophila, human paraplegin, human FtsH, human AFG3L2, murine ftsH; angiogenic growth factors, including acidic fibroblast growth factor (FGF-1), basic fibroblast growth factor (FGF-2), Vascular endothelial growth factor / vascular permeability factor (VEGF/VPF), transforming growth factor-a (TGF a), tumor necrosis factor-alpha (TNF-α), angiogenin, interleukin-3 (IL-3), interleukin-8 (IL-8), platelet-derived endothelial growth factor (PD-ECGF), placental growth factor (PlGF), midkine platelet-derived growth factor-BB (PDGF), fractalkine.

### Stress proteins (heat shock proteins)

HSPs are normally found intracellularly. When they are found extracellularly, it is an indicator that a cell has died and spilled out its contents. This unprogrammed cell death (necrosis) only occurs when as a result of trauma, disease or injury and therefore *in vivo,* extracellular HSPs trigger a response from the immune system that will fight infection and disease. A dual specific which binds to extracellular HSP can be localised to a disease site.

Proteins involved in Fc transport

Brambell receptor (also known as FcRB)

This Fc receptor has two functions, both of which are potentially useful for delivery

The functions are

The transport of IgG from mother to child across the placenta

the protection of IgG from degradation thereby prolonging its serum half life of IgG. It is thought that the receptor recycles IgG from endosome.

See Holliger et al, Nat Biotechnol 1997 Jul;15(7):632-6.

Ligands may designed to be specific for the above targets without requiring any increase in or increasing half life *in vivo*. For example, ligands according to the invention can be specific for targets selected from the foregoing which are tissue-specific, thereby enabling tissue-specific targeting of the dual specific ligand, or a dAb monomer that binds a tissue-specific therapeutically relevant target, irrespective of any increase in half-life, although this may result. Moreover, where the ligand or dAb monomer targets kidney or liver, this may redirect the ligand or dAb monomer to an alternative clearance pathway *in vivo* (for example, the ligand may be directed away from liver clearance to kidney clearance).

### Use of Multispecific Ligands According to the Second Configuration of the Invention

Multispecific ligands according to the method of the second configuration of the present invention may be employed in *in vivo* therapeutic and prophylactic applications, *in vitro* and *in vivo* diagnostic applications, *in vitro* assay and reagent applications, and the like. For example antibody molecules may be used in antibody based assay techniques, such as ELISA techniques, according to methods known to those skilled in the art.

As alluded to above, the multispecific ligands according to the invention are of use in diagnostic, prophylactic and therapeutic procedures. Multispecific antibodies according to the invention are of use diagnostically in Western analysis and *in situ* protein detection by standard immunohistochemical procedures; for use in these applications, the ligands may be labelled in accordance with techniques known to the art. In addition, such antibody polypeptides may be used preparatively in affinity chromatography procedures, when complexed to a chromatographic support, such as a resin. All such techniques are well known to one of skill in the art.

Diagnostic uses of the closed conformation multispecific ligands include homogenous assays for analytes which exploit the ability of closed conformation multispecific ligands to bind two targets in competition, such that two targets cannot bind simultaneously (a closed conformation), or alternatively their ability to bind two targets simultaneously (an open conformation).

### Therapeutic and diagnostic compositions and uses

The invention provides compositions comprising an antagonist of Tumor Necrosis Factor 1 (TNFR1) that binds TNFR1 and inhibits signal transduction through TNFR1; (i) wherein said antagonist does not inhibit binding of TNFα to TNFR1; (ii) wherein said antagonist is a domain antibody (dAb); and (iii) wherein said dAb comprises an amino acid sequence that is at least 90% homologous to the amino acid sequence of TAR2h-205 (SEQ ID NO: 627 in Figure 27I), and a pharmaceutically acceptable carrier, diluent or excipient, and therapeutic and diagnostic methods that employ the ligands or compositions of the invention. Antagonists and ligands (e.g., dual-specific ligands, multispecific ligands, dAb monomers) according to the method of the present invention may be employed in *in vivo* therapeutic and prophylactic applications, *in vivo* diagnostic applications and the like.

Therapeutic and prophylactic uses of antagonists and ligands (e.g., multispecific ligands, dual-specific ligands, dAb monomers) of the invention involve the administration of antagonists and/or ligands according to the invention to a recipient mammal, such as a human. Dual-specific and Multi-specific ligands (e.g., dual-specific antibody formats) to bind to multimeric antigen with great avidity. Dual- or Multispecific ligands can allow the cross-linking of two antigens, for example in recruiting cytotoxic T-cells to mediate the killing of tumour cell lines.

Substantially pure ligands or binding proteins thereof, for example dAb monomers, of at least 90 to 95% homogeneity are preferred for administration to a mammal, and 98 to 99% or more homogeneity is most preferred for pharmaceutical uses, especially when the mammal is a human. Once purified, partially or to homogeneity as desired, the ligands may be used diagnostically or therapeutically (including extracorporeally) or in developing and performing assay procedures, immunofluorescent stainings and the like (Lefkovite and Pemis, (1979 and 1981) Immunological Methods, Volumes I and II, Academic Press, NY).

For example, the ligands or binding proteins thereof, for example dAb monomers, of the present invention will typically find use in preventing, suppressing or treating inflammatory states including acute and chronic inflammatory diseases. For example, the antagonists and/or ligands can be administered to treat, suppress or prevent a chronic inflammatory disease, allergic hypersensitivity, cancer, bacterial or viral infection, autoimmune disorders (which include, but are not limited to, Type I diabetes, asthma, multiple sclerosis, rheumatoid arthritis, juvenile rheumatoid arthritis, psoriatic arthritis, spondylarthropathy (e.g., ankylosing spondylitis), systemic lupus erythematosus, inflammatory bowel disease (e.g., Crohn's disease, ulcerative colitis), myasthenia gravis and Behcet's syndrome), psoriasis, endometriosis, and abdominal adhesions (e.g., post abdominal surgery).

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of the antagonists of TNFR1 of the invention (e.g, ligands, antibodies or binding proteins thereof) in protecting against or treating the disease are available. Methods for the testing of systemic lupus erythematosus (SLE) in susceptible mice are known in the art (Knight et al. (1978) J. Exp. Med., 147: 1653; Reinersten et al. (1978) New Eng. J. Med., 299: 515). Myasthenia Gravis (MG) is tested in SJL/J female mice by inducing the disease with soluble AchR protein from another species (Lindstrom et al. (1988) Adv. Immunol., 42: 233). Arthritis is induced in a susceptible strain of mice by injection of Type II collagen (Stuart et al. (1984) Ann. Rev. Immunol., 42: 233). A model by which adjuvant arthritis is induced in susceptible rats by injection of mycobacterial heat shock protein has been described (Van Eden et al. (1988) Nature, 331: 171). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAE in mouse and rat serves as a model for MS in human. In this model, the demyelinating disease is induced by administration of myelin basic protein (see Paterson (1986) Textbook of Immunopathology, Mischer et al., eds., Grune and Stratton, New York, pp. 179-213; McFarlin et al. (1973) Science, 179: 478: and Satoh et al. (1987) J. Immunol., 138: 179).

Generally, the present antagonists of the invention (e.g., ligands) will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition). A variety of suitable formulations can be used, including extended release formulations.

The antagonists (e.g., ligands) of the present invention may be used as separately administered compositions or in conjunction with other agents. These can include various immunotherapeutic drugs, such as cylcosporine, methotrexate, adriamycin or cisplatinum, and immunotoxins. Pharmaceutical compositions can include "cocktails" of various cytotoxic or other agents in conjunction with the antagonists (e.g., ligands) of the present invention, or even combinations of ligands according to the present invention having different specificities, such as ligands selected using different target antigens or epitopes, whether or not they are pooled prior to administration.

The route of administration of pharmaceutical compositions according to the invention may be any of those commonly known to those of ordinary skill in the art. For therapy, including without limitation immunotherapy, the selected ligands thereof of the invention can be administered to any patient in accordance with standard techniques. The administration can be by any appropriate mode, including parenterally, intravenously, intramuscularly, intraperitoneally, transdermally, *via* the pulmonary route, or also, appropriately, by direct infusion with a catheter. The dosage and frequency of administration will depend on the age, sex and condition of the patient, concurrent administration of other drugs, counterindications and other parameters to be taken into account by the clinician. Administration can be local (e.g., local delivery to the lung by pulmonary administration, e.g., intranasal administration) or systemic as indicated.

The ligands of this invention can be lyophilised for storage and reconstituted in a suitable carrier prior to use. This technique has been shown to be effective with conventional immunoglobulins and art-known lyophilisation and reconstitution techniques can be employed. It will be appreciated by those skilled in the art that lyophilisation and reconstitution can lead to varying degrees of antibody activity loss (e.g. with conventional immunoglobulins, IgM antibodies tend to have greater activity loss than IgG antibodies) and that use levels may have to be adjusted upward to compensate.

The compositions containing the present antagonists of the invention (e.g., ligands) or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of ligand, e.g. antibody, receptor (e.g. a T-cell receptor) or binding protein thereof *per* kilogram of body weight, with doses of 0.05 to 2.0 mg/kg/dose being more commonly used. For prophylactic applications, compositions containing the present ligands or cocktails thereof may also be administered in similar or slightly lower dosages, to prevent, inhibit or delay onset of disease (e.g., to sustain remission or quiescence, or to prevent acute phase). The skilled clinician will be able to determine the appropriate dosing interval to treat, suppress or prevent disease. When an antagonist of TNFR1 (e.g., ligand) is administered to treat, suppress or prevent a chronic inflammatory disease, it can be administered up to four times per day, twice weekly, once weekly, once every two weeks, once a month, or once every two months, at a dose off, for example, about 10 µg/kg to about 80 mg/kg, about 100 µg/kg to about 80 mg/kg, about 1 mg/kg to about 80 mg/kg, about 1 mg/kg to about 70 mg/kg, about 1 mg/kg to about 60 mg/kg, about 1 mg/kg to about 50 mg/kg, about 1 mg/kg to about 40 mg/kg, about 1 mg/kg to about 30 mg/kg, about 1 mg/kg to about 20 mg/kg , about 1 mg/kg to about 10 mg/kg, about 10 µg/kg to about 10 mg/kg, about 10 µg/kg to about 5 mg/kg, about 10 µg/kg to about 2.5 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg or about 10 mg/kg. In particular embodiments, the antagonist or TNFR1 (e.g., ligand) is administered to treat, suppress or prevent a chronic inflammatory disease once every two weeks or once a month at a dose of about 10 µg/kg to about 10 mg/kg (e.g., about 10 µg/kg, about 100 µg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg or about 10 mg/kg.)

Treatment or therapy performed using the compositions described herein is considered "effective" if one or more symptoms are reduced (e.g., by at least 10% or at least one point on a clinical assessment scale), relative to such symptoms present before treatment, or relative to such symptoms in an individual (human or model animal) not treated with such composition or other suitable control. Symptoms will obviously vary depending upon the disease or disorder targeted, but can be measured by an ordinarily skilled clinician or technician. Such symptoms can be measured, for example, by monitoring the level of one or more biochemical indicators of the disease or disorder (e.g., levels of an enzyme or metabolite correlated with the disease, affected cell numbers, etc.), by monitoring physical manifestations (e.g., inflammation, tumor size, etc.), or by an accepted clinical assessment scale, for example, the Expanded Disability Status Scale (for multiple sclerosis), the Irvine Inflammatory Bowel Disease Questionnaire (32 point assessment evaluates quality of life with respect to bowel function, systemic symptoms, social function and emotional status - score ranges from 32 to 224, with higher scores indicating a better quality of life), the Quality of Life Rheumatoid Arthritis Scale, or other accepted clinical assessment scale as known in the field. A sustained (e.g., one day or more, preferably longer) reduction in disease or disorder symptoms by at least 10% or by one or more points on a given clinical scale is indicative of "effective" treatment. Similarly, prophylaxis performed using a composition as described herein is "effective" if the onset or severity of one or more symptoms is delayed, reduced or abolished relative to such symptoms in a similar individual (human or animal model) not treated with the composition.

A composition containing an antagonist (e.g., ligand) or cocktail thereof according to the present invention may be utilised in prophylactic and therapeutic settings to aid in the alteration, inactivation, killing or removal of a select target cell population in a mammal. In addition, the selected repertoires of polypeptides described herein may be used extracorporeally or *in vitro* selectively to kill, deplete or otherwise effectively remove a target cell population from a heterogeneous collection of cells. Blood from a mammal may be combined extracorporeally with the ligands, e.g. antibodies, cell-surface receptors or binding proteins thereof whereby the undesired cells are killed or otherwise removed from the blood for return to the mammal in accordance with standard techniques.

A composition containing an antagonist (e.g., ligand) according to the present invention may be utilised in prophylactic and therapeutic settings to aid in the alteration, inactivation, killing or removal of a select target cell population in a mammal.

The antagonists of TNFR1 (e.g., ligands, dAb monomers) can be administered and or formulated together with one or more additional therapeutic or active agents. When an antagonist of TNFR1 (e.g" ligand, dAb monomer) is administered with an additional therapeutic agent , the antagonist of TNFR1 can be administered before, simultaneously with or subsequent to administration of the additional agent. Generally, the antagonist of TNFR1 (e.g., ligand, dAb monomer) and additional agent are administered in a manner that provides an overlap of therapeutic effect.

In one embodiment, the invention provides the use of a ligand of the invention for the manufacture of a medicament for treating, suppressing or preventing a chronic inflammatory disease.

In one embodiment, the invention provides the use of a ligand of the invention for the manufacture of a medicament for treating, suppressing or preventing arthritis (e.g., rheumatoid arthritis, juvenile rheumatoid arthritis, ankylosing spondylitis, psoriatic arthritis).

In another embodiment, the invention provides the use of a ligand of the invention for the manufacture of a medicament for treating, suppressing or preventing psoriasis.

In another embodiment, the invention provides the use of a ligand of the invention for the manufacture of a medicament for treating, suppressing or preventing inflammatory bowel disease (e.g., Crohn's disease, ulcerative colitis).

In another embodiment, the invention provides the use of a ligand of the invention for the manufacture of a medicament for treating, suppressing or preventing chronic obstructive pulmonary disease (e.g., chronic bronchitis, chronic obstructive bronchitis, emphysema).

In another embodiment, the invention is the use of a ligand of the invention for the manufacture of a medicament for treating, suppressing or preventing pneumonia (e.g., bacterial pneumonia, such as Staphylococcal pneumonia).

The invention provides the use of a ligand of the invention for the manufacture of a medicament for treating, suppressing or preventing other pulmonary diseases in addition to chronic obstructive pulmonary disease, and pneumonia. Other pulmonary diseases that can be treated, suppressed or prevented in accordance with the invention include, for example, cystic fibrosis and asthma (e.g., steroid resistant asthma).

Thus, in another embodiment, the inventionprovides the use of a ligand of the invention for the manufacture of a medicament for treating, suppressing or preventing a pulmonary disease (e.g., cystic fibrosis, asthma).

In particular embodiments, an antagonist of the invention is administered via pulmonary delivery, such as by inhalation (*e.g*., intrabronchial, intranasal or oral inhalation, intranasal drops) or by systemic delivery (e.g., parenteral, intravenous, intramuscular, intraperitoneal, subcutaneous).

In another embodiment, the invention provides the use of a ligand of the invention for the manufacture of a medicament for treating, suppressing or preventing septic shock comprising administering to a mammal in need thereof a therapeutically-effective dose or amount of an antagonist of TNFR1 (e.g., a ligand that comprises a dAb monomer that binds TNFR1).

In a preferred embodiment of the invention the disease is cancer or an inflammatory disease, eg rheumatoid arthritis, asthma or Crohn's disease.

### EXAMPLES

The invention is further described, for the purposes of illustration only, in the following examples. As used herein, for the purposes of dAb nomenclature, human TNFα is referred to as TAR1 and human TNFα receptor 1 (p55 receptor) is referred to as TAR2.

The Examples relating to TAR1 are included soley for the purpose of illustrating and supporting methods of dAb identification and preparation.

### Example 1. Selection of a dual specific scFv antibody (K8) directed against human serum albumin (HSA) and β-galactosidase (β -gal).

This example explains a method for making a dual specific antibody directed against β-gal and HSA in which a repertoire of V_{κ} variable domains linked to a germline (dummy) V_{H} domain is selected for binding to β-gal and a repertoire of V_{H} variable domains linked to a germline (dummy) V_{κ} domain is selected for binding to HSA. The selected variable V_{H} HSA and V_{κ} β-gal domains are then combined and the antibodies selected for binding to β-gal and HSA. HSA is a half-life increasing protein found in human blood.

Four human phage antibody libraries were used in this experiment.

| | | |
|---|---|---|
| Library 1 | Germline V_{κ}/DVT V_{H} | 8.46 x 10⁷ |
| Library 2 | Germline V_{κ}/NNK V_{H} | 9.64 x 10⁷ |
| Library 3 | Germline V_{H}/DVT V_{κ} | 1.47 x 10⁸ |
| Library 4 | Germline V_{H}/NNK V_{κ} | 1.45 x 10⁸ |

All libraries are based on a single human framework for V_{H} (V3-23/DP47 and J_{H}4b) and V_{κ} (O12/02/DPK9 and J_{κ}l) with side chain diversity incorporated in complementarity determining regions (CDR2 and CDR3).

Library 1 and Library 2 contain a dummy V_{κ} sequence, whereas the sequence of V_{H} is diversified at positions H50, H52, H52a, H53, H55, H56, H58, H95, H96, H97 and H98 (DVT or NNK encoded, respectively) (Figure 1). Library 3 and Library 4 contain a dummy V_{H} sequence, whereas the sequence of V_{κ} is diversified at positions L50, L53, L91, L92, L93, L94 and L96 (DVT or NNK encoded, respectively) (Figure 1). The libraries are in phagemid pIT2/ScFv format (Figure 2) and have been preselected for binding to generic ligands, Protein A and Protein L, so that the majority of clones in the unselected libraries are functional. The sizes of the libraries shown above correspond to the sizes after preselection. Library 1 and Library 2 were mixed prior to selections on antigen to yield a single V_{H}/dummy V_{κ} library and Library 3 and Library 4 were mixed to form a single V_{κ}/dummy V_{H} library.

Three rounds of selections were performed on β-gal using V_{κ}/dummy V_{H} library and three rounds of selections were performed on HSA using V_{H}/dummy V_{κ} library.

In the case of β-gal the phage titres went up from 1.1 x 10⁶ in the first round to 2.0 x 10⁸ in the third round. In the case of HSA the phage titres went up from 2 x 10⁴ in the first round to 1.4 x 10⁹ in the third round. The selections were performed as described by Griffith *et al.,* (1993), except that KM13 helper phage (which contains a pIII protein with a protease cleavage site between the D2 and D3 domains) was used and phage were eluted with 1 mg/ml trypsin in PBS. The addition of trypsin cleaves the pIII proteins derived from the helper phage (but not those from the phagemid) and elutes bound scFv-phage fusions by cleavage in the c-myc tag (Figure 2), thereby providing a further enrichment for phages expressing functional scFvs and a corresponding reduction in background (Kristensen & Winter, Folding & Design 3: 321-328, Jul 9, 1998). Selections were performed using immunotubes coated with either HSA or β-gal at 100µg/ml concentration.

To check for binding, 24 colonies from the third round of each selection were screened by monoclonal phage ELISA. Phage particles were produced as described by Harrison et al., Methods Enzymol. 1996;267:83-109. 96-will ELISA plates were coated with 100µl of HSA or β-gal at 10µg/ml concentration in PBS overnight at 4°C. A standard ELISA protocol was followed (Hoogenboom *et al.,* 1991) using detection of bound phage with anti-M13-HRP conjugate. A selection of clones gave ELISA signals of greater than 1.0 with 50µl supernatant.

Next, DNA preps were made from V_{H}/dummy V_{κ} library selected on HSA and from V_{κ}/dummy V_{H} library selected on β-gal using the QIAprep Spin Miniprep kit (Qiagen). To access most of the diversity, DNA preps were made from each of the three rounds of selections and then pulled together for each of the antigens. DNA preps were then digested with Sal*I*/Not*I* overnight at 37°C. Following gel purification of the fragments, V_{κ} chains from the V_{κ}/dummy V_{H} library selected on β-gal were ligated in place of a dummy V_{κ} chain of the V_{H}/dummy V_{κ} library selected on HSA creating a library of 3.3 x 10⁹ clones.

This library was then either selected on HSA (first round) and β-gal (second round), HSA/β-gal selection, or on β-gal (first round) and HSA (second round), β-gal/HSA selection. Selections were performed as described above. In each case after the second round 48 clones were tested for binding to HSA and β-gal by the monoclonal phage ELISA (as described above) and by ELISA of the soluble scFv fragments. Soluble antibody fragments were produced as described by Harrison *et al.,* (1996), and standard ELISA protocol was followed Hoogenboom et al. (1991) Nucleic Acids Res., 19: 4133, except that 2% Tween/PBS was used as a blocking buffer and bound scFvs were detected with Protein L-HRP. Three clones (E4, E5 and E8) from the HSA/β-gal selection and two clones (K8 and K10) from the β-gal/HSA selection were able to bind both antigens. scFvs from these clones were PCR amplified and sequenced as described by Ignatovich et al., (1999) J Mol Biol 1999 Nov 26;294(2):457-65, using the primers LMB3 and pHENseq. Sequence analysis revealed that all clones were identical. Therefore, only one clone encoding a dual specific antibody (K8) was chosen for further work (Figure 3).

### Example 2. Characterisation of the binding properties of the K8 antibody.

Firstly, the binding properties of the K8 antibody were characterised by the monoclonal phage ELISA. A 96-well plate was coated with 100µl of HSA and β-gal alongside with alkaline phosphatase (APS), bovine serum albumin (BSA), peanut agglutinin, lysozyme and cytochrome c (to check for cross-reactivity) at 10µg/ml concentration in PBS overnight at 4°C. The phagemid from K8 clone was rescued with KM13 as described by Harrison *et al.*, (1996) and the supernatant (50µl) containing phage assayed directly. A standard ELISA protocol was followed (Hoogenboom *et al.*, 1991) using detection of bound phage with anti-M13-HRP conjugate. The dual specific K8 antibody was found to bind to HSA and β-gal when displayed on the surface of the phage with absorbance signals greater than 1.0 (Figure 4). Strong binding to BSA was also observed (Figure 4). Since HSA and BSA are 76% homologous on the amino acid level, it is not surprising that K8 antibody recognised both of these structurally related proteins. No cross-reactivity with other proteins was detected (Figure 4).

Secondly, the binding properties of the K8 antibody were tested in a soluble scFv ELISA. Production of the soluble scFv fragment was induced by IPTG as described by Harrison *et al.*, (1996). To determine the expression levels of K8 scFv, the soluble antibody fragments were purified from the supernatant of 50ml inductions using Protein A-Sepharose columns as described by Harlow and Lane, Antibodies: a Laboratory Manual, (1988) Cold Spring Harbor. OD₂₈₀ was then measured and the protein concentration calculated as described by Sambrook *et al.*, (1989). K8 scFv was produced in supernatant at 19mg/1.

A soluble scFv ELISA was then performed using known concentrations of the K8 antibody fragment. A 96-well plate was coated with 100µl of HSA, BSA and β-gal at 10µg/ml and 100µl of Protein A at 1µg/ml concentration. 50µl of the serial dilutions of the K8 scFv was applied and the bound antibody fragments were detected with Protein L-HRP. ELISA results confirmed the dual specific nature of the K8 antibody (Figure 5).

To confirm that binding to β-gal is determined by the V_{κ} domain and binding to HSA/BSA by the V_{H} domain of the K8 scFv antibody, the V_{κ} domain was cut out from K8 scFv DNA by Sal*I*/Not*I* digestion and ligated into a Sal*I*/Not*I* digested pIT2 vector containing dummy V_{H} chain (Figures 1 and 2). Binding characteristics of the resulting clone K8V_{κ}/dummy V_{H} were analysed by soluble scFv ELISA. Production of the soluble scFv fragments was induced by IPTG as described by Harrison *et al.*, (1996) and the supernatant (50µ) containing scFvs assayed directly. Soluble scFv ELISA was performed as described in Example 1 and the bound scFvs were detected with Protein L-HRP. The ELISA results revealed that this clone was still able to bind β-gal, whereas binding to BSA was abolished (Figure 6).

### Example 3. Selection of single V_{H} domain antibodies antigens A and B and single V_{κ} domain antibodies directed against antigens C and D.

This example describes a method for making single V_{H} domain antibodies directed against antigens A and B and single V_{κ} domain antibodies directed against antigens C and D by selecting repertoires of virgin single antibody variable domains for binding to these antigens in the absence of the complementary variable domains.

Selections and characterisation of the binding clones is performed as described previously (see Example 5, PCT/GB 02/003014). Four clones are chosen for further work:
VH1 - Anti A V_{H}
VH2 - Anti B V_{H}
VK1 - Anti C V_{κ}
VK2 - Anti D V_{κ}

The procedures described above in Examples 1-3 may be used, in a similar manner as that described, to produce dimer molecules comprising combinations of V_{H} domains (i.e., V_{H}-V_{H} ligands) and combinations of V_{L} domains (V_{L}-V_{L} ligands).

Example 4. Creation and characterisation of the dual specific ScFv antibodies (VH1/VH2 directed against antigens A and B and VK1/VK2 directed against antigens C and D).

This example demonstrates that dual specific ScFv antibodies (VH1/VH2 directed against antigens A and B and VK1/VK2 directed against antigens C and D) could be created by combining V_{κ} and V_{H} single domains selected against respective antigens in a ScFv vector.

To create dual specific antibody VH1/VH2, VH1 single domain is excised from variable domain vector 1 (Figure 7) by *Nco*I/*Xho*I digestion and ligated into *Nco*I/*Xho*I digested variable domain vector 2 (Figure 7) to create VH1/ variable domain vector 2. VH2 single domain is PCR amplified from variable domain vector 1 using primers to introduce *Sal*I restriction site to the 5' end and *Not*I restriction site to the 3' end. The PCR product is then digested with *Sal*I/*Not*I and ligated into *Sal*I/*Not*I digested VH1/ variable domain vector 2 to create VH1/VH2/ variable domain vector 2.

VK1/VK2/ variable domain vector 2 is created in a similar way. The dual specific nature of the produced VH1/VH2 ScFv and VK1/VK2 ScFv is tested in a soluble ScFv ELISA as described previously (see Example 6, PCT/GB 02/003014). Competition ELISA is performed as described previously (see Example 8, PCT/GB 02/003014).

Possible outcomes:
- VH1/VH2 ScFv is able to bind antigens A and B simultaneously
- VK1/VK2 ScFv is able to bind antigens C and D simultaneously
- VH1/VH2 ScFv binding is competitive (when bound to antigen A, VH1/VH2 ScFv cannot bind to antigen B)
- VK1/VK2 ScFv binding is competitive (when bound to antigen C, VK1/VK2 ScFv cannot bind to antigen D)

### Example 5. Construction of dual specific VH1/VH2 Fab and VK1/VK2 Fab and analysis of their binding properties.

To create VH1/VH2 Fab, VH1 single domain is ligated into *Nco*I/*Xho*I digested CH vector (Figure 8) to create VH1/CH and VH2 single domain is ligated into *Sal*I/*NotI* digested CK vector (Figure 9) to create VH2/CK. Plasmid DNA from VH1/CH and VH2/CK is used to co-transform competent *E. coli* cells as described previously (see Example 8, PCT/GB02/003014).

The clone containing VH1/CH and VH2/CK plasmids is then induced by IPTG to produce soluble VH1/VH2 Fab as described previously (see Example 8, PCT/GB 02/003014).

VK1/VK2 Fab is produced in a similar way.

Binding properties of the produced Fabs are tested by competition ELISA as described previously (see Example 8, PCT/GB 02/003014).

Possible outcomes:
- VH1/VH2 Fab is able to bind antigens A and B simultaneously
- VK1/VK2 Fab is able to bind antigens C and D simultaneously
- VH1/VH2 Fab binding is competitive (when bound to antigen A, VH1/VH2 Fab cannot bind to antigen B)
- VK1/VK2 Fab binding is competitive (when bound to antigen C, VK1/VK2 Fab cannot bind to antigen D)

### Example 6. Chelating dAb Dimers

### Summary

VH and VK homo-dimers are created in a dAb-linker-dAb format using flexible polypeptide linkers. Vectors were created in the dAb linker-dAb format containing glycine-serine linkers of different lengths 3U:(Gly₄Ser)₃ (SEQ ID NO:199), 5U:(Gly₄Ser)₅ (SEQ ID NO:629), 7U:(Gly₄Ser)₇ (SEQ ID NO:630). Dimer libraries were created using guiding dAbs upstream of the linker: TAR1-5 (VK), TAR1-27(VK), TAR2-5(VH) or TAR2-6(VK) and a library of corresponding second dAbs after the linker. Using this method, novel dimeric dAbs were selected. The effect of dimerisation on antigen binding was determined by ELISA and BIAcore studies and in cell neutralisation and receptor binding assays. Dimerisation of both TAR1-5 and TAR1-27 resulted in significant improvement in binding affinity and neutralisation levels.

### 1.0 Methods

### 1.1 Library generation

### 1.1.1 Vectors

pEDA3U, pEDA5U and pEDA7U vectors were designed to introduce different linker lengths compatible with the dAb-linker-dAb format. For pEDA3U, sense and anti-sense 73-base pair oligo linkers were annealed using a slow annealing program (95°C-5mins, 80°C-10mins, 70°C-15mins, 56°C-15mins, 42°C until use) in buffer containing 0.1MNaCl, 10mM Tris-HCl pH7.4 and cloned using the *Xho1* and *Not1* restriction sites. The linkers encompassed 3 (Gly₄Ser) units and a stuffer region housed between *Sal1* and *Not1* cloning sites (scheme 1). In order to reduce the possibility of monomeric dAbs being selected for by phage display, the stuffer region was designed to include 3 stop codons, a *Sac1* restriction site and a frame shift mutation to put the region out of frame when no second dAb was present. For pEDA5U and 7U due to the length of the linkers required, overlapping oligo-linkers were designed for each vector, annealed and elongated using Klenow. The fragment was then purified and digested using the appropriate enzymes before cloning using the *Xho1* and *Not1* restriction sites.

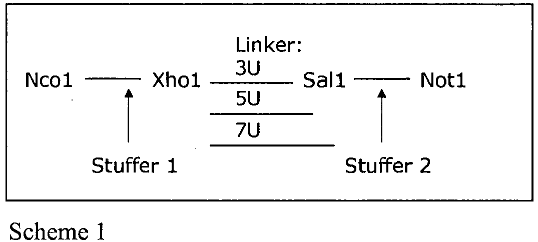

### 1.1.2 Library preparation

The N-terminal V gene corresponding to the guiding dAb was cloned upstream of the linker using *Nco1* and *Xho1* restriction sites. VH genes have existing compatible sites, however cloning VK genes required the introduction of suitable restriction sites. This was achieved by using modifying PCR primers (VK-DLIBF: 5' cggccatggcgtcaacggacat (SEQ ID NO:377); VKXho1R: 5' atgtgcgctcgagcgtttgattt 3' (SEQ ID NO:378)) in 30 cycles of PCR amplification using a 2:1 mixture of SuperTaq (HTBiotechnology Ltd)and *pfu* turbo (Stratagene). This maintained the *Nco1* site at the 5' end while destroying the adjacent *Sal1* site and introduced the *Xho1* site at the 3' end. 5 guiding dAbs were cloned into each of the 3 dimer vectors: TAR1-5 (VK), TAR1-27(VK), TAR2-5(VH), TAR2-6(VK) and TAR2-7(VK). All constructs were verified by sequence analysis.

Having cloned the guiding dAbs upstream of the linker in each of the vectors (pEDA3U, 5U and 7U): TAR1-5 (VK), TAR1-27(VK), TAR2-5(VH) or TAR2-6(VK) a library of corresponding second dAbs were cloned after the linker. To achieve this, the complimentary dAb libraries were PCR amplified from phage recovered from round 1 selections of either a VK library against Human TNFα (at approximately 1 x 10⁶ diversity after round 1) when TAR1-5 or TAR1-27 are the guiding dAbs, or a VH or VK library against human p55 TNF receptor (both at approximately 1 x 10⁵ diversity after round 1) when TAR2-5 or TAR2-6 respectively are the guiding dAbs. For VK libraries PCR amplification was conducted using primers in 30 cycles of PCR amplification using a 2:1 mixture of SuperTaq and *pfu* turbo. VH libraries were PCR amplified using primers in order to introduce a *Sal1* restriction site at the 5' end of the gene. The dAb library PCRs were digested with the appropriate restriction enzymes, ligated into the corresponding vectors down stream of the linker, using *Sal1*/*Not1* restriction sites and electroporated into freshly prepared competent TG1 cells.

The titres achieved for each library are as follows:
TAR1-5: pEDA3U = 4x10⁸, pEDA5U = 8x10⁷, pEDA7U = 1x10⁸
TAR1-27: pEDA3U = 6.2x10⁸, pEDA5U = 1x10⁸, pEDA7U = 1x10⁹
TAR2h-5: pEDA3U = 4x10⁷, pEDA5U = 2 x 10⁸, pEDA7U = 8x10⁷
TAR2h-6: pEDA3U = 7.4x10⁸, pEDA5U = 1.2 x 10⁸, pEDA7U = 2.2x10⁸

### 1.2 Selections

### 1.2.1 TNFα

Selections were conducted using human TNFα passively coated on immunotubes. Briefly, Immunotubes are coated overnight with 1-4mls of the required antigen. The immunotubes were then washed 3 times with PBS and blocked with 2%milk powder in PBS for 1-2hrs and washed a further 3 times with PBS. The phage solution is diluted in 2%milk powder in PBS and incubated at room temperature for 2hrs. The tubes are then washed with PBS and the phage eluted with 1mg/ml trypsin-PBS. Three selection strategies were investigated for the TAR1-5 dimer libraries. The first round selections were carried out in immunotubes using human TNFα coated at 1µg/ml or 20µg/ml with 20 washes in PBS 0.1%Tween. TG1 cells are infected with the eluted phage and the titres are determined (eg, Marks et al J Mol Biol. 1991 Dec 5;222(3):581-97, Richmann et al Biochemistry. 1993 Aug 31;32(34):8848-55).

The titres recovered were:
pEDA3U=2.8x10 (1µg/ml TNF) 1.5x10⁸ (20µg/mlTNF),
pEDA5U = 1.8x10⁷ (1µg/ml TNF), 1.6x10⁸ (20µg/ml TNF)
pEDA7U = 8x10⁶ (1µg/ml TNF), 7x10⁷ (20µg/ml TNF).

The second round selections were carried out using 3 different methods.
1. In immunotubes, 20 washes with overnight incubation followed by a further 10 washes.
2. In immunotubes, 20 washes followed by 1hr incubation at RT in wash buffer with (1µg/ml TNFα) and 10 further washes.
3. Selection on streptavidin beads using 33 pmoles biotinylated human TNFα (Henderikx et al., 2002, Selection of antibodies against biotinylated antigens. Antibody Phage Display : Methods and protocols, Ed. O'Brien and Atkin, Humana Press). Single clones from round 2 selections were picked into 96 well plates and crude supernatant preps were made in 2ml 96 well plate format.

**Table 1.**

| | Round 1 Human TNFαimmuno tube coating concentration | Round 2 selection method 1 | Round 2 selection method 2 | Round 2 selection method 3 |
|---|---|---|---|---|
| pEDA3U | 1µg/ml | 1 x 10⁹ | 1.8 x 10⁹ | 2.4 x 10¹⁰ |
| pEDA3U | 20µg/ml | 6 x 10⁹ | 1.8 x 10¹⁰ | 8.5 x 10¹⁰ |
| pEDA5U | 1µg/ml | 9 x 10⁸ | 1.4 x 10⁹ | 2.8 x 10¹⁰ |
| pEDA5U | 20µg/ml | 9.5 x 10⁹ | 8.5 x 10⁹ | 2.8 x 10¹⁰ |
| pEDA7U | 1µg/ml | 7.8 x 10⁸ | 1.6 x 10⁸ | 4 x 10¹⁰ |
| pEDA7U | 20µg/ml | 1 x 10¹⁰ | 8 x 10⁹ | 1.5 x 10¹⁰ |

For TAR1-27, selections were carried out as described previously with the following modifications. The first round selections were carried out in immunotubes using human TNFα coated at 1µg/ml or 20µg/ml with 20 washes in PBS 0.1 %Tween. The second round selections were carried out in immunotubes using 20 washes with overnight incubation followed by a further 20 washes. Single clones from round 2 selections were picked into 96 well plates and crude supernatant preps were made in 2ml 96 well plate format.

TAR1-27 titres are as follows:

**Table 2.**

| | Human TNFαimmunotube coating conc | Round 1 | Round 2 |
|---|---|---|---|
| pEDA3U | 1µg/ml | 4 x 10⁹ | 6 x 10⁹ |
| pEDA3U | 20µg/ml | 5 x 10⁹ | 4.4 x 10¹⁰ |
| pEDA5U | 1µg/ml | 1.5 x 10⁹ | 1.9 x 10¹⁰ |
| pEDA5U | 20µg/ml | 3.4 x 10⁹ | 3.5 x 10¹⁰ |
| pEDA7U | 1µg/ml | 2.6 x 10⁹ | 5 x 10⁹ |
| pEDA7U | 20µg/ml | 7 x 10⁹ | 1.4 x 10¹⁰ |

### 1.2.2 TNF RECEPTOR 1 (p55 RECEPTOR; TAR2)

Selections were conducted as described previously for the TAR2h-5 libraries only. 3 rounds of selections were carried out in immunotubes using either 1 µg/ml human p55 TNF receptor or 10µg/ml human p55 TNF receptor with 20 washes in PBS 0.1%Tween with overnight incubation followed by a further 20 washes. Single clones from round 2 and 3 selections were picked into 96 well plates and crude supernatant preps were made in 2ml 96 well plate format.

TAR2h-5 titres are as follows:

**Table 3.**

| | Round 1 human p55 TNF receptor immunotube coating concentration | Round 1 | Round 2 | Round 3 |
|---|---|---|---|---|
| pEDA3U | 1µg/ml | 2.4 x 10⁶ | 1.2 x 10⁷ | 1.9 x 10⁹ |
| pEDA3U | 10µg/ml | 3.1 x 10⁷ | 7 x 10⁷ | 1 x 10⁹ |
| pEDA5U | 1µg/ml | 2.5 x 10⁶ | 1.1 x 10⁷ | 5.7 x 10⁸ |
| pEDA5U | 10µg/ml | 3.7 x 10⁷ | 2.3 x 10⁸ | 2.9 x 10⁹ |
| pEDA7U | 1µg/ml | 1.3 x 10⁶ | 1.3 x 10⁷ | 1.4 x 10⁹ |
| pEDA7U | 10µg/ml | 1.6 x 10⁷ | 1.9 x 10⁷ | 3 x 10¹⁰ |

### 1.3 Screening

Single clones from round 2 or 3 selections were picked from each of the 3U, 5U and 7U libraries from the different selections methods, where appropriate. Clones were grown in 2xTY with 100µg/ml ampicillin and 1% glucose overnight at 37°C. A 1/100 dilution of this culture was inoculated into 2mls of 2xTY with 100µg/ml ampicillin and 0.1% glucose in 2ml, 96 well plate format and grown at 37°C shaking until OD600 was approximately 0.9. The culture was then induced with 1mM IPTG overnight at 30°C. The supernatants were clarified by centrifugation at 4000rpm for 15 mins in a sorval plate centrifuge. The supernatant preps the used for initial screening.

### 1.3.1 ELISA

Binding activity of dimeric recombinant proteins was compared to monomer by Protein A/L ELISA or by antigen ELISA. Briefly, a 96 well plate is coated with antigen or Protein A/L overnight at 4°C. The plate washed with 0.05% Tween-PBS, blocked for 2hrs with 2% Tween-PBS. The sample is added to the plate incubated for 1 hr at room temperature. The plate is washed and incubated with the secondary reagent for 1hr at room temperature. The plate is washed and developed with TMB substrate. Protein A/L-HRP or India-HRP was used as a secondary reagent. For antigen ELISAs, the antigen concentrations used were 1µg/ml in PBS for Human TNFα and human THF receptor 1. Due to the presence of the guiding dAb in most cases dimers gave a positive ELISA signal therefore off rate determination was examined by BIAcore.

### 1.3.2 BIAcore

BIAcore analysys was conducted for TAR1-5 and TAR2h-5 clones. For screening, Human TNFαwas coupled to a CM5 chip at high density (approximately 10000 RUs). 50 µl of Human TNFα(50 µg/ml) was coupled to the chip at 5µl/min in acetate buffer - pH5.5. Regeneration of the chip following analysis using the standard methods is not possible due to the instability of Human TNFα, therefore after each sample was analysed, the chip was washed for 10mins with buffer. For TAR1-5, clones supernatants from the round 2 selection were screened by BIAcore.

48 clones were screened from each of the 3U, 5U and 7U libraries obtained using the following selection methods:
R1: 1µg/ml human TNFα immunotube, R2 1µg/ml human TNFα immunotube, overnight wash.
R1: 20µg/ml human TNFα immunotube, R2 20µg/ml human TNFα immunotube, overnight wash.
R1: 1µg/ml human TNFα immunotube, R2 33 pmoles biotinylated human TNFα on beads.
R1: 20µg/ml human TNFα immunotube, R2 33 pmoles biotinylated human TNFα beads.

For screening, human p55 TNF receptor was coupled to a CM5 chip at high density (approximately 4000 RUs). 100 µl of human p55 TNF receptor (10 µg/ml) was coupled to the chip at 5µl/min in acetate buffer - pH5.5. Standard regeneration conditions were examined (glycine pH2 or pH3) but in each case antigen was removed from the surface of the chip therefore as with TNFα, therefore after each sample was analysed, the chip was washed for 10mins with buffer.

For TAR2-5, clones supernatants from the round 2 selection were screened. 48 clones were screened from each of the 3U, 5U and 7U libraries, using the following selection methods:
R1: 1µg/ml human p55 TNF receptor immunotube, R2 1µg/ml human p55 TNF receptor immunotube, overnight wash.
R1: 10µg/ml human p55 TNF receptor immunotube, R2 10µg/ml human p55 TNF receptor immunotube, overnight wash.

### 1.3.3 Receptor and Cell Assays

The ability of the dimers to neutralise in the receptor assay was conducted as follows:

### Receptor binding

Anti-TNF dAbs were tested for the ability to inhibit the binding of TNF to recombinant TNF receptor 1 (p55). Briefly, Maxisorp plates were incubated overnight with 30mg/ml anti-human Fc mouse monoclonal antibody (Zymed, San Francisco, USA). The wells were washed with phosphate buffered saline (PBS) containing 0.05% Tween-20 and then blocked with 1% BSA in PBS before being incubated with 100ng/ml TNF receptor 1 Fc fusion protein (R&D Systems, Minneapolis, USA). Anti-TNF dAb was mixed with TNF which was added to the washed wells at a final concentration of 10ng/ml. TNF binding was detected with 0.2mg/ml biotinylated anti-TNF antibody (HyCult biotechnology, Uben, Netherlands) followed by 1 in 500 dilution of horse radish peroxidase labelled streptavidin (Amersham Biosciences, UK) and then incubation with TMB substrate (KPL,

Gaithersburg, USA). The reaction was stopped by the addition of HCl and the absorbance was read at 450nm. Anti-TNF dAb activity lead to a decrease in TNF binding and therefore a decrease in absorbance compared with the TNF only control.

### L929 Cytotoxicity Assay

Anti-TNF dAbs were also tested for the ability to neutralise the cytotoxic activity of TNF on mouse L929 fibroblasts (Evans, T. (2000) Molecular Biotechnology 15, 243-248). Briefly, L929 cells plated in microtitre plates were incubated overnight with anti-TNF dAb, 100pg/ml TNF and 1mg/ml actinomycin D (Sigma, Poole, UK). Cell viability was measured by reading absorbance at 490nm following an incubation with [3-(4,5-dimethylthiazol-2-yl)-5-(3-carbboxymethoxyphenyl)-2-(4-sulfophenyl)-2H-tetrazolium (Promega, Madison, USA). Anti-TNF dAb activity lead to a decrease in TNF cytotoxicity and therefore an increase in absorbance compared with the TNF only control.

In the initial screen, supernatants prepared for BIAcore analysis, described above, were also used in the receptor assay. Further analysis of selected dimers was also conducted in the receptor and cell assays using purified proteins.

### HeLα IL-8 assay

Anti-TNFR1 or anti-TNF alpha dAbs were tested for the ability to neutralise the induction of IL-8 secretion by TNF in HeLa cells (method adapted from that of Akeson, L. et al (1996) Journal of Biological Chemistry 271, 30517-30523, describing the induction of IL-8 by IL-1 in HUVEC; here we look at induction by human TNF alpha and we use HeLa cells instead of the HUVEC cell line). Briefly, HeLa cells plated in microtitre plates were incubated overnight with dAb and 300pg/ml TNF. Post incubation the supernatant was aspirated off the cells and IL-8 concentration measured via a sandwich ELISA (R&D Systems). Anti-TNFR1 dAb activity lead to a decrease in IL-8 secretion into the supernatant compared with the TNF only control.

The L929 assay is used throughout the following experiments; however, the use of the HeLa IL-8 assay is preferred to measure anti-TNF receptor 1 (p55) ligands; the presence of mouse p55 in the L929 assay poses certain limitations in its use.

### 1.4 Sequence analysis

Dimers that proved to have interesting properties in the BIAcore and the receptor assay screens were sequenced. Sequences are detailed in the sequence listing.

### 1.5 Formatting

### 1.5.1 TAR1-5-19 dimers

The TAR1-5 dimers that were shown to have good neutralisation properties were re-formatted and analysed in the cell and receptor assays. The TAR1-5 guiding dab was substituted with the affinity matured clone TAR1-5-19. To achieve this TAR1-5 was cloned out of the individual dimer pair and substituted with TAR1-5-19 that had been amplified by PCR. In addition, TAR1-5-19 homodimers were also constructed in the 3U, 5U and 7U vectors. The N terminal copy of the gene was amplified by PCR and cloned as described above and the C-terminal gene fragment was cloned using existing *Sal1* and *Not1* restriction sites.

### 1.5.2 Mutagenesis

The amber stop codon present in dAb2, one of the C-terminal dAbs in the TAR1-5 dimer pairs was mutated to a glutamine by site-directed mutagenesis.

### 1.5.3 Fabs

The dimers containing TAR1-5 or TAR1-5-19 were re-formatted into Fab expression vectors. dAbs were cloned into expression vectors containing either the CK or CH genes using *Sfi1* and *Not1* restriction sites and verified by sequence analysis. The CK vector is derived from a pUC based ampicillin resistant vector and the CH vector is derived from a pACYC chloramphenicol resistant vector. For Fab expression the dAb-CH and dAb-CK constructs were co-transformed into HB2151 cells and grown in 2xTY containing 0.1% glucose, 100µg/ml ampicillin and 10µg/ml chloramphenicol.

### 1.5.3 Hinge dimerisation

Dimerisation of dAbs via cystine bond formation was examined. A short sequence of amino acids EPKSGDKTHTCPPCP (SEQ ID NO:379) a modified form of the human IgGC1 hinge was engineered at the C terminal region on the dAb. An oligo linker encoding for this sequence was synthesised and annealed, as described previously. The linker was cloned into the pEDA vector containing TAR1-5-19 using *Xho1* and *Not1* restriction sites. Dimerisation occurs *in situ* in the periplasm.

### 1.6 Expression and purification

### 1.6.1 Expression

Supernatants were prepared in the 2ml, 96-well plate format for the initial screening as described previously. Following the initial screening process selected dimers were analysed further. Dimer constructs were expressed in TOP10F' or HB2151 cells as supernatants. Briefly, an individual colony from a freshly streaked plate was grown overnight at 37°C in 2xTY with 100µg/ml ampicillin and 1% glucose. A 1/100 dilution of this culture was inoculated into 2xTY with 100µg/ml ampicillin and 0.1% glucose and grown at 37°C shaking until OD600 was approximately 0.9. The culture was then induced with 1mM IPTG overnight at 30°C. The cells were removed by centrifugation and the supernatant purified with protein A or L agarose.

Fab and cysteine hinge dimers were expressed as periplasmic proteins in HB2152 cells. A 1/100 dilution of an overnight culture was inoculated into 2xTY with 0.1% glucose and the appropriate antibiotics and grown at 30°C shaking until OD600 was approximately 0.9. The culture was then induced with 1mM IPTG for 3-4 hours at 25°C. The cells were harvested by centrifugation and the pellet resuspended in periplasmic preparation buffer (30mM Tris-HCl pH8.0, 1mM EDTA, 20% sucrose). Following centrifugation the supernatant was retained and the pellet resuspended in 5mM MgSO₄, The supernatant was harvested again by centrifugation, pooled and purified.

### 1.6.2 Protein A/L purification

Optimisation of the purification of dimer proteins from Protein L agarose (Affitech, Norway) or Protein A agarose (Sigma, UK) was examined. Protein was eluted by batch or by column elution using a peristaltic pump. Three buffers were examined 0.1M Phosphate-citrate buffer pH2.6, 0.2M Glycine pH2.5 and 0.1M Glycine pH2.5. The optimal condition was determined to be under peristaltic pump conditions using 0.1M Glycine pH2.5 over 10 column volumes. Purification from protein A was conducted peristaltic pump conditions using 0.1M Glycine pH2.5.

### 1.6.3 FPLC purification

Further purification was carried out by FPLC analysis on the AKTA Explorer 100 system (Amersham Biosciences Ltd). TAR1-5 and TAR1-5-19 dimers were fractionated by cation exchange chromatography (1ml Resource S - Amersham Biosciences Ltd) eluted with a 0-1M NaCl gradient in 50mM acetate buffer pH4. Hinge dimers were purified by ion exchange (1ml Resource Q Amersham Biosciences Ltd) eluted with a 0-1M NaCl gradient in 25mMTris HCl pH 8.0. Fabs were purified by size exclusion chromatography using a superose 12 (Amersham Biosciences Ltd) column run at a flow rate of 0.5ml/min in PBS with 0.05% tween. Following purification samples were concentrated using vivaspin 5K cut off concentrators (Vivascience Ltd).

### 2.0 Results

### 2.1 TAR1-5 dimers

6 x 96 clones were picked from the round 2 selection encompassing all the libraries and selection conditions. Supernatant preps were made and assayed by antigen and Protein L ELISA, BIAcore and in the receptor assays. In ELISAs, positive binding clones were identified from each selection method and were distributed between 3U, 5U and 7U libraries. However, as the guiding dAb is always present it was not possible to discriminate between high and low affinity binders by this method therefore BIAcore analysis was conducted.

BIAcore analysis was conducted using the 2ml supernatants. BIAcore analysis revealed that the dimer Koff rates were vastly improved compared to monomeric TAR1-5. Monomer Koff rate was in the range of 10⁻¹M compared with dimer Koff rates which were in the range of 10⁻³ - 10⁻⁴M. 16 clones that appeared to have very slow off rates were selected, these came from the 3U, 5U and 7U libraries and were sequenced. In addition the supernatants were analysed for the ability to neutralise human TNFα in the receptor assay.

6 lead clones (d1-d6 below) that neutralised in these assays and have been sequenced. The results shows that out of the 6 clones obtained there are only 3 different second dAbs (dAb1, dAb2 and dAb3) however where the second dAb is found more than once they are linked with different length linkers.
TAR1-5d1: 3U linker 2^{nd} dAb=dAb1 - 1µg/ml Ag immunotube overnight wash
TAR1-5d2: 3U linker 2^{nd} dAb=dAb2 - 1µg/ml Ag immunotube overnight wash
TAR1-5d3: 5U linker 2^{nd} dAb=dAb2 - 1µg/ml Ag immunotube overnight wash
TAR1-5d4: 5U linker 2^{nd} dAb=dAb3 - 20µg/ml Ag immunotube overnight wash
TAR1-5d5: 5U linker 2^{nd} dAb=dAb1 - 20µg/ml Ag immunotube overnight wash
TAR1-5d6: 7U linker 2^{nd} dAb=dAb1- R1:1µg/ml Ag immunotube overnight wash, R2:beads

The 6 lead clones were examined further. Protein was produced from the periplasm and supernatant, purified with protein L agarose and examined in the cell and receptor assays. The levels of neutralisation were variable (Table 1). The optimal conditions for protein preparation were determined. Protein produced from HB2151 cells as supernatants gave the highest yield (approximately 10mgs/L of culture). The supernatants were incubated with protein L agarose for 2hrs at room temperature or overnight at 4°C. The beads were washed with PBS/NaCl and packed onto an FPLC column using a peristaltic pump. The beads were washed with 10 column volumes of PBS/NaCl and eluted with 0.1M glycine pH2.5. In general, dimeric protein is eluted after the monomer.

TAR1-5d1-6 dimers were purified by FPLC. Three species were obtained, by FPLC purification and were identified by SDS PAGE. One species corresponds to monomer and the other two species corresponds to dimers of different sizes. The larger of the two species is possibly due to the presence of C terminal tags. These proteins were examined in the receptor assay. The data presented in table 1 represents the optimum results obtained from the two dimeric species (Figure 11)

The three second dAbs from the dimer pairs (ie, dAb1, dAb2 and dAb3) were cloned as monomers and examined by ELISA and in the cell and receptor assay. All three dAbs bind specifically to TNF by antigen ELISA and do not cross react with plastic or BSA. As monomers, none of the dAbs neutralise in the cell or receptor assays.

### 2.1.2 TAR1-5-19 dimers

TAR1-5-19 was substituted for TAR1-5 in the 6 lead clones. Analysis of all TAR1-5-19 dimers in the cell and receptor assays was conducted using total protein (protein L purified only) unless otherwise stated (Table 2). TAR1-5-19d4 and TAR1-5-19d3 have the best ND₅₀ (~5nM) in the cell assay, this is consistent with the receptor assay results and is an improvement over TAR1-5-19 monomer (ND₅₀~30nM). Although purified TAR1-5 dimers give variable results in the receptor and cell assays TAR1-5-19 dimers were more consistent. Variability was shown when using different elution buffers during the protein purification. Elution using 0.1M Phosphate-citrate buffer pH2.6 or 0.2M Glycine pH2.5 although removing all protein from the protein L agarose in most cases rendered it less functional.

TAR1-5-19d4 was expressed in the fermenter and purified on cation exchange FPLC to yield a completely pure dimer. As with TAR1-5d4 three species were obtained, by FPLC purification corresponding to monomer and two dimer species. This dimer was amino acid sequenced. TAR1-5-19 monomer and TAR1-5-19d4 were then examined in the receptor assay and the resulting IC50 for monomer was 30nM and for dimer was 8nM. The results of the receptor assay comparing TAR1-5-19 monomer, TAR1-5-19d4 and TAR1-5d4 is shown in figure 10.

TAR1-5-19 homodimers were made in the 3U, 5U and 7U vectors, expressed and purified on Protein L. The proteins were examined in the cell and receptor assays and the resulting IC₅₀s (for receptor assay) and ND₅₀s (for cell assay) were determined (table 3, figure 12).

### 2.2 Fabs

TAR1-5 and TAR1-5-19 dimers were also cloned into Fab format, expressed and purified on protein L agarose. Fabs were assessed in the receptor assays (Table 4). The results showed that for both TAR1-5-19 and TAR1-5 dimers the neutralisation levels were similar to the original Gly₄Ser linker dimers from which they were derived. A TAR1-5-19 Fab where TAR1-5-19 was displayed on both CH and CK was expressed, protein L purified and assessed in the receptor assay. The resulting IC50 was approximately 1nM.

### 2.3 TAR1-27 dimers

3 x 96 clones were picked from the round 2 selection encompassing all the libraries and selection conditions. 2ml supernatant preps were made for analysis in ELISA and bioassays. Antigen ELISA gave 71 positive clones. The receptor assay of crude supernatants yielded 42 clones with inhibitory properties (TNF binding 0-60%). In the majority of cases inhibitory properties correlated with a strong ELISA signal. 42 clones were sequenced, 39 of these have unique second dAb sequences. The 12 dimers that gave the best inhibitory properties were analysed further.

The 12 neutralising clones were expressed as 200ml supernatant preps and purified on protein L. These were assessed by protein L and antigen ELISA, BIAcore and in the receptor assay. Strong positive ELISA signals were obtained in all cases. BIAcore analysis revealed all clones to have fast on and off rates. The off rates were improved compared to monomeric TAR1-27, however the off rate of TAR1-27 dimers was faster (Koff is approximately in the range of 10⁻¹ and 10⁻²M) than the TAR1-5 dimers examined previously (Koff is approximately in the range of 10⁻³ - 10⁻⁴M). The stability of the purified dimers was questioned and therefore in order to improve stability, the addition on 5% glycerol, 0.5% Triton X100 or 0.5% NP40 (Sigma) was included in the purification of 2 TAR1-27 dimers (d2 and d16). Addition of NP40 or Triton X100™ improved the yield of purified product approximately 2 fold. Both dimers were assessed in the receptor assay. TARl-27d2 gave IC50 of ~30nM under all purification conditions. TAR1-27d16 showed no neutralisation effect when purified without the use of stabilising agents but gave an IC50 of ~50nM when purified under stabilising conditions. No further analysis was conducted.

### 2.4 TAR2-5 dimers

3 x 96 clones were picked from the second round selections encompassing all the libraries and selection conditions. 2ml supernatant preps were made for analysis. Protein A and antigen ELISAs were conducted for each plate. 30 interesting clones were identified as having good off-rates by BIAcore (Koff ranges between 10⁻² - 10³M). The clones were sequenced and 13 unique dimers were identified by sequence analysis.

**Table 4: TAR1-5 dimers.**

| Dimer | Cell type | Purification | Protein Fraction | Elution conditions | Receptor/ Cell assay |
|---|---|---|---|---|---|
| TAR1-5d1 | HB2151 | Protein L + FPLC | small dimeric species | 0.1M glycine pH2.5 | RA~30nM |
| TAR1-5d2 | HB2151 | Protein L + FPLC | small dimeric species | 0.1M glycine pH2.5 | RA~50nM |
| TAR1-5d3 | HB2151 | Protein L + FPLC | large dimeric species | 0.1M glycine pH2.5 | RA~300nM |
| TAR1-5d4 | HB2151 | Protein L + FPLC | small dimeric species | 0.1M glycine pH2.5 | RA~3nM |
| TAR1-5d5 | HB2151 | Protein L + FPLC | large dimeric species | 0.1M glycine pH2.5 | RA~200nM |
| TAR1-5d6 | HB2151 | Protein L +FPLC | Large dimeric species | 0.1M glycine pH2.5 | RA~100nM |

| | | | | | |
|---|---|---|---|---|---|
| *note dimer 2 and dimer 3 have the same second dAb (called dAb2), however have different linker lengths (d2 = (Gly₄Ser)₃, d3 = (Gly₄Ser)₃). dAb1 is the partner dAb to dimers 1, 5 and 6. dAb3 is the partner dAb to dimer4. None of the partner dAbs neutralise alone. FPLC purification is by cation exchange unless otherwise stated. The optimal dimeric species for each dimer obtained by FPLC was determined in these assays. | | | | | |

**Table 5: TAR1-5-19 dimers**

| Dimer | Cell type | Purification | Protein Fraction | Elution conditions | Receptor/ Cell assay |
|---|---|---|---|---|---|
| TAR1-5-19d1 | TOP10F' | Protein L | Total protein | 0.1M glycine pH 2.0 | RA~15nM |
| TAR1-5-19 d2 (no stop codon) | TOP10F' | Protein L | Total protein | 0.1M glycine pH 2.0 + 0.05%NP40 | RA~2nM |
| TAR1-5-19d3 (no stop codon) | TOP10F' | Protein L | Total protein | 0.1M glycine pH 2.5 + 0.05%NP40 | RA~8nM |
| TAR1-5-19d4 | TOP10F' | Protein L + FPLC | FPLC purified fraction | 0.1M glycine pH2.0 | RA~2-5nM CA~12nM |
| TAR1-5-19d5 | TOP10F' | Protein L | Total protein | 0.1M glycine pH2.0 + NP40 | RA~8nM CA~10nM |
| TAR1-5-19 d6 | TOP10F' | Protein L | Total protein | 0.1M glycine pH 2.0 | RA~10nM |

**Table 6: TAR1-5-19 homodimers**

| Dimer | Cell type | Purification | Protein Fraction | Elution conditions | Receptor/ Cell assay |
|---|---|---|---|---|---|
| TAR1-5-19 3U homodimer | HB2151 | Protein L | Total protein | 0.1M glycine pH2.5 | RA~20nM CA~30nM |
| TAR1-5-19 5U homodimer | HB2151 | Protein L | Total protein | 0.1M glycine pH2.5 | RA~2nM CA~3nM |
| TAR1-5-19 7U homodimer | HB2151 | Protein L | Total protein | 0.1M glycine pH2.5 | RA~10nM CA~15nM |
| TAR1-5-19 cys hinge | HB2151 | Protein L + FPLC | FPLC purified dimer fraction | 0.1M glycine pH2.5 | RA~2nM |
| TAR1-5-19CH/ TAR1-5-19 CK | HB2151 | Protein | Total protein | 0.1M glycine pH2.5 | RA~1nM |

**Table 7: TAR1-5/TAR1-5-19 Fabs**

| Dimer | Cell type | Purification | Protein Fraction | Elution conditions | Receptor/ Cell assay |
|---|---|---|---|---|---|
| TAR1-5CH/ dAb1 CK | HB2151 | Protein L | Total protein | 0.1M citrate pH2.6 | RA~90nM |
| TAR1-5CH/ dAb2 CK | HB2151 | Protein L | Total protein | 0.1M glycine pH2.5 | RA~30nM CA~60nM |
| dAb3CH/ TAR1-5CK | HB2151 | Protein L | Total protein | 0.1M citrate pH2.6 | RA~100n M |
| TAR1-5-19CH/ dAb1 CK | HB2151 | Protein L | Total protein | 0.1M glycine pH2.0 | RA~6nM |
| dAb1 CH/ TAR1-5-19CK | HB2151 | Protein L | 0.1M glycine pH2.0 | Myc/flag | RA~6nM |
| TAR1-5-19CH/ dAb2 CK | HB2151 | Protein L | Total protein | 0.1M glycine pH2.0 | RA~8nM CA~12nM |
| TAR1-5-19CH/ dAb3CK | HB2151 | Protein L | Total protein | 0.1M glycine pH2.0 | RA~3nM |

### Example 7. dAb dimerisation by terminal cysteine linkage

### Summary

For dAb dimerisation, a free cysteine has been engineered at the C-terminus of the protein. When expressed the protein forms a dimer which can be purified by a two step purification method.

### PCR construction of TAR1-5-19CYS dimer

See example 8 describing the dAb trimer. The trimer protocol gives rise to a mixture of monomer, dimer and trimer.

### Expression and purification of TAR1-5-19CYS dimer

The dimer was purified from the supernatant of the culture by capture on Protein L agarose as outlined in the example 8.

### Separation of TAR1-5-19CYS monomer from the TAR1-5-19CYS dimer

Prior to cation exchange separation, the mixed monomer/dimer sample was buffer exchanged into 50 mM sodium acetate buffer pH 4.0 using a PD-10 column (Amersham Pharmacia), following the manufacturer's guidelines. The sample was then applied to a 1mL Resource S cation exchange column (Amersham Pharmacia), which had been pre-equilibrated with 50 mM sodium acetate pH 4.0. The monomer and dimer were separated using the following salt gradient in 50 mM sodium acetate pH 4.0:
150 to 200 mM sodium chloride over 15 column volumes
200 to 450 mM sodium chloride over 10 column volumes
450 to 1000 mM sodium chloride over 15 column volumes

Fractions containing dimer only were identified using SDS-PAGE and then pooled and the pH increased to 8 by the addition of 1/5 volume of 1M Tris pH 8.0.

*In vitro* functional binding assay: TNF receptor assay and cell assay

The affinity of the dimer for human TNFα was determined using the TNF receptor and cell assay. IC50 in the receptor assay was approximately 0.3-0.8 nM; ND50 in the cell assay was approximately 3-8 nM.

Other possible TAR1-5-19CYS dimer formats

### PEG dimers and custom synthetic maleimide dimers

Nektar (Shearwater) offer a range of bi-maleimide PEGs [mPEG2-(MAL)2 or mPEG-(MAL)2] which would allow the monomer to be formatted as a dimer, with a small linker separating the dAbs and both being linked to a PEG ranging in size from 5 to 40 kDa. It has been shown that the 5kDa mPEG-(MAL)2 (ie, [TAR1-5-19]-Cys-maleimide-PEG x 2, wherein the maleimides are linked together in the dimer) has an affinity in the TNF receptor assay of ~ 1-3 nM. Also the dimer can also be produced using TMEA (Tris[2-maleimidoethyl]amine) (Pierce Biotechnology) or other bi-functional linkers.

It is also possible to produce the disulphide dimer using a chemical coupling procedure using 2,2'-dithiodipyridine (Sigma Aldrich) and the reduced monomer.

### Addition of a polypeptide linker or hinge to the C-terminus of the dAb.

A small linker, either (Gly₄Ser)ₙ where n=1 to 10, eg, 1, 2, 3, 4, 5, 6 or 7, an immunoglobulin (eg, IgG hinge region or random peptide sequence (eg, selected from a library of random peptide sequences) can be engineered between the dAb and the terminal cysteine residue. This can then be used to make dimers as outlined above.

### Example 8. dAb trimerisation

### Summary

For dAb trimerisation, a free cysteine is required at the C-terminus of the protein. The cysteine residue, once reduced to give the free thiol, can then be used to specifically couple the protein to a trimeric maleimide molecule, for example TMEA (Tris[2-maleimidoethyl]amine).

### PCR construction of TAR1-5-19CYS

The following oligonucleotides were used to specifically PCR TAR1-5-19 with a *Sal*I and *Bam*HI sites for cloning and also to introduce a C-terminal cysteine residue: 
SalI
~~~~~~~~~
Trp Ser Ala Ser Thr Asp* Ile Gln Met Thr Gln Ser Pro Ser Ser Leu Ser Ala Ser Val
1 TGG AGC GCG TCG ACG GAC ATC CAG ATG ACC CAG TCT CCA TCC TCT CTG TCT GCA TCT GTA
ACC TCG CGC AGC TGC CTG TAG GTC TAC TGG GTC AGA GGT AGG AGA GAC AGA CGT AGA CAT

Gly Asp Arg Val Thr Ile Thr Cys Arg Ala Ser Gln Ser Ile Asp Ser Tyr Leu His Trp
61 GGA GAC CGT GTC ACC ATC ACT TGC CGG GCA AGT CAG AGC ATT GAT AGT TAT TTA CAT TGG
CCT CTG GCA CAG TGG TAG TGA ACG GCC CGT TCA GTC TCG TAA CTA TCA ATA AAT GTA ACC

Tyr Gln Gln Lys Pro Gly Lys Ala Pro Lys Leu Leu Ile Tyr Ser Ala Ser Glu Leu Gln
121 TAC CAG CAG AAA CCA GGG AAA GCC CCT AAG CTC CTG ATC TAT AGT GCA TCC GAG TTG CAA
ATG GTC GTC TTT GGT CCC TTT CGG GGA TTC GAG GAC TAG ATA TCA CGT AGG CTC AAC GTT

Ser Gly Val Pro Ser Arg Phe Ser Gly Ser Gly Ser Gly Thr Asp Phe Thr Leu Thr Ile
181 AGT GGG GTC CCA TCA CGT TTC AGT GGC AGT GGA TCT GGG ACA GAT TTC ACT CTC ACC ATC
TCA CCC CAG GGT AGT GCA AAG TCA CCG TCA CCT AGA CCC TGT CTA AAG TGA GAG TGG TAG

Ser Ser Leu Gln Pro Glu Asp Phe Ala Thr Tyr Tyr Cys Gln Gln Val Val Trp Arg Pro
241 AGC AGT CTG CAA CCT GAA GAT TTT GCT ACG TAC TAC TGT CAA CAG GTT GTG TGG CGT CCT
TCG TCA GAC GTT GGA CTT CTA AAA CGA TGC ATG ATG ACA GTT GTC CAA CAC ACC GCA GGA

BamHI
~~~~~~~~~
Phe Thr Phe Gly Gln Gly Thr Lys Val Glu Ile Lys Arg Cys *** *** Gly Ser Gly
301 TTT ACG TTC GGC CAA GGG ACC AAG GTG GAA ATC AAA CGG TGC TAA TAA GGA TCC GGC
AAA TGC AAG CCG GTT CCC TGG TTC CAC CTT TAG TTT GCC ACG ATT ATT CCT AGG CCG
 (* start of TAR1-5-19CYS sequence; TAR1-5-19CYS amino acid sequence (SEQ ID NO:293; TAR1-5-19CYS nucleotide sequences (SEQ ID NO:294, coding strand; SEQ ID NO:295, noncoding strand))
Forward primer
5'-TGGAGCGCGTCGACGGACATCCAGATGACCCAGTCTCCA-3' (SEQ ID NO:296)
Reverse primer
5'-TTAGCAGCCGGATCCTTATTAGCACCGTTTGATTTCCAC-3' (SEQ ID NO:297)

The PCR reaction (50µL volume) was set up as follows: 200µM dNTPs, 0.4µM of each primer, 5 µL of 10x *Pfu*Turbo buffer (Stratagene), 100 ng of template plasmid (encoding TAR1-5-19), 1µL of *Pfu*Turbo enzyme (Stratagene) and the volume adjusted to 50µL using sterile water. The following PCR conditions were used: initial denaturing step 94 °C for 2 mins, then 25 cycles of 94 °C for 30 secs, 64 °C for 30 sec and 72 °C for 30 sec. A final extension step was also included of 72 °C for 5 mins. The PCR product was purified and digested with *Sal*I and *Bam*HI and ligated into the vector which had also been cut with the same restriction enzymes. Correct clones were verified by DNA sequencing.

### Expression and purification of TAR1-5-19CYS

TAR1-5-19CYS vector was transformed into BL21 (DE3) pLysS chemically competent cells (Novagen) following the manufacturer's protocol. Cells carrying the dAb plasmid were selected for using 100µg/mL carbenicillin and 37 µg/mL chloramphenicol. Cultures were set up in 2L baffled flasks containing 500 mL of terrific broth (Sigma-Aldrich), 100µg/mL carbenicillin and 37 µg/mL chloramphenicol. The cultures were grown at 30 °C at 200rpm to an O.D.600 of 1-1.5 and then induced with 1mM IPTG (isopropyl-beta-D-thiogalactopyranoside, from Melford Laboratories). The expression of the dAb was allowed to continue for 12-16 hrs at 30 °C. It was found that most of the dAb was present in the culture media. Therefore, the cells were separated from the media by centrifugation (8,000xg for 30 mins), and the supernatant used to purify the dAb. Per litre of supernatant, 30 mL of Protein L agarose (Affitech) was added and the dAb allowed to batch bind with stirring for 2 hours. The resin was then allowed to settle under gravity for a further hour before the supernatant was siphoned off. The agarose was then packed into a XK 50 column (Amersham Phamacia) and was washed with 10 column volumes of PBS. The bound dAb was eluted with 100 mM glycine pH 2.0 and protein containing fractions were then neutralized by the addition of 1/5 volume of 1 M Tris pH 8.0. Per litre of culture supernatant 20 mg of pure protein was isolated, which contained a 50:50 ratio of monomer to dimer.

### Trimerisation of TAR1-5-19CYS

2.5 ml of 100 µM TAR1-5-19CYS was reduce with 5 mM dithiothreitol and left at room temperature for 20 minutes. The sample was then buffer exchanged using a PD-10 column (Amersham Pharmacia). The column had been pre-equilibrated with 5 mM EDTA, 50 mM sodium phosphate pH 6.5, and the sample applied and eluted following the manufactures guidelines. The sample was placed on ice until required. TMEA (Tris[2-maleimidoethyl]amine) was purchased from Pierce Biotechnology. A 20 mM stock solution of TMEA was made in 100% DMSO (dimethyl sulphoxide). It was found that a concentration of TMEA greater than 3:1 (molar ratio of dAb:TMEA) caused the rapid precipitation and cross-linking of the protein. Also the rate of precipitation and cross-linking was greater as the pH increased. Therefore using 100 µM reduced TAR1-5-19CYS, 25 µM TMEA was added to trimerise the protein and the reaction allowed to proceed at room temperature for two hours. It was found that the addition of additives such as glycerol or ethylene glycol to 20% (v/v), significantly reduced the precipitation of the trimer as the coupling reaction proceeded. After coupling, SDS-PAGE analysis showed the presence of monomer, dimer and trimer in solution.

### Purification of the trimeric TAR1-5-19CYS

40 µL of 40% glacial acetic acid was added per mL of the TMEA-TAR1-5-19cys reaction to reduce the pH to ~4. The sample was then applied to a 1mL Resource S cation exchange column (Amersham Pharmacia), which had been pre-equilibrated with 50 mM sodium acetate pH 4.0. The dimer and trimer were partially separated using a salt gradient of 340 to 450 mM Sodium chloride, 50 mM sodium acetate pH 4.0 over 30 column volumes. Fractions containing trimer only were identified using SDS-PAGE and then pooled and the pH increased to 8 by the addition of 1/5 volume of 1M Tris pH 8.0. To prevent precipitation of the trimer during concentration steps (using 5K cut off Viva spin concentrators; Vivascience), 10% glycerol was added to the sample.

### In vitro functional binding assay: TNF receptor assay and cell assay

The affinity of the trimer for human TNFα was determined using the TNF receptor and cell assay. IC50 in the receptor assay was 0.3nM; ND50 in the cell assay was in the range of 3 to 10nM (eg, 3nM).

### Other possible TAR1-5-19CYS trimer formats

TAR1-5-19CYS may also be formatted into a trimer using the following reagents:
PEG trimers and custom synthetic maleimide trimers
Nektar (Shearwater) offer a range of multi arm PEGs, which can be chemically modified at the terminal end of the PEG. Therefore using a PEG trimer with a maleimide functional group at the end of each arm would allow the trimerisation of the dAb in a manner similar to that outlined above using TMEA. The PEG may also have the advantage in increasing the solubility of the trimer thus preventing the problem of aggregation. Thus, one could produce a dAb trimer in which each dAb has a C-terminal cysteine that is linked to a maleimide functional group, the maleimide functional groups being linked to a PEG trimer.

### Addition of a polypeptide linker or hinge to the C-terminus of the dAb

A small linker, either (Gly₄Ser)ₙ where n= 1 to 10, eg, 1, 2, 3, 4, 5, 6 or 7 , an immunoglobulin (eg, IgG hinge region or random peptide sequence (eg, selected from a library of random peptide sequences) could be engineered between the dAb and the terminal cysteine residue. When used to make multimers (eg, dimers or trimers), this again would introduce a greater degree of flexibility and distance between the individual monomers, which may improve the binding characteristics to the target, eg a multisubunit target such as human TNFα.

### Example 9. Selection of a collection of single domain antibodies (dAbs) directed against human serum albumin (HSA) and mouse serum albumin (MSA).

This example explains a method for making a single domain antibody (dAb) directed against serum albumin. Selection of dAbs against both mouse serum albumin (MSA) and human serum albumin (HSA) is described. Three human phage display antibody libraries were used in this experiment, each based on a single human framework for V_{H} (see Figure 13: sequence of dummy V_{H} based on V3-23/DP47 and JH4b) or Vκ (see Figure 15: sequence of dummy Vκ based on o12/o2/DPK9 and Jk1) with side chain diversity encoded by NNK codons incorporated in complementarity determining regions (CDR1, CDR2 and CDR3).
Library 1 (V_{H}):
   Diversity at positions: H30, H31, H33, H35, H50, H52, H52a, H53, H55, H56, H58, H95, H97, H98.
   Library size: 6.2 x 10⁹
Library 2 (V_{H}):
   Diversity at positions: H30, H31, H33, H35, H50, H52, H52a, H53, H55, H56, H58, H95, H97, H98, H99, H100, H100a, H100b.
   Library size: 4.3 x 10⁹
Library 3 (Vκ):
   Diversity at positions: L30, L31, L32, L34, L50, L53, L91, L92, L93, L94, L96 Library size: 2 x 10⁹

The V_{H} and Vκ libraries have been preselected for binding to generic ligands protein A and protein L respectively so that the majority of clones in the unselected libraries are functional. The sizes of the libraries shown above correspond to the sizes after preselection.

Two rounds of selection were performed on serum albumin using each of the libraries separately. For each selection, antigen was coated on immunotube (nunc) in 4ml of PBS at a concentration of 100µg/ml. In the first round of selection, each of the three libraries was panned separately against HSA (Sigma) and MSA (Sigma). In the second round of selection, phage from each of the six first round selections was panned against (i) the same antigen again (eg 1^{st} round MSA, 2^{nd} round MSA) and (ii) against the reciprocal antigen (eg 1^{st} round MSA, 2^{nd} round HSA) resulting in a total of twelve 2^{nd} round selections. In each case, after the second round of selection 48 clones were tested for binding to HSA and MSA. Soluble dAb fragments were produced as described for scFv fragments by Harrison et al, Methods Enzymol. 1996;267:83-109 and standard ELISA protocol was followed (Hoogenboom et al. (1991) Nucleic Acids Res., 19: 4133) except that 2% tween PBS was used as a blocking buffer and bound dAbs were detected with either protein L-HRP (Sigma) (for the Vκs) and protein A -HRP (Amersham Pharmacia Biotech) (for the V_{H}s).

dAbs that gave a signal above background indicating binding to MSA, HSA or both were tested in ELISA insoluble form for binding to plastic alone but all were specific for serum albumin. Clones were then sequenced (see table below) revealing that 21 unique dAb sequences had been identified. The minimum similarity (at the amino acid level) between the Vκ dAb clones selected was 86.25% ((69/80)x100; the result when all the diversified residues are different, eg clones 24 and 34). The minimum similarity between the V_{H} dAb clones selected was 94 % ((127/136)x100).

Next, the serum albumin binding dAbs were tested for their ability to capture biotinylated antigen from solution. ELISA protocol (as above) was followed except that ELISA plate was coated with 1µg/ml protein L (for the Vκ clones) and 1µg/ml protein A (for the V_{H} clones). Soluble dAb was captured from solution as in the protocol and detection was with biotinylated MSA or HSA and streptavidin HRP. The biotinylated MSA and HSA had been prepared according to the manufacturer's instructions, with the aim of achieving an average of 2 biotins per serum albumin molecule. Twenty four clones were identified that captured biotinylated MSA from solution in the ELISA. Two of these (clones 2 and 38 below) also captured biotinylated HSA. Next, the dAbs were tested for their ability to bind MSA coated on a CM5 biacore chip. Eight clones were found that bound MSA on the biacore.

**Table 8.**

| dAb (all capture biotinylated MSA) | H or κ | CDR1 | CDR2 | CDR3 | Binds MSA in biacore ? | Captures biotinylated HSA? |
|---|---|---|---|---|---|---|
| Vκ library 3 template (dummy) | κ | XXXLX (SEQ ID NO:298) | XASXLQS (SEQ ID NO:299) | QQXXXXPXT (SEQ ID NO:300) | | |
| 2,4,7,41, | κ | SSYLN (SEQ ID NO:301) | RASPLQS (SEQ ID NO:302) | QQTYSVPPT (SEQ ID NO:303) | | ✔ all 4 bind |
| 38, 54 | κ | SSYLN (SEQ ID NO:304) | RASPLQS (SEQ ID NO:305) | QQTYRIPPT (SEQ ID NO:306) | | ✔ both bind |
| 46,47,52,56 | κ | FKSLK (SEQ ID NO:307) | NASYLQS (SEQ ID NO:308) | QQVVYWPVT (SEQ ID NO:309) | | |
| 13, 15 | κ | YYHLK (SEQ ID NO:310) | KASTLQS (SEQ ID NO:311) | QQVRKVPRT (SEQ ID NO:312) | | |
| 30,35 | κ | RRYLK (SEQ ID NO:313) | QASVLQS (SEQ ID NO:314) | QQGLYPPIT (SEQ ID NO:315) | | |
| 19, | κ | YNWLK (SEQ ID NO:316) | RASSLQS (SEQ ID NO:317) | QQNVVIPRT (SEQ ID NO:318) | | |
| 22, | κ | LWHLR (SEQ ID NO:319) | HASLLQS (SEQ ID NO:320) | QQSAVYPKT (SEQ ID NO:321) | | |
| 23, | κ | FRYLA (SEQ ID NO:322) | HASHLQS (SEQ ID NO:323) | QQRLLYPKT (SEQ ID NO:324) | | |
| 24, | κ | FYHLA (SEQ ID NO:325) | PASKLQS (SEQ ID NO:326) | QQRARWPRT (SEQ ID NO:327) | | |
| 31, | κ | IWHLN (SEQ ID NO:328) | RASRLQS (SEQ ID NO:329) | QQVARVPRT (SEQ ID NO:330) | | |
| 33, | κ | YRYLR (SEQ ID NO:331) | KASSLQS (SEQ ID NO:332) | QQYVGYPRT (SEQ ID NO:333) | | |
| 34, | κ | LKYLK (SEQ ID NO:334) | NASHLQS (SEQ ID NO:335) | QQTTYYPIT (SEQ ID NO:336) | | |
| 53, | κ | LRYLR (SEQ ID NO:337) | KASWLQS (SEQ ID NO:338) | QQVLYYPQT (SEQ ID NO:339) | | |
| 11, | κ | LRSLK (SEQ ID NO:340) | AASRLQS (SEQ ID NO:341) | QQVVYWPAT (SEQ ID NO:342) | ✔ | |
| 12, | κ | FRHLK (SEQ ID NO:343) | AASRLQS (SEQ ID NO:344) | QQVALYPKT (SEQ ID NO:345) | ✔ | |
| 17, | κ | RKYLR (SEQ ID NO:346) | TASSLQS (SEQ ID NO:347) | QQNLFWPRT (SEQ ID NO:348) | ✔ | |
| 18, | κ | RRYLN (SEQ ID NO:349) | AASSLQS (SEQ ID NO: 350) | QQMLFYPKT (SEQ ID NO:351) | ✔ | |
| 16, 21 | κ | IKHLK (SEQ ID NO:352) | GASRLQS (SEQ ID NO:353) | QQGARWPQT (SEQ ID NO:354) | ✔ | |
| 25,26 | κ | YYHLK (SEQ ID NO:355) | KASTLQS (SEQ ID NO:356) | QQVRKVPRT (SEQ ID NO:357) | ✔ | |
| 27, | κ | YKHLK (SEQ ID NO:358) | NASHLQS (SEQ ID NO:359) | QQVGRYPKT (SEQ ID NO:360) | ✔ | |
| 55, | κ | FKSLK (SEQ ID NO:361) | NASYLQS (SEQ ID NO:362) | QQVVYWPVT (SEQ ID NO:363) | ✔ | |
| | | | | | | |
| V_{H} library 1 (and 2) template (dummy) | H | XXYXXX (SEQ ID NO:364) | XIXXXGXXTXYADS VKG (SEQ ID NO:365) | XXXX (XXXX) FDY (SEQ ID NO:366) | | |
| 8, 10 | H | WVYQMD (SEQ ID NO:367) | SISAFGAKTLYADS VKG (SEQ ID NO:368) | LSGKFDY (SEQ ID NO:369) | | |
| 36, | H | WSYQMT (SEQ ID NO:370) | SISSFGSSTLYADS VKG (SEQ ID NO:371) | GRDHNYSLFDY (SEQ ID NO:372) | | |

In all cases the frameworks were identical to the frameworks in the corresponding dummy sequence, with diversity in the CDRs as indicated in the table above.

Of the eight clones that bound MSA on the biacore, two clones that are highly expressed in *E. coli* (clones MSA16 and MSA26) were chosen for further study (see example 10). Full nucleotide and amino acid sequences for MSA16 and 26 are given in figure 16.

Example 10. Determination of affinity and serum half-life in mouse of MSA binding dAbs MSA16 and MSA26.

dAbs MSA16 and MSA26 were expressed in the periplasm of *E. coli* and purified using batch absorbtion to protein L-agarose affinity resin (Affitech, Norway) followed by elution with glycine at pH 2.2. The purified dAbs were then analysed by inhibition biacore to determine K_{d}. Briefly, purified MSA16 and MSA26 were tested to determine the concentration of dAb required to achieve 200RUs of response on a biacore CM5 chip coated with a high density of MSA. Once the required concentrations of dAb had been determined, MSA antigen at a range of concentrations around the expected K_{d} was premixed with the dAb and incubated overnight. Binding to the MSA coated biacore chip of dAb in each of the premixes was then measured at a high flow-rate of 30 µl/minute. The resulting curves were used to create Klotz plots, which gave an estimated K_{d} of 200nM for MSA16 and 70nM for MSA 26 (Figure 17 A & B).

Next, clones MSA16 and MSA26 were cloned into an expression vector with the HA tag (nucleic acid sequence: TATCCTTATGATGTTCCTGATTATGCA (SEQ ID NO: 373) and amino acid sequence: YPYDVPDYA (SEQ ID NO:374)) and 2-10 mg quantities were expressed in *E. coli* and purified from the supernatant with protein L-agarose affinity resin (Affitech, Norway) and eluted with glycine at pH2.2. Serum half life of the dAbs was determined in mouse. MSA26 and MSA16 were dosed as single i.v. injections at approx 1.5mg/kg into CD1 mice. Analysis of serum levels was by goat anti-HA (Abcam, UK) capture and protein L-HRP (invitrogen) detection ELISA which was blocked with 4% Marvel. Washing was with 0.05% tween PBS. Standard curves of known concentrations of dAb were set up in the presence of 1xmouse serum to ensure comparability with the test samples. Modelling with a 2 compartment model showed MSA-26 had a t1/2α of 0.16hr, a t1/2β of 14.5hr and an area under the curve (AUC) of 465hr.mg/ml (data not shown) and MSA-16 had a t1/2α of 0.98hr, a t1/2β of 36.5hr and an AUC of 913hr.mg/ml (figure 18). Both anti-MSA clones had considerably lengthened half life compared with HEL4 (an anti-hen egg white lysozyme dAb) which had a t1/2α of 0.06hr, and a t1/2β of 0.34hr.

### Example 11. Creation of V_{H}-V_{H} and Vκ- Vκ dual specific Fab like fragments

This example describes a method for making V_{H}- V_{H} and Vκ-Vκ dual specifics as Fab like fragments. Before constructing each of the Fab like fragments described, dAbs that bind to targets of choice were first selected from dAb libraries similar to those described in example 9. A V_{H} dAb, HEL4, that binds to hen egg lysozyme (Sigma) was isolated and a second V_{H} dAb (TAR2h-5) that binds to TNFα receptor (R and D systems) was also isolated. The sequences of these are given in the sequence listing. A Vκ dAb that binds TNFα (TAR1-5-19) was isolated by selection and affinity maturation and the sequence is also set forth in the sequence listing. A second Vκ dAb (MSA 26) described in example 9 whose sequence is in figure 17B was also used in these experiments.

DNA from expression vectors containing the four dAbs described above was digested with enzymes SalI and NotI to excise the DNA coding for the dAb. A band of the expected size (300-400bp) was purified by running the digest on an agarose gel and excising the band, followed by gel purification using the Qiagen gel purification kit (Qiagen, UK). The DNA coding for the dAbs was then inserted into either the C_{H} or Cκ vectors (Figs 8 and 9) as indicated in the table below.

**Table 9.**

| dAb | Target antigen | dAb V_{H} or dAb Vκ | Inserted into vector | tag (C terminal ) | Antibiotic resistance |
|---|---|---|---|---|---|
| HEL4 | Hen egg lysozyme | V_{H} | C_{H} | Myc | Chloramphenic ol |
| TAR2-5 | TNF receptor | V_{H} | Cκ | Flag | Ampicillin |
| TAR1-5-19 | TNF α | Vκ | C_{H} | Myc | Chloramphenic ol |
| MSA 26 | Mouse serum albumin | Vκ | Cκ | Flag | Ampicillin |

The V_{H} C_{H} and V_{H} Cκ constructs were cotransformed into HB2151 cells. Separately, the Vκ C_{H} and Vκ Cκ constructs were contransformed into HB2151 cells. Cultures of each of the cotransformed cell lines were grown overnight (in 2xTy containing 5% glucose, 10µg/ml chloramphenicol and 100µg/ml ampicillin to maintain antibiotic selection for both C_{H} and Cκ plasmids). The overnight cultures were used to inoculate fresh media (2xTy, 10µg/ml chloramphenicol and 100µg/ml ampicillin) and grown to OD 0.7-0.9 before induction by the addition of IPTG to express their C_{H} and Cκ constructs. Expressed Fab like fragment was then purified from the periplasm by protein A purification (for the contransformed V_{H} C_{H} and V_{H} Cκ) and MSA affinity resin purification (for the contransformed Vκ C_{H} and Vκ Cκ).

### V_{H}-V_{H} dual specific

Expression of the V_{H} C_{H} and V_{H} Cκ dual specific was tested by running the protein on a gel. The gel was blotted and a band the expected size for the Fab fragment could be detected on the Western blot via both the myc tag and the flag tag, indicating that both the V_{H} C_{H} and V_{H} Cκ parts of the Fab like fragment were present. Next, in order to determine whether the two halves of the dual specific were present in the same Fab-like fragment, an ELISA plate was coated overnight at 4°C with 100 µl per well of hen egg lysozyme (HEL) at 3 mg/ml in sodium bicarbonate buffer. The plate was then blocked (as described in example 1) with 2% tween PBS followed by incubation with the V_{H} C_{H} /V_{H} Cκ dual specific Fab like fragment. Detection of binding of the dual specific to the HEL was via the non cognate chain using 9e10 (a monoclonal antibody that binds the myc tag, Roche) and anti mouse IgG-HRP (Amersham Pharmacia Biotech). The signal for the V_{H} C_{H}/V_{H} Cκ dual specific Fab like fragment was 0.154 compared to a background signal of 0.069 for the V_{H} Cκ chain expressed alone. This demonstrates that the Fab like fragment has binding specificity for target antigen.

### V_{κ}-V_{κ} dual specific

After purifying the contransformed Vκ C_{H} and Vκ Cκ dual specific Fab like fragment on an MSA affinity resin, the resulting protein was used to probe an ELISA plate coated with 1µg/ml TNFα and an ELISA plate coated with 10µg/ml MSA. As predicted, there was signal above background when detected with protein L-HRP on both ELISA plates (data not shown). This indicated that the fraction of protein able to bind to MSA (and therefore purified on the MSA affinity column) was also able to bind TNFα in a subsequent ELISA, confirming the dual specificity of the antibody fragment. This fraction of protein was then used for two subsequent experiments. Firstly, an ELISA plate coated with 1µg/ml TNFα was probed with dual specific Vκ C_{H} and Vκ Cκ Fab like fragment and also with a control TNFα binding dAb at a concentration calculated to give a similar signal on the ELISA. Both the dual specific and control dAb were used to probe the ELISA plate in the presence and in the absence of 2mg/ml MSA. The signal in the dual specific well was reduced by more than 50% but the signal in the dAb well was not reduced at all (see figure 19a). The same protein was also put into the receptor assay with and without MSA and competition by MSA was also shown (see figure 19c). This demonstrates that binding of MSA to the dual specific is competitive with binding to TNFα.

### Example 12. Creation of a Vκ- Vκ dual specific cys bonded dual specific with specificity for mouse serum albumin and TNFα

This example describes a method for making a dual specific antibody fragment specific for both mouse serum albumin and TNFα by chemical coupling via a disulphide bond. Both MSA16 (from example 1) and TAR1-5-19 dAbs were recloned into a pET based vector with a C terminal cysteine and no tags. The two dAbs were expressed at 4-10 mg levels and purified from the supernatant using protein L-agarose affinity resin (Affitiech, Norway). The cysteine tagged dAbs were then reduced with dithiothreitol. The TAR1-5-19 dAb was then coupled with dithiodipyridine to block reformation of disulphide bonds resulting in the formation of PEP 1-5-19 homodimers. The two different dAbs were then mixed at pH 6.5 to promote disulphide bond formation and the generation of TAR1-5-19, MSA16 cys bonded heterodimers. This method for producing conjugates of two unlike proteins was originally described by King *et al.* (King TP, Li Y Kochoumian L Biochemistry. 1978 voll7:1499-506 Preparation of protein conjugates via intermolecular disulfide bond formation.) Heterodimers were separated from monomeric species by cation exchange. Separation was confirmed by the presence of a band of the expected size on a SDS gel. The resulting heterodimeric species was tested in the TNF receptor assay and found to have an IC50 for neutralising TNF of approximately 18 nM. Next, the receptor assay was repeated with a constant concentration of heterodimer (18nM) and a dilution series of MSA and HSA. The presence of HSA at a range of concentrations (up to 2 mg/ml) did not cause a reduction in the ability of the dimer to inhibit TNFα. However, the addition of MSA caused a dose dependant reduction in the ability of the dimer to inhibit TNFα (figure 20).This demonstrates that MSA and TNFα compete for binding to the cys bonded TAR1-5-19, MSA16 dimer.

### Data Summary

A summary of data obtained in the experiments set forth in the foregoing examples is set forth in Annex 4.

### EXAMPLE 13.

Activity of Anti-mouse TNFR1 dAbs and Anti-human TNFR1 dAbs

**Table 10 Activity of Anti-mouse TNFR1 dAbs**

| dAb | Activity (IC50) | |
|---|---|---|
| | L929 Cell Assay | Receptor Binding Assay |
| TAR2m-19 | 10 µM | 2µM |
| TAR2m-20 | n/d | 150 nM |
| TAR2m-21 | 400 nM | n/d |
| TAR2m-24 | 1 µM | 1.3 µM |
| TAR2m-21-23 | 1 nM | n/d |
| TAR2m-21-07 | 10 nM | n/d |
| TAR2m-21-43 | 6nM | n/d |
| TAR2m-21-48 | 6nM | n/d |
| TAR2m-21-10 | 30 nM | n/d |
| TAR2m-21-06 | 100 nM | n/d |
| TAR2m-21-17 | 300 nM | n/d |

| | | |
|---|---|---|
| n/d, not determined | | |

**Table 11 Activity of Anti-human TNFR1 dAbs**

| dAb | Activity (IC50) | |
|---|---|---|
| | HeLa IL-8 Cell Assay | Receptor Binding Assay |
| TAR2h-10 | 50 nM | 30 nM |
| TAR2h-12 | 100 nM | n/d |
| TAR2h-13 | 300 nM | n/d |
| TAR2h-14 | 300 nM | 30 nM |
| TAR2h-15 | n/d | 5 nM |
| TAR2h-16 | 200 nM | 30 nM |
| TAR2h-17 | n/d | 100 nM |
| TAR2h-18 | 400 nM | n/d |
| TAR2h-22 | n/d | 200 nM |
| TAR2h-27 | 3000 nM | 30 nM |
| TAR2h-29 | 300 nM | 300 nM |
| TAR2h-32 | 100 nM | n/d |
| TAR2h-34 | n/d | 300 nM |
| TAR2h-35 | 800 nM | n/d |
| TAR2h-41 | 30 nM | 8nM |
| TAR2h-42 | 10 nM | 15 nM |
| TAR2h-44 | 300 nM | 10 nM |
| TAR2h-47 | n/d | 8nM |
| TAR2h-51 | n/d | 80 nM |
| TAR2h-67 | 300 nM | n/d |
| TAR2h-10-1 | n/d | 10 nM |
| TAR2h-10-2 | n/d | 11 nM |
| TAR2h-10-3 | n/d | 11 nM |
| TAR2h-10-4 | n/d | 8 nM |
| TAR2h-10-5 | n/d | 11 nM |
| TAR2h-10-7 | 30 nM | n/d |
| TAR2h-10-27 | 10 nM | 2nM |
| TAR2h-10-55 | 20 nM | n/d |

| | | |
|---|---|---|
| n/d, not determined | | |

### MRC-5 IL-8 release assay

The activities of certain dAbs that bind human TNFR1 were assessed in the following MRC-5 cell assay. The assay is based on the induction of IL-8 secretion by TNF in MRC-5 cells and is adapted from the method described in Alceson, L. et al. Journal of Biological Chemistry 271:30517-30523 (1996), describing the induction of IL-8 by IL-1 in HUVEC. The activity of the dAbs was assayed by assessing IL-8 induction by human TNFα using MRC-5 cells instead of the HUVEC cell line. Briefly, MRC-5 cells were plated in microtitre plates and the plates were incubated overnight with dAb and human TNFα (300 pg/ml). Following incubation, the culture supernatant was aspirated and the IL-8 concentration in the supernatant was measured via a sandwich ELISA (R&D Systems). Anti-TNFR1 dAb activity resulted in a decrease in IL-8 secretion into the supernatant compared with control wells that were incubated with TNFα only.

### Example 14. Mouse septic shock model

The *in vivo* efficacy of an anti-TNFR1 dAb was assessed in a well-established experimental model for septic shock syndrome (Rothe *et al., Circulatory Shock 44*:51-56, (1995)). LPS-induced death in this model is dependent upon TNFR-1 (p55) activation. In this model mice were sensitized to the toxicity of LPS using D-galactosamine (D-GaIN). The lethal LPS dose for wild-type animals in this study was about 10 ng.

LPS (*Salmonella enteritidis,* Sigma, USA) and D-Galactosamine (D-GaIN, Sigma, USA) were injected intraperitoneally. D-Ga1N-sensitized (10 mg/mouse) control mice died within 18 hour following challenge with LPS (10 ng). Mortality of non-sensitized mice was recorded over a period of 1 day after challenge.

Mice were administered a dual specific ligand that binds mouse TNFR1 and mouse serum albumin (TAR2m-21-23 3U TAR7m-16; TAR7m-16 is also referred to herein as MSA16) or ENBREL® (entarecept; Immunex Corporation) by intraperitoneal injections 4 hours prior to the administration of LPS. (See, Table 12). Survival was monitored at 4-6 hour intervals over a period of 48 hours. Efficacy of anti-mouse TNFR1 dAbs was demonstrated by survival.

**Table 12.**

| Treatment Group | Agent and Dose | LPS dose per mouse (ng) | Number of animals | Number of Survivors at 24 hours |
|---|---|---|---|---|
| 1 | Saline | 10 | 8 | 0/8 |
| 2 | 10 mg/kg ENBREL® (entarecept; Immunex Corporation) | 10 | 8 | 8/8 |
| 3 | 5.4 mg/kg TAR2m-21-23 3U TAR7m-16 | 10 | 8 | 4/8 |
| 4 | 1 mg/kg TAR2m-21-23 3U TAR7m-16 | 10 | 8 | 2/8 |
| 5 | 5.4 mg/kg TAR2m-21-23 3U TAR7m-16 | 0 | 2 | 2/2 |

TAR2m-21-23 3U TAR7m-16 is a dual specific ligand that contains a dAb that binds mouse TNFR1 that is joined through a peptide linker to a dAb that binds mouse serum albumin. A nucleotide sequence encoding TAR2m-21-23 3U TAR7m-16 and the amino acid sequence of the dual specific ligand are presented below as SEQ ID NO: 375 and SEQ ID NO:376, respectively.

GAGGTGCAGCTGTTGGAGTCTGGGGGAGGCTTGGTACAGCCTGGGGGGTCCCTGCG
TCTCTCCTGTGCAGCCTCCGGATTCACCTTTAATAGGTATAGTATGGGGTGGCTCC
GCCAGGCTCCAGGGAAGGGTCTAGAGTGGGTCTCACGGATTGATTCTTATGGTCGT
GGTACATACTACGAAGACCCCGTGAAGGGCCGGTTCAGCATCTCCCGCGACAATTC
CAAGAACACGCTGTATCTGCAAATGAACAGCCTGCGTGCCGAGGACACCGCCGTAT
ATTACTGTGCGAAAATTTCTCAGTTTGGGTCAAATGCGTTTGACTACTGGGGTCAG
GGAACCCAGGTCACCGTCTCGAGCGGTGGAGGCGGTTCAGGCGGAGGTGGCAGCGG
CGGTGGCGGGTCGACGGACATCCAGATGACCCAGTCTCCATCCTCCCTGTCTGCAT
CTGTAGGAGACCGTGTCACCATCACTTGCCGGGCAAGTCAGAGCATTATTAAGCAT
TTAAAGTGGTACCAGCAGAAACCAGGGAAAGCCCCTAAGCTCCTGATCTATGGTGC
ATCCCGGTTGCAAAGTGGGGTCCCATCACGTTTCAGTGGCAGTGGATCTGGGACAG
ATTTCACTCTCACCATCAGCAGTCTGCAACCTGAAGATTTTGCTACGTACTACTGT
CAACAGGGGGCTCGGTGGCCTCAGACGTTCGGCCAAGGGACCAAGGTGGAAATCAA
ACGGGCGGCCGCAGAACAAAAACTCATCTCAGAAGAGGATCTGAAT (SEQ ID
NO:375)

EVQLLESGGGLVQPGGSLRLSCAASGFTFNRYSMGWLRQAPGKGLEWVSRIDSYGR
GTYYEDPVKGRFSISRDNSKNTLYLQMNSLRAEDTAVYYCAKISQFGSNAFDYWGQ
GTQVTVSSGGGGSGGGGSGGGGSTDIQMTQSPSSLSASVGDRVTITCRASQSIIKH

LKWYQQKPGKAPKLLIYGASRLQSGVPSRFSGSGSGTDFTLTISSLQPEDFATYYC
QQGARWPQTFGQGTKVEIKRAAAEQKLISEEDLN (SEQ ID NO:376)

The presence of survivors in the TAR2m-21-23 3U TAR7m-16 treatment groups demonstrates that the anti-TNFR1 dAb was efficacious in inhibiting the activity of the receptor *in vivo,* and the results demonstrate that the effect was dose dependent. Moreover, the efficacy of the TAR2m-21-23 3U TAR7m-16 treatment compared favorably with the efficacy of ENBREL® (entarecept; Immunex Corporation). The survival of the animals which were treated with TAR2m-21-23 3U TAR7m-16 alone (Group 5, no LPS challenge) also demonstrates that TAR2m-21-23 3U TAR7m-16 was not toxic and did not agonise the receptor *in vivo* by receptor cross-linking.

Further studies confirmed that anti-TNFR1 dAbs do not agonise TNFR1 (act as TNFR1 agonists) in the absence of TNFα. L929 cells were cultured in media that contained a range of concentrations of either TAR2m-21-23 monomer, TAR2m-21-23 monomer cross-linked to a commercially available anti-myc antibody (9E10), TAR2m-21-23 3U TAR7m-16 or TAR2m-21-23 40K PEG. In the case of TAR2m-21-23 monomer cross-linked with the anti-myc antibody, the dAb and antibody were mixed in a 2:1 ratio and pre-incubated for one hour at room-temperature to simulate the effects of *in vivo* immune cross-linking prior to culture. TAR2m-21-23 monomer was incubated with the L929 cells at a concentration of 3000 nM. TAR2m-21-23 monomer and anti-Myc antibody were incubated at a dAb concentration of 3000 nM. TAR2m-21-23 3U TAR7m-16 was incubated with the cells at 25 nM, 83.3 nM, 250 nM, 833 nM and 2500 nM concentrations. TAR2m-21-23 40K PEG was incubated with the cells at 158.25 nM, 527.5 nM, 1582.5 nM, 5275 nM and 15825 nM concentrations. After incubation overnight, cell viability was assessed as described for the L929 cell cytotoxicity assay. The results revealed that incubation with various amounts of dAbs did not result in an increase in the number of non-viable cells in the cultures. The incubation of L929 cells with 10 nM, 1 nM and 0.1 nM of a commercially-available anti-TNFR1 IgG antibody resulted in a dose-dependent increase in non-viable cells thereby demonstrating the sensitivity of these cells to TNFR1-mediated agonism. (Figure 26).

### Example 15. Models of Chronic Inflammatory Diseases.

### A. Mouse Collagen-Induced Arthritis Model

DBA/1 mice were injected once with an emulsion of Arthrogen-CIA adjuvant and Arthrogen-CIA collagen (MD-biosciences). At day 21, animals with high arthritic scores were removed from the study and the remainder of the animals were divided into groups of 10 with equal numbers of male and female animals. At day 21 treatments commenced with intraperitoneal injections of either saline, ENBREL® (entarecept; Immunex Corporation) or TAR2m-21-23 40k PEG and continued for 28 days. Clinical arthritic scores on a scale of 0 to 4 were measured for each of the 4 limbs of the animals, a score of 0 was assigned for a normal limb and a score of 4 was assigned for a maximally inflamed limb with involvement of multiple joints.

A reduction of the summation of the arthritic scores of the four limbs from the maximum of 16 to (a) 14-15, (b) 12-15, (c) 10-15, (d) 9-15, (e) 7-15, (f) 5-15, (g), 3-15, or (h) 1-15 is a beneficial effect in this model. A beneficial effect can result is a summation of the arthritic scores of the four limbs of 0, 1, 2, 3, 4, 5, 6, 7, 8, 9, 10, 11, 12, 13, 14 or 15. A delay in the onset of arthritis, compared with the untreated control group, is also a beneficial effect in this model.

The clinical scores clearly demonstrated that treatment with TAR2m-21-23 40k PEG had a very favourable impact of inhibition of the development of arthritis when compared with the saline control, and moreover the TAR2m-21-23 40k PEG treatment compared favourably with ENBREL® (entarecept; Immunex Corporation). This is the first demonstration of inhibition of TNFR1 being efficacious in the treatment of a chronic inflammatory disease model.

### B. Mouse ΔARE model of IBD and arthritis

Mice that bear a targeted deletion in the 3' AU-rich elements (AREs) of the TNF mRNA (referred to as Tnf^{ΔARE} mice) overproduce TNF and develop an inflammatory bowel disease that is histopathologically similar to Crohn's disease. (Kontoyiannis et al., J Exp Med 196:1563-74 (2002).) In these mice, the Crohn's-like disease develops between 4 and 8 weeks of age, and the animals also develop clinical signs of rheumatoid arthritis.

dAbs that bind mouse TNFR-1 were assessed for efficacy in inhibiting Crohn's-like pathology and arthritis in Tnf^{ΔARE} mice. ENBREL® (entarecept; Immunex Corporation) was used as a positive control. The agents were administered by intraperitoneal injections according to the administration and dosing regiment presented in Table 13.

The dAbs that were studied include:
1) TAR2m-21-23 PEGylated with one 40 kD PEG moiety;
2) dual specific TAR2m-21-23 3U TAR7m-16 which binds mouse TNFR-1 and mouse serum albumin.

**Table 13.**

| Group | Treatment | Dose | Number of Doses | Number of Animals |
|---|---|---|---|---|
| 6 | ENBREL® (entarecept; Immunex Corporation) (administered 3 times/week) | 10 mg/kg | 8 | 10 |
| 5 | TAR2m21-23 40 kD PEG (administered twice/week) | 1 mg/kg | 8 | 10 |
| 4 | TAR2m21-23 40 kD PEG (administered twice/week) | 10 mg/kg | 8 | 10 |
| 3 | TAR2m-21-23 3U TAR7m-16 (administered twice/week) | 1 mg/kg | 8 | 10 |
| 2 | TAR2m-21-23 3U TAR7m-16 (administered twice/week) | 10 mg/kg | 8 | 10 |
| 1 | Saline | NA | 8 | 10 |

At the conclusion of the dosing period, the mice were sacrificed and the terminal ileums and proximal colons were removed for analysis.

For histological analysis, the tissue samples will be sectioned and stained with hematoxylin and eosin, and acute and chronic inflammation will be scored using a semi-quantitative scoring system. The scores will be assigned as follows: acute inflammation score 0 = 0-1 polymorphonuclear (PMN) cell per high powered field (PMN/hpf); 1 = 2-10 PMN/hpf within mucosa; 2 = 11-20 PMN/hpf within mucosa; 3 = 21-30 PMN/hpf within mucosa or 11-20 PMN/hpf with extension below muscularis mucosae; 4 = >30 PMN/hpf within mucosa or >20 PMN/hpf with extension below muscularis mucosae; chronic inflammation score 0 = 0-10 mononuclear leukocytes (ML) per hpf (ML/hpf) within mucosa; 1 = 11-20 ML/hpf within mucosa; 2 = 21-30 ML/hpf within mucosa or 11-20 ML/hpf with extension below muscularis mucosae; 3 = 31-40 ML/hpf within mucosa or follicular hyperplasia; and 4 = >40 ML/hpf within mucosa or >30 ML/hpf with extension below muscularis mucosae or follicular hyperplasia.

The macrophenotypic signs of arthritis were scored weekly according to the following system: 0 = no arthritis (normal appearance and flexion); 1 = mild arthritis (joint distortion); 2 = moderate arthritis (swelling, joint deformation); 3 = heavy arthritis (severely impaired movement).

The TAR2m-21-23 dAb demonstrated good *in vivo* efficacy in the delta ARE mouse model of arthritis as both a 40 kD PEGylated monomer (TAR2m21-23 40 kD PEG) and as a dual specific anti-TNFR1/anti-SA format (TAR2m-21-23 3U TAR7m-16). At week 9 the mean arthritic scores of both the TAR2m-21-23 and the TAR2m-21-23 3U TAR7m-16 treated groups were less than 0.4. In contrast the saline control group had moderate to severe arthritis with an average arthritic score that was >1.0. The group treated with ENBREL® (entarecept; Immunex Corporation), which was administered 3 times per week as compared with TAR2m-21-23 and the TAR2m-21-23 3U TAR7m-16 which were administered twice per week, had an average score of 0.5-1.0. These results indicate that therapy with dAb formats that bind TNFR1 is a highly efficacious anti-arthritis therapy, and that both the PEGylated and dual specificity dAb formats studied are highly effective drugs for chronic inflammatory disease. Moreover these results further demonstrate that dAbs that bind TNFR1 engage the receptor only in an antagonistic manner.

### C. Mouse DSS model of IBD.

IBD will be induced in mice by administering dextran sulfate sodium (DSS) in the drinking water. (See, e.g., Okayasu I. et al., Gastroenterology 98:694-702 (1990); Podolsky K., J Gasteroenterol. 38 suppl XV:63-66 (2003).) Adult BDF1 mice that are *H. pylori* free will be housed for 2 weeks to stabilize their circadian rhythms. All mice will be held in individually ventilated cages in a specific pathogen free (SPF) barrier unit on a 12 hour light:dark cycle. Animals will be allowed food and water *ad libitum* throughout.

The study will run for 7 days. The drinking water will contain 5% DSS for the duration of the study. All animals will be treated on days 1-7 in the morning (0900-1000) and evening (1600-1700). (See Table 14.) Day 1 is equivalent to a singe prophylactic dose. All animals to be weighed daily and any diarrhoea incidence will be noted. All animals will be sacrifices 24 hours following the last treatment, and will be administered a pulse of bromodeoxyuridine 40 minutes prior to sacrifice. The distal large intestines will be removed from the animals. A small sample of the distal large intestine will be placed into "RNAlater", and the remainder will be fixed in Carnoy's fixative, embedded in paraffin, sectioned (non-serial sections per slide) and stained with hematoxylin and eosin. Sections will be visually assessed for IBD severity and assigned a severity score. Histometric analyses will be performed and mean lesion area (ulcer area), mean epithelial area, and mean intramural inflammatory area will be determined.

H&E cross sections of the large intestine will be used to record a series of tissue dimensions using a Zeiss Axiohome microscope, which enables accurate quantification of areas. For each cross section, the area of epithelium plus lamina propria and the area of connective tissue will be measured. The epithelial area will then be measured separately, the difference being the area of lamina propria. In normal tissue the relative contribution of this tissue to the area is about 10%, but this increases as inflammation increases. The relative proportion of epithelium: lamina propria therefore changes.

With increasing severity the depth of this area narrows (contributing to the ulceration) and length of the colon shortens. Together these phenomena cause the cross-sectional area of the lumen to increase. This parameter can therefore also be a useful measurement of disease severity.

The tissue samples will be observed microscopically and assigned a severity score where 0 = no inflammation; 1 = mild inflammation around crypt base; 2 = massive inflammatory infiltration, and disrupted mucosal architecture; 3 = massive inflammatory infiltration, and disrupted mucosal architecture plus ulceration.

Efficacy is indicated in this model when the treatment produces a reduction of in severity score, relative to the severity score of the saline control group. For example the severity score of the treatment group can be reduced by 0.1 to about 1, 1 to about 2, or 2 to about 3. Efficacy would be indicated by a score of about 2 or less, 1 to about 2, or 1 or less.

9 groups of 6 animals will be treated as follows:

**Table 14.**

| Group | |
|---|---|
| 1 | DSS in drinking water |
| 2 | DSS in drinking water + ip PEG TAR2m-21-23, 10 mg/kg 1x/d |
| 3 | DSS in drinking water + ip PEG TAR2m-21-23, 1 mg/kg 1x/d |
| 4 | DSS in drinking water + ip saline |
| 5 | DSS in drinking water + oral gavage PEG TAR2m-21-23, 0.25 mg/animal 2x/d |
| 6 | DSS in drinking water + oral gavage saline |
| 7 | DSS in drinking water + ip dosing +ve control e.g. steroid |
| 8 | DSS in drinking water + oral gavage +ve control e.g. 5' aminosalicylic acid or similar |
| 9 | Untreated animals |

| | |
|---|---|
| Oral gavages will be given with ZANTAC® (ranitidine hydrochloride; GlaxoSmithKline). | |

### D. Mouse model of chronic obstructive pulmonary disease (COPD)

Efficacy of anti-TNFR1 dAbs in progression of disease in a mouse sub-chronic tobacco smoke (TS) model will be assessed. (See, e.g., Wright JL and Churg A., Chest 122:3065-3095 (2002).) Anti-mouse TNFR1 dAbs will be administered by intraperitoneal injections every 48 hours (starting 24 hours before the first exposure to TS) and will be given as extended serum half life format (*e.g.*, PEGylated, dual specific ligand comprising anti-SA dAb).

Alternatively the anti-TNFR1 dAb will be administered by intranasal delivery every 24 hours (starting 4 hours before the first exposure to TS) and will be given as a monomer dAb. ENBREL® (entarecept; Immunex Corporation) will be used as a positive control. TS exposure will be daily and the study will last for 1-2 weeks. (See, *e.g.,* Vitalis et al., Eur. Respir. J., 11:664-669 (1998).) Following the last TS exposure bronchoaveolar lavage will be analysed for total and differential cell counts to include neutrophils, eosinophils, macrophages and T-lymphocyte subsets. The lung lobes will be fixed in 10% buffered formalin and tissue sections analysed for enlargement of the alveoli and alveolar ducts, thickening of the small airway walls and for cell counts to include neutrophils, eosinophils, macrophages and T-lymphocyte subsets. Efficacy will be evident by a reduction in the number of neutrophils, eosinophils, macrophages and T-lymphocyte subsets that were elevated by TS exposure and a reduction in the TS-induced enlargement of the alveoli and alveolar ducts and thickening of the small airway walls.

### Example 18. TAR2m21-23 dimers are high avidity TNFR1 antagonists.

TAR2m21-23 dimers were prepared by producing a form of TAR2m21-23 that contained a cys residue at the carboxy-terminus using the methods described in Example 8. The protein (TAR2m21-23CYS) was expressed in *Pichia* and purified using Streamline Protein A. Non-reducing SDS-PAGE analysis showed that ∼40-50% of the protein was present in solution as a dimer. The dimer was further purified using gel filtration chromatography.

2 mg of protein was concentrated down to about 250 µl and applied to a Superdex 75 HR gel filtration column (Amersham Bioscience) which had previously been equilibrated with PBS. The column was run at a flow rate of 0.5 ml/min and 0.5 ml fractions were collected. Elution of protein from the column was monitored at 280 nm and dimer containing fractions were identified by non-reducing SDS-PAGE. Fractions that contained dimers but no monomeric TAR2m-21-23CYS were combined. The combined fractions were concentrated and the potency of the dimeric dAb determined in the L929 TNF cell cytotoxicity assay (Example 6).

The biological potency of TAR2m21-23 dimer was compared against monomeric TAR2m-21-23 in the L929 cytotoxicity assay. In this assay, inhibition of TNF-induced cytotoxicity of mouse L929 cells by TAR2m21-23 monomer and TAR2m21-23 dimer was assessed, and results were expressed as the concentration of dAb monomer or dimer that inhibited cytotoxicity by 50% in the assay (neutralizing dose 50, ND50).

The monomeric dAb had an ND50 of about 600 pM in the assay. The ND50 of the dimerized dAb (TAR2m21-23 dimer) was about 10-fold lower (ND50 about 60-70 pM) in the assay. These results show that TAR2m21-23 dimer had a significantly improved affinity for cell surface TNFR1 in comparison to the dAb monomer. The results indicate that TAR2m21-23 dimer binds to two separate TNFR1 molecules on the cell surface simultaneously, and the resulting avidity effect upon multimerisation results in improved inhibition of TNFR1.

The dimeric format (TAR2m21-23 dimer) was then tested to see if it displayed any signs of TNFR1 agonism, i.e. the ability to cause cross-linking of TNFR1 and initiate intracellular signaling and cell death. This was achieved using a modified L929 cell cytotoxicity assay in which no TNF-α was added, but anti-TNFR1 antibodies or the dAb formats were tested to see if they agonize TNFR1 and induce cell death. Anti-TNFR1 antibody AF-425-PB (R&D Systems) which is a known agonists of TNFR1, and anti-TNFR1 antibody MAB430 (R&D Systems), a reported antagonist of TNFR1, TAR2m-21-23 and TAR2m21-23 dimer were tested in the assay.

Antibody AF-425-PB activated TNFR1 and induced cytotoxicity in the assay with a ND50 of about 100 pM. Even the reported antagonist antibody MAB430 caused receptor cross-linking and cell killing in the assay with an ND50 of about 10nM. In contrast, TAR2m-21-23 dimer did not cause any cell death in the assay, even when present at very high concentrations (>1 µM). These results show that TAR2m21-23 dimer is not a TNFR1 agonist.

The results indicate that dimers, trimers or other multimers of dAbs that bind TNFR1 have high avidity for TNFR1 expressed on the surface of cells and are effective TNFR1 antagonists. Moreover, the results of this study show that multimers of dAbs that bind Domain 1 of TNFR1, such as TAR2m21-23 dimer, can bind two TNFR1 molecules (as can the antibodies that acted as agonists in the assay) and that binding the domain or epitope target on TNFR1 (Domain 1) prevented the close association of receptor chains that is required for the initiation of TNFR1 signaling. This property is unique for a bivalent molecule in that it is able to crosslink TNFR1 on the cell surface and yet not cause TNFR1 signaling and cell death.

### Example 20. Isolation of dAbs that bind human TNFR1 and mouse TNFR1

dAbs of known sequence were expressed in *E. coli* and purified with Protein A streamline resin. After elution into Tris-Glycine, dAbs were flowed over an SPR chip to which biotinylated human TNFR1 had been immobilized (flow cell 2) and biotinylated murine TNFR1 had been immobilized (flow cell 4). (The SPR chip was coated with human TNFR1 and murine TNFR1 at similar densities.) Flow cells 1 and 3 were left blank and acted as no antigen reference surfaces for the detection and subtraction of non-specific binding.

dAb was flowed over the 4 flow cells in series (ie flow cell 1, then 2, 3 and finally 4) with the response differences between flow cells 2 and 1 measured and the response differences between flow cells 4 and 3 being also measured. The former being a measure of binding to human TNFR1 and the later binding to murine TNFR1. Specific binding curves were noted for binding to both human TNFR1 and murine TNFR1, the nature of the curves being such that a faster on-rate for human TNFR1 than murine TNFR1 was noted. Off-rates were broadly similar. An assessment of the cross-reactivity is given by the number of response units (RU) maximally achieved by each binding event. In this example the human biotinylated TNFR1 surface comprised approximately 900RU of TNFR1 on flow cell 2, while flow cell 4 comprised about 1400RU of murine TNFR1. As a control, TAR2h-154-7, a human specific dAb at a concentration of 2 micromolar bound the human surface with a maximal response of 385RU, giving a response on the mouse surface of only 4.5RU. 2 micromolar of TAR2h-205 gave a response on the human surface of 435RU, and a response on the mouse surface of 266RU.

### Annex 1; polypeptides which enhance half-life in vivo.

Alpha-1 Glycoprotein (Orosomucoid) (AAG)
Alpha-1 Antichyromotrypsin (ACT)
Alpha-1 Antitrypsin (AAT)
Alpha-1 Microglobulin (Protein HC) (AIM)
Alpha-2 Macroglobulin (A2M)
Antithrombin III (AT III)
Apolipoprotein A-1 (Apo A-1)
Apoliprotein B (Apo B)
Beta-2-microglobulin (B2M)
Ceruloplasmin (Cp)
Complement Component (C3)
Complement Component (C4)
C1 Esterase Inhibitor (C1 INH)
C-Reactive Protein (CRP)
Cystatin C (Cys C)
Ferritin (FER)
Fibrinogen (FIB)
Fibronectin (FN)
Haptoglobin (Hp)
Hemopexin (HPX)
Immunoglobulin A (IgA)
Immunoglobulin D (IgD)
Immunoglobulin E (IgE)
Immunoglobulin G (IgG)
Immunoglobulin M (IgM)
Immunoglobulin Light Chains (kapa/lambda)
Lipoprotein(a) [Lp(a)]
Mannose-bindign protein (MBP)
Myoglobin (Myo)
Plasminogen (PSM)
Prealbumin (Transthyretin) (PAL)
Retinol-binding protein (RBP)
Rheomatoid Factor (RF)
Serum Amyloid A (SAA)
Soluble Tranferrin Receptor (sTfR)
Transferrin (Tf)

### Annex 2

### Data Summary

| TARGET | dAb | Equilibrium dissocation constant (Kd = Koff/Kon) | Koff | IC50 for ligand assay | ND50 for cell based neutralisn assay |
|---|---|---|---|---|---|
| TAR1 | TAR1 monomers | 300nM to 5pM (ie, 3 x 10⁻⁷ to 5 x 10⁻¹²), preferably 50nM to 20pM | 5 x 10⁻¹ to 1 x 10⁻⁷ | 500nM to 100pM | 500nM to 50pM |
| | TAR1 dimers | As TAR1 monomer | As TAR1 monomer | As TAR1 monomer | As TAR1 monomer |
| | TAR1 trimers | As TAR1 monomer | As TAR1 monomer | As TAR1 monomer | As TAR1 monomer |
| | TAR1-5 | | | | |
| | TAR1-27 | | | | |
| | TAR1-5-19 monomer | 30nM | | | |
| | TAR1-5-19 homodimer | | | With (Gly₄Ser)₃ linker = 20nm | =30nM |
| | | | | | =3nM |
| | | | | With (Gly₄Ser)₅ linker = 2nm | =15nM |
| | | | | With (Gly₄Ser)₇ linker = 10nm In Fab format = 1nM | |
| | TAR1-5-19 heterodimers | | | With (Gly₄Ser)ₙ linker TAR1-5-19d2= 2nM | = 12nM |
| | | | | TAR1-5-19d3=8nM | = 10nM |
| | | | | TAR1-5-19 d4 = 2-5nM | = 12nM |
| | | | | TAR1-5-19 d5 = 8nM | |
| | | | | In Fab format | |
| | | | | TAR1-5-19CH d1CK = 6nM | |
| | | | | TAR1-5-19CK d1CH = 6nM | |
| | | | | TAR1-5-19CH d2CK = 8nM | |
| | | | | TAR1-5-19CH d3CK = 3nM | |
| | TAR1-5 heterodimers | | | With (Gly₄Ser)ₙ linker | |
| | | | | TAR1-5d1 = 30nM | |
| | | | | TAR1-5d2 = 50nM | |
| | | | | TAR1-5d3 = 300nM | |
| | | | | TAR1-5d4 = 3nM | = 60nM |
| | | | | TAR1-5d5 = 200nM | |
| | | | | TAR1-5d6 =100nM | |
| | | | | In Fab format | |
| | | | | TAR1-5CH d2CK = 30nM TAR1-5CK d3 CH = 100nM | |
| | TAR1-5-19 homotrimer | | | 0.3nM | 3-10nM (eg, 3nM) |
| | | | | | |
| TAR2 | TAR2 monomers | As TAR1 monomer | As TAR1 monomer | 500nM to 100pM | 500nM to 50pM |
| | TAR2-10 | | | | |
| | TAR2-5 | | | | |
| Serum Albumin | Anti-SA monomers | 1nM to 500µM, preferably 100nM to 10 µM | | 1nM to 500µM, preferably 100nM to10 µM | |
| | | In Dual Specific format, target affinity is 1 to 100,000 x affinity of SA dAb affinity, eg 100pM (target) and 10 µM SA affinity. | | In Dual Specific format, target affinity is 1 to 100,000 x affinity of SA dAb affinity, eg 100pM (target) and 10 µM SA affinity. | |
| | MSA-16 | 200nM | | | |
| | MSA-26 | 70nM | | | |

## Claims

1. An antagonist of Tumor Necrosis Factor 1 (TNFR1) that binds TNFR1 and inhibits signal transduction through TNFR1; (i) wherein said antagonist does not inhibit binding of TNFα to TNFR1; (ii) wherein said antagonist is a domain antibody (dAb); and (iii) wherein said dAb comprises an amino acid sequence that is at least 90% homologous to the amino acid sequence of TAR2h-205 (SEQ ID NO: 627 in Figure 271).

2. The antagonist of claim 1, wherein said antagonist inhibits TNFα-induced cell death in a standard L929 cytotoxicity assay of TNFα-induced secretion of IL-8 in a standard HeLa IL-8 assay.

3. A ligand comprising a domain antibody (dAb) as defined in claim 1 or claim 2.

4. The ligand of claim 3, wherein the ligand comprises said TNFR1-binding dAb and a half-life extending moiety.

5. The ligand of claim 4, wherein the half-life extending moiety is a polyethylene glycol moiety, serum albumin or a fragment thereof, transferrin receptor or a transferrin-binding portion thereof, or an antibody or antibody fragment comprising a binding site for a polypeptide that enhances half-life *in vivo.*

6. The ligand of claim 5, wherein the half-life extending moiety is an antibody or antibody fragment comprising a binding site for serum albumin or neonatal Fc receptor.

7. An isolated nucleic acid encoding dAb or ligand as defined in any one of claims 1 to 6.

8. A vector comprising a recombinant nucleic acid encoding a dAb or ligand as defined in any one of claims 1 to 6.

9. A host cell comprising a vector of claim 8 or a nucleic acid of claim 7.

10. A method for producing a polypeptide comprising maintaining a host cell of claim 9 under conditions suitable for expression of the recombinant nucleic acid or vector, whereby a polypeptide is produced.

11. A pharmaceutical composition comprising a dAb of claim 1 or 2, or ligand of any of claims 3 to 6 and a pharmacologically acceptable carrier.

12. Use of a ligand of any of claims 3 to 6 for the manufacture of a medicament for treating, supressing or preventing a chronic inflammatory disease, an autoimmune disorder, inflammatory disease, arthritis, multiple sclerosis, inflammatory bowel disease, chronic obstructive pulmonary disease, pneumonia, or septic shock.

## Patentansprüche

1. Antagonist des Tumornekrosefaktors-1 (TNFR1), der an TNFR1 bindet und die Signalübertragung durch TNFR1 inhibiert;
(i) wobei der Antagonist nicht die Bindung von TNFα an TNFR1 inhibiert;
(ii) wobei der Antagonist ein Domänenantikörper (dAb) ist; und
(iii) wobei der dAb eine Aminosäuresequenz umfasst, die zu mindestens 90% homolog ist zu der Aminosäuresequenz von TAR2h-205 (SEQ ID NO:627 in Figur 27I).

2. Antagonist nach Anspruch 1, wobei der Antagonist TNFα-induzierten Zelltod in einem standardisierten L929-Cytotoxizitätstest der TNFα-induzierten Sekretion von IL-8 in einem standardisierten HeLa-IL-8-Test inhibiert.

3. Ligand, der einen wie in Anspruch 1 oder 2 definierten Domänenantikörper (dAb) umfasst.

4. Ligand nach Anspruch 3, wobei der Ligand den TNFR1-bindenden Domänenantikörper und eine Einheit umfasst, die die Halbwertszeit verlängert.

5. Ligand nach Anspruch 4, wobei die Einheit, die die Halbwertszeit verlängert, eine Polyethylenglycol-Gruppe, Serumalbumin oder ein Fragment davon, ein Transferrin-Rezeptor oder ein Transferrin-bindender Teil davon, oder ein Antikörper oder Antikörperfragment umfassend eine Bindungsstelle für ein Polypeptid, das die in vivo-Halbwertszeit erhöht, ist.

6. Ligand nach Anspruch 5, wobei die Einheit, die die Halbwertszeit verlängert, ein Antikörper oder Antikörperfragment, umfassend eine Bindungsstelle für Serumalbumin oder neonatalen Fc-Rezeptor, ist.

7. Isolierte Nucleinsäure, die einen wie in einem der Ansprüche 1 bis 6 definierten dAb oder Liganden codiert.

8. Vektor, der eine rekombinante Nucleinsäure umfasst, die einen wie in einem der Ansprüche 1 bis 6 definierten dAb oder Liganden codiert.

9. Wirtszelle, die einen Vektor nach Anspruch 8 oder eine Nucleinsäure nach Anspruch 7 umfasst.

10. Verfahren zur Herstellung eines Polypeptids, das das Halten einer Wirtszelle nach Anspruch 9 unter Bedingungen umfasst, die für die Expression der rekombinanten Nucleinsäure oder des Vektors geeignet sind, wodurch ein Polypeptid hergestellt wird.

11. Arzneimittel, das einen dAb nach Anspruch 1 oder 2 oder einen Liganden nach einem der Ansprüche 3 bis 6 und einen pharmazeutisch verträglichen Träger umfasst.

12. Verwendung eines Liganden nach einem der Ansprüche 3 bis 6 für die Herstellung eines Arzneimittels zur Behandlung, Unterdrückung oder Vorbeugung einer chronischen entzündlichen Erkrankung, einer Autoimmunerkrankung, einer entzündlichen Erkrankung, von Arthritis, von multipler Sklerose, von entzündlicher Darmerkrankung, von chronischer obstruktiver Lungenerkrankung, von Lungenentzündung oder von septischem Schock.

## Revendications

1. Antagoniste du facteur de nécrose tumorale 1 (TNFR1) qui se lie à TNFR1 et inhibe la transduction de signal par TNFR1 ; (i) dans lequel ledit antagoniste n'inhibe pas la liaison de TNFα à TNFR1 ; (ii) dans lequel ledit antagoniste est un anticorps à domaine (dAb) ; et (iii) dans lequel ledit dAb comprend une séquence d'acides aminés qui est homologue à au moins 90 % à la séquence d'acides aminés de TAR2h-205 (SEQ ID N° : 627 sur la figure 271).

2. Antagoniste selon la revendication 1, dans lequel ledit antagoniste inhibe la mort cellulaire induite par TNFα dans un essai standard de cytotoxicité L929 de la sécrétion d'IL-8 induite par TNFα d'IL-8 dans un essai d'IL-8 HeLa standard.

3. Ligand comprenant un anticorps à domaine (dAb) tel que défini dans la revendication 1 ou la revendication 2.

4. Ligand selon la revendication 3, dans lequel le ligand comprend ledit dAb se liant à TNFR1 et un fragment prolongeant la demi-vie.

5. Ligand selon la revendication 4, dans lequel le fragment prolongeant la demi-vie est un fragment de polyéthylène glycol, la séralbumine ou un fragment de celle-ci, un récepteur de la transferrine ou une partie de celui-ci se liant à la transferrine, ou un anticorps ou un fragment d'anticorps comprenant un site de liaison pour un polypeptide qui renforce la demi-vie *in vivo.*

6. Ligand selon la revendication 5, dans lequel le fragment prolongeant la demi-vie est un anticorps ou un fragment d'anticorps comprenant un site de liaison pour la séralbumine ou le récepteur Fc néonatal.

7. Acide nucléique isolé codant pour un dAb ou un ligand selon l'une quelconque des revendications 1 à 6.

8. Vecteur comprenant un acide nucléique recombinant codant pour un dAb ou un ligand tel que défini dans l'une quelconque des revendications 1 à 6.

9. Cellule hôte comprenant un vecteur selon la revendication 8 ou un acide nucléique selon la revendication 7.

10. Procédé de production d'un polypeptide comprenant le maintien d'une cellule hôte selon la revendication 9 dans des conditions appropriées pour l'expression de l'acide nucléique recombinant ou du vecteur, un polypeptide étant ainsi produit.

11. Composition pharmaceutique comprenant un dAb selon la revendication 1 ou 2 ou ligand selon l'une quelconque des revendications 3 à 6 et un véhicule pharmacologiquement acceptable.

12. Utilisation d'un ligand selon l'une quelconque des revendications 3 à 6 pour la fabrication d'un médicament pour le traitement, la suppression ou la prévention d'une maladie chronique inflammatoire, d'une maladie auto-immune, d'une maladie inflammatoire, de l'arthrite, de la sclérose en plaque, d'une maladie intestinale inflammatoire, d'une maladie pulmonaire obstructive chronique, d'une pneumonie ou d'un choc septique.
